(19) Europäisches Patentamt
European Patent Office
Office européen des brevets

(11) EP 1 814 880 B1

(12) **EUROPEAN PATENT SPECIFICATION**

(45) Date of publication and mention of the grant of the patent:
**28.01.2009 Bulletin 2009/05**

(21) Application number: **05808135.7**

(22) Date of filing: **04.11.2005**

(51) Int Cl.:
***C07D 471/04*** *(2006.01)* ***A61K 31/44*** *(2006.01)*

(86) International application number:
**PCT/EP2005/055753**

(87) International publication number:
**WO 2006/051063 (18.05.2006 Gazette 2006/20)**

(54) **IMIDAZO[1,2-A]PYRIDINE COMPOUNDS, COMPOSITIONS, USES AND METHODS RELATED THERETO**

IMIDAZO[1,2-A]PYRIDINVERBINDUNGEN, ZUSAMMENSETZUNGEN, ANWENDUNGEN UND VERFAHREN, DIE DAMIT IN ZUSAMMENHANG STEHEN

DÉRIVÉS D'IMIDAZO[1,2-A]PYRIDINE, ET PRÉPARATIONS, EMPLOIS ET MÉTHODES LIÉS À CES DÉRIVÉS

(84) Designated Contracting States:
**AT BE BG CH CY CZ DE DK EE ES FI FR GB GR HU IE IS IT LI LT LU LV MC NL PL PT RO SE SI SK TR**

(30) Priority: **11.11.2004 EP 04105698**

(43) Date of publication of application:
**08.08.2007 Bulletin 2007/32**

(73) Proprietor: **FERRER INTERNACIONAL, S.A.**
**08028 Barcelona (ES)**

(72) Inventors:
- **FALCÓ, José, Luis**
  **E-08004 Barcelona (ES)**
- **PALOMER, Albert**
  **E-08014 Barcelona (ES)**
- **GUGLIETTA, Antonio**
  **E-08750 Molins de Rei (ES)**

(74) Representative: **Barlocci, Anna**
**ZBM Patents**
**Zea, Barlocci & Markvardsen**
**C/ Balmes, 114 - 4º**
**08008 Barcelona (ES)**

(56) References cited:
**EP-A- 0 172 096** **EP-A- 0 234 970**
**US-A- 4 767 755** **US-A1- 2004 204 443**

- **SCHMITT M ET AL: "IMIDAZO1,2-B) PYRIDAZINES. XXIII SOME 5-DEAZA ANALOGUES. SYNTHESES OF SOME 2-ARYL-6-(CHLORO, METHOXY OR UNSUBSTITUTED)-3-(VARIOUSLY SUBSTITUTED)IMIDAZO1,2-APYRIDINES AND THEIR AFFINITY FOR CENTRAL AND MITOCHONDRIAL BENZODIAZEPINE RECEPTORS" AUSTRALIAN JOURNAL OF CHEMISTRY, XX, XX, vol. 50, no. 7, 1997, pages 719-725, XP001010372 ISSN: 0004-9425**
- **ALMIRANTE L ET AL: "DERIVATI DELL'IMIDAZOLO NOTA VI - SINTESI E ATTIVITA FARMACOLOGICA DE DERIVATI AZOTATI DI IMIDAZO-?,2-A?PIRIDINE IMIDAZOLE DERIVATIVES. VI. SYNTHESIS AND PHARMACOLOGICAL ACTIVITY OF NITROGEN-CONTAINING DERIVATIVES OF IMIDAZO?,2-A?YRIDINE" BOLLETTINO CHIMICO FARMACEUTICO, SOCIETA EDITORIALE FARMACEUTICA, MILANO, IT, vol. 110, no. 6, 1971, pages 322-329, XP009023479 ISSN: 0006-6648**
- **TRAPANI G: "Novel 2-Phenylimidazo[1,2-a] pyridine Derivatives as Potent and Selective Ligands for Peripheral Benzodiazepine Receptors: Synthesis, Binding Affinity, and in Vivo Studies" JOURNAL OF MEDICINAL CHEMISTRY, AMERICAN CHEMICAL SOCIETY. WASHINGTON, US, vol. 42, 1999, pages 3934-3941, XP002154069 ISSN: 0022-2623**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

Remarks:

The file contains technical information submitted after the application was filed and not included in this specification

## Description

### Technical field

This invention is directed to agents with affinity for $GABA_A$ receptor, specifically to imidazo[1,2-a]pyridine compounds.

### Background of the invention

$GABA_A$ receptor (γ-aminobutyric $acid_A$) is a pentameric protein which forms a membrane ion channel. $GABA_A$ receptor is implicated in the regulation of sedation, anxiety, muscle tone, epileptogenic activity and memory functions. These actions are due to defined subunits of $GABA_A$ receptor, particularly the $\alpha_1$- and $\alpha_2$-subunits.

Sedation is modulated by the $\alpha_1$-subunit. Zolpidem is characterized by a high affinity for the $\alpha_1$-receptors and its sedative and hypnotic action is mediated by these receptors in vivo. Similarly, the hypnotic action of zaleplon is also mediated by the $\alpha_1$-receptors.

The anxiolytic action of diazepam is mediated by the enhancement of GABAergic transmission in a population of neurons expressing the $\alpha_2$-receptors. This indicates that the $\alpha_2$-receptors are highly specific targets for the treatment of anxiety.

Muscle relaxation in diazepam is mainly mediated by $\alpha_2$-receptors, since these receptors exhibit a highly specific expression in spinal cord.

The anticonvulsant effect of diazepam is partly due to $\alpha_1$-receptors. In diazepam, a memory-impairing compound, anterograde amnesia is mediated by $\alpha_1$-receptors.

$GABA_A$ receptor and its $\alpha_1$- and $\alpha_2$-subunits have been widely reviewed by H. Möhler et al.(J. Pharmacol. Exp. Ther., 300, 2-8, 2002); H. Möhler et al.(Curr. Opin. Pharmacol., 1, 22-25, 2001); U. Rudolph et al.(Nature, 401, 796-800, 1999); and D.J. Nutt et al. (Br. J. Psychiatry, 179, 390-396, 2001).

Diazepam and other classical benzodiazepines are extensively used as anxiolytic agents, hypnotic agents, anticonvulsants and muscle relaxants. Their side effects include anterograde amnesia, decrease in motor activity and potentiation of ethanol effects.

In this context, the compounds of this invention are ligands of $\alpha_1$- and $\alpha_2$-$GABA_A$ receptor for their clinical application in sleep disorders, preferably insomnia, anxiety and epilepsy.

Insomnia is a highly prevalent disease. Its chronicity affects 10% of the population and 30% when transitory insomnia is computed as well. Insomnia describes the trouble in falling asleep, staying asleep or waking up too early, experiencing a non-refreshing sleep, and is associated with next-day hangover effects such as weariness, lack of energy, low concentration and irritability. The social and health impact of this complaint is important and results in evident socioeconomic repercussions.

Pharmacological therapy in the management of insomnia firstly included barbiturates and chloral hydrate, but these drugs elicit numerous known adverse effects, for example, overdose toxicity, metabolic induction, and enhanced dependence and tolerance. In addition, they affect the architecture of sleep by decreasing above all the duration and the number of REM sleep stages. Later, benzodiazepines meant an important therapeutic advance because of their lower toxicity, but they still showed serious problems of dependence, muscle relaxation, amnesia and rebound insomnia following discontinuation of medication.

The latest known therapeutic approach has been the introduction of non-benzodiazepine hypnotics, such as pyrrolo[3,4-b]pyrazines (zopiclone), imida-zo[1,2-a] pyridines (zolpidem) and, finally, pyrazolo[1,5-a] pyrimidines (zaleplon) . Later, two new pyrazolo[1,5-a] pyrimidines, indiplon and ocinaplon, have entered into development, the latter with rather anxiolytic action. All these compounds show a rapid sleep induction and have less next-day hangover effects, lower potential for abuse and lower risk of rebound insomnia than benzodiazepines. The mechanism of action of these compounds is the alosteric activation of $GABA_A$ receptor through its binding to benzodiazepines binding site (C. F. P. George, The Lancet, 358, 1623-1626, 2001). While benzodiazepines are unspecific ligands at $GABA_A$ receptor binding site, zolpidem and zaleplon show a greater selectivity for $\alpha_1$-subunit. Notwithstanding that, these drugs still affect the architecture of sleep and may induce dependence in long-term treatments.

Zolpidem is disclosed in US 4382938. Some other related hypnotic imidazo[1,2-a]pyridines have been disclosed in FR 2593818, US 4650796 and EP 172096. In US 4626538 (zaleplon), US 4654347, US 6399621 (indiplon) and EP 129847 (ocinaplon) hypnotic pyrazolo[1,5-a]pyrimidines are disclosed. The use of N-[[(ethyl-4-phenyl)-2-imidazo[1,2-a] pyridinyl-3]methyl-N,3-dimethylbutanamide, a compound previously disclosed in EP 172096, has been claimed in the manufacturing of anesthetic medicaments in EP 430738.

Research for new active compounds in the management of insomnia answers an underlying health need, because even recently introduced hypnotics still affect the architecture of sleep and may induce dependence in long-term treatments.

It is therefore desirable to focus on the development of new hypnotic agents with a lower risk of side effects.

Thus, the present invention is directed to new imidazo[1,2-a]pyridine compounds which are active versus $GABA_A$ and, particularly, versus its $\alpha_1$- and $\alpha_2$-subunits. Consequently, the compounds of this invention are useful in the treatment and prevention of all those diseases mediated by $GABA_A$ receptor $\alpha_1$- and $\alpha_2$-subunits. Non-limitative examples of such diseases are sleep disorders, preferably insomnia, anxiety and epilepsy. Non-limitative examples of the relevant indications of the compounds of this invention are all those diseases or conditions, such as insomnia or anesthesia, in which an induction of sleep, an induction of sedation or an induction of muscle relaxation are needed.

**Detailed description of the invention**

The present invention relates to novel imidazo[1,2-a]pyridine compounds of general formula (I):

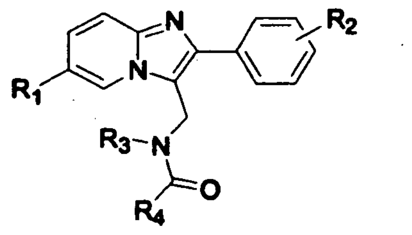

(I)

as well as pharmaceutically acceptable salts thereof;
wherein
$R_1$ and $R_2$ are independently selected from the group consisting of hydrogen, linear or branched alkyl($C_1$-$C_6$), alkenyl ($C_2$-$C_6$), alkynyl ($C_2$-$C_6$), haloalkyl ($C_1$-$C_6$), O-alkyl($C_1$-$C_6$), fluoro, chloro and bromo;
$R_3$ is selected from the group consisting of hydrogen, linear or branched alkyl ($C_1$-$C_6$), cycloalkyl ($C_3$-$C_6$), cycloalkyl ($C_3$-$C_6$) alkyl ($C_1$-$C_6$), alkenyl ($C_2$-$C_6$), alkenyl ($C_2$-$C_6$) alkyl ($C_1$-$C_6$), alkynyl ($C_2$-$C_6$) alkynyl ($C_3$-$C_6$)alkyl($C_1$-$C_6$) ;
$R_4$ is selected from the group consisting of haloalkyl($C_2$-$C_6$), cycloalkyl ($C_3$-$C_5$), cycloalkyl- ($C_3$-$C_6$) alkyl ($C_1$-$C_6$), alkynyl ($C_2$-$C_6$)alkyl($C_1$-$C_6$), alkyl ($C_1$-$C_6$) - O-alkyl ($C_1$-$C_6$), alkyl ($C_1$-$C_6$) -NH-alkyl ($C_1$-$C_6$) alkyl ($C_1$-$C_6$)-N(dialkyl($C_1$-$C_6$)), $-OR_5$, $-NMR_5$, $-NR_5R_6$,

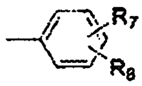

phenylalkyl ($C_2$-$C_6$), phenylalkenyl ($C_2$-$C_6$), naphthyl, monosubstituted naphthyl, disubstituted naphthyl, naphthylalkyl ($C_1$-$C_6$), naphthylalkenyl($C_2$-$C_6$), furyl, substituted furyl benzofuryl, substituted benzofuryl, pyrrolyl, substituted pyrrolyl, isoxazolyl, substituted isoxazolyl, benzoisoxazolyl, substituted benzoisoxazolyl, imidazolyl, substituted imidazolyl, benzimidazolyl, substituted benzimidazolyl, indolyl, substituted indolyl, pyrazolyl, substituted pyrazolyl, thienyl, substituted thienyl, benzothienyl, substituted benzothienyl, thiazolyl, substituted thiazolyl, benzothiazolyl, substituted benzothiazolyl, quinolinyl, substituted quinolinyl, isoquinolinyl, substituted isoquinolinyl, pyridyl, substituted pyridyl, pyrazinyl, substituted pyrazinyl, 6-oxo-1,4,5,6-tetrahydropyridazinyl, substituted 6-oxo-1,4,5,6-tetrahydropyridazinyl, thiadiazolyl, substituted thiadiazolyl, isothiazolyl, substituted isothiazolyl, thienylmethyl, 2-oxochromenyl, substituted 2-oxochromenyl, 2-(furan-2-yl)vinyl, oxazolyl, substituted oxazolyl, and benzisoxazolyl;
$R_5$ and $R_6$ are independently selected from the group consisting of hydrogen, linear or branched alkyl($C_1$-$C_6$), phenylalkyl ($C_1$-$C_6$), haloalkyl ($C_1$-$C_6$), cycloalkyl ($C_3$-$C_6$), cycloalkyl ($C_3$-$C_6$) alkyl ($C_1$-$C_6$), alkenyl ($C_2$-$C_6$) and alkynyl ($C_2$-$C_6$), alkenyl ($C_2$-$C_6$) alkyl($C_1$-$C_6$), alkynyl($C_2$-$C_6$)alkyl($C_1$-$C_6$), phenyl, substituted phenyl, heteroaryl, substituted heteroaryl; and
$R_7$ and $R_8$ are independently selected from the group consisting of linear or branched alkyl($C_2$-$C_6$), cycloalkyl ($C_3$-$C_6$), alkenyl ($C_2$-$C_6$), alkynyl ($C_2$-$C_6$), -OH, - O-alkyl ($C_1$-$C_6$), -SH, -S-alkyl ($C_1$-$C_6$), halo-alkyl ($C_1$-$C_6$), $\omega,\omega,\omega$-trifluoroalkyl ($C_1$-$C_6$), -NHalkyl ($C_1$-$C_6$), - Ndialkyl ($C_1$-$C_6$), $-NO_2$, -CN, $-SO_2$alkyl($C_1$-$C_6$), - COalkyl ($C_1$-$C_6$), -COOalkyl ($C_1$-$C_6$), -CO-NHalkyl ($C_1$-$C_6$), - CONdialkyl($C_1$-$C_6$), phenyl, substituted phenyl, heteroaryl and substituted heteroaryl.

The term "pharmaceutically acceptable salt" used herein encompasses any salt formed from organic and inorganic acids, such as hydrobromic, hydrochloric, phosphoric, nitric, sulfuric, acetic, adipic, aspartic, benzenesulfonic,

benzoic, citric, ethanesulfonic, formic, fumaric, glutamic, lactic, maleic, malic, malonic, mandelic, methanesulfonic, 1,5-naphthalendisulfonic, oxalic, pivalic, propionic, p-toluenesulfonic, succinic, tartaric acids and the like.

The term "substituted" used herein refers to the substitution of the corresponding radical or compound with at least one suitable substituent preferably selected from the group consisting of linear or branched alkyl ($C_2$-$C_6$), cycloalkyl ($C_3$-$C_6$), alkenyl($C_2$-$C_6$), alkynyl ($C_2$-$C_6$), -OH, -O-alkyl ($C_1$-$C_6$), -SH, -S-alkyl ($C_1$-$C_6$), halo-alkyl ($C_1$-$C_6$), ω,ω,ω-trifluoroalkyl ($C_1$-$C_6$), - NHalkyl ($C_1$-$C_6$), -Ndialkyl ($C_1$-$C_6$), $-NO_2$, -CN, $SO_2$alkyl ($C_1$-$C_6$), -COalkyl($C_1$-$C_6$), -COOalkyl($C_1$-$C_6$), -CO-NHalkyl ($C_1$-$C_6$), -CONdialkyl ($C_1$-$C_6$), phenyl, substituted phenyl, heteroaryl, substituted heteroaryl, fluoro, chloro and bromo.

The preferred compounds of the present invention are shown below:

Furan-2-carboxylic acid (6-methyl-2-p-tolyl-imidazo[1,2-a] pyridin-3-ylmethyl)-amide;
Pyridine-2-carboxylic acid (6-methyl-2-p-tolyl-imidazo [1,2-a]pyridin-3-ylmethyl)-amide:
Thiophene-2-carboxylic_acid (6-methyl-2-p-tolyl-imidazo [1,2-a]pyridin-3-ylmethyl)-amide;
Cyclopropanecarboxylic acid (6-methyl-2-p-tolyl-imidazo [1,2-a]pyridin-3-ylmethyl)-amide:
5-Nitro-furan-2-carboxylic acid (6-methyl-2-p-tolyl-imidazo [1,2-a]pyridin-3-ylmethyl)-amide;
3,5-Difluoro-pyridine-2-carboxylic acid (6-methyl-2-p-tolyl-imidazo[1,2-a]pyridin-3-ylmethyl)-amide;
6-Methoxy-benzothiazole-2-carboxylic acid(6-methyl-2-p-tolyl-imidazo[1,2-a]pyridin-3-ylmethyl)-amide;
4-Dimethylamino-N-methyl-N-(6-methyl-2-p-tolyl-imidazo [1,2-a]pyridin_3-ylmethyl)-benzamide:
Cyclopropanecarboxylic acid methyl-(6-methyl-2-p-tolyl-imidazo[1,2-a]pyridin-3-ylmethyl)-amide:
Pyridine-2-carboxylic acid methyl-(6-methyl-2-p-tolyl-imidazo[1,2-a]pyridin-3-ylmethyl)-amide:
Thiophene-2-carboxylic acid methyl-(6-methyl-2-p-tolyl-imidazo[1,2-a]pyridin-3-ylmethyl)-amide:
5-Nitro-furan-2-carboxylic acid methyl-(6-methyl-2-p-tolyl-imidazo[1,2-a]pyridin-3-ylmethyl)-amide;
2-Chloro-N-methyl-N-(6-methyl-2-p-tolyl-imidazo[1,2-a]pyridin-3-ylmethyl)-isonicotinamide;
Cyclobutanecarboxylic acid (6-methyl-2-p-tolyl-imidazo[1,2-a]pyridin-3-ylmethyl)-amide:
5-Methyl-pyrazine-2-carboxylic acid (6-methyl-2-p-tolyl-imidazo[1,2-a]pyridin-3-ylmethyl)-amide:
6-Oxo-1,4,5,6-tetrahydro-pyridazine-3-carboxylic acid (6-methyl-2-p-tolyl-imidazo[1,2-a]pyridin-3-ylmethyl)-amide;
[1,2,3]Thiadiazole-4-carboxylic acid (6-methyl-2-p-tolyl-imidazo[1,2-a]pyridin-3-ylmethyl)-amide:
N-(6-Methyl-2-p-tolyl-imidazo[1,2-a]pyridin-3-ylmethyl)-2-thiophen-2-yl-acetamide;
1-Methyl-1H-imidazole-2-carboxylic acid (6-methyl-2-p-tolyl-imidazo[1,2-a]pyridin-3-ylmethyl)-amide;
Thiazole-4-carboxylic acid (6-methyl-2-p-tolyl-imidazo[1,2-a]pyridin-3-ylmethyl)-amide;
2,5-Dimethyl-oxazole-4-carboxylic acid (6-methyl-2-p-tolyl-imidazo[1,2-a]pyridin-3-ylmethyl)-amide:
3,5-Dimethyl-isoxazole-4-carboxylic acid (6-methyl-2-p-tolyl-imidazo[1,2-a]pyridin-3-ylmethyl)-amide;
Thiazole-4-carboxylic acid methyl-(6-methyl-2-p-tolyl-imidazo[1,2-a]pyridin-3-ylmethyl)-amide;
1-(4-Dimethylamino-phenyl)-3-(6-methyl-2-p-tolyl-imidazo[1,2-a]pyridin-3-ylmethyl)-urea;
1-Ethyl-3-(6-methyl-2-p-tolyl-imidazo[1,2-a]pyridin-3-ylmethyl)-urea;
1-Isopropyl-3-(6-methyl-2-p-tolyl-imidazo[1,2-a]pyridin-3-ylmethyl)-urea;
1-Cyclopentyl-3-(6-methyl-2-p-tolyl-imidazo[1,2-a]pyridin-3-ylmethyl)-urea;
1-Cyclohexyl-3-(6-methyl-2-p-tolyl-imidazo[1,2-a]pyridin-3-ylmethyl)-urea;
1-(6-Methyl-2-p-tolyl-imidazo[1,2-a]pyridin-3-ylmethyl)-3-phenyl-urea;
(6-Methyl-2-p-tolyl-imidazo[1,2-a]pyridin-3-ylmethyl)-carbamic acid p-tolyl ester;
(6-Methyl-2-p-tolyl-imidazo[1,2-a]pyridin-3-ylmethyl)-carbamic acid prop-2-ynyl ester;
(6-Methyl-2-p-tolyl-imidazo[1,2-a]pyridin-3-ylmethyl)-carbamic acid methyl ester;
(6-Methyl-2-p-tolyl-imidazo[1,2-a]pyridin-3-ylmethyl)-carbamic acid benzyl ester;
(6-Methyl-2-p-tolyl-imidazo[1,2-a]pyridin-3-ylmethyl)-carbamic acid 4-methoxy-phenyl ester;
(6-Methyl-2-p-tolyl-imidazo[1,2-a]pyridin-3-ylmethyl)-carbamic acid ethyl ester;
(6-Methyl-2-p-tolyl-imidazo[1,2-a]pyridin-3-ylmethyl)-carbamic acid phenyl ester; and
(6-Methyl-2-p-tolyl-imidazo[1,2-a]pyridin-3-ylmethyl)-carbamic acid isopropyl ester.

Another aspect of the present invention is to provide a process for preparing the compounds of formula (I) and their pharmaceutically acceptable salts.

Another aspect of the present invention is to provide a method for treating or preventing diseases associated with $GABA_A$ receptor modulation in a mammal which comprises administering to said mammal an effective amount of a compound of formula (I) or a pharmaceutically acceptable salt thereof.

Another aspect of the present invention is to provide a method for treating or preventing diseases associated with $\alpha_1$-$GABA_A$ receptor modulation in a mammal which comprises administering to said mammal an effective amount of a compound of formula (I) or a pharmaceutically acceptable salt thereof.

Another aspect of the present invention is to provide a method for treating or preventing diseases associated with $\alpha_2$-$GABA_A$ receptor modulation in a mammal which comprises administering to said mammal an effective amount

of a compound of formula (I) or a pharmaceutically acceptable salt thereof.

Another aspect of the present invention is to provide a method for treating or preventing anxiety in a mammal which comprises administering to said mammal an effective amount of a compound of formula (I) or a pharmaceutically acceptable salt thereof.

Another aspect of the present invention is to provide a method for treating or preventing epilepsy in a mammal which comprises administering to said mammal an effective amount of a compound of formula (I) or a pharmaceutically acceptable salt thereof.

Another aspect of the present invention is to provide a method for treating or preventing sleep disorders in a mammal which comprises administering to said mammal an effective amount of a compound of formula (I) or a pharmaceutically acceptable salt thereof.

Another aspect of the present invention is to provide a method for treating or preventing insomnia in a mammal which comprises administering to said mammal an effective amount of a compound of formula (I) or a pharmaceutically acceptable salt thereof.

Another aspect of the present invention is to provide a method for inducing sedation-hypnosis in a mammal which comprises administering to said mammal an effective amount of a compound of formula (I) or a pharmaceutically acceptable salt thereof.

Another aspect of the present invention is to provide a method for inducing anesthesia in a mammal which comprises administering to said mammal an effective amount of a compound of formula (I) or a pharmaceutically acceptable salt thereof.

Another aspect of the present invention is to provide a method for modulating the necessary time to induce sleep and its duration in a mammal which comprises administering to said mammal an effective amount of a compound of formula (I) or a pharmaceutically acceptable salt thereof.

Another aspect of the present invention is to provide a method for inducing muscle relaxation in a mammal which comprises administering to said mammal an effective amount of a compound of formula (I) or a pharmaceutically acceptable salt thereof.

Another aspect of the present invention is to provide a pharmaceutical composition containing a compound of formula (I) or a pharmaceutically acceptable salt thereof in association with therapeutically inert carriers.

Another aspect of the present invention is to provide the use of a compound of formula (I) or a pharmaceutically acceptable salt thereof for preparing a medicament for treating or preventing diseases associated with $GABA_A$ receptor modulation.

Another aspect of the present invention is to provide the use of a compound of formula (I) or a pharmaceutically acceptable salt thereof for preparing a medicament for treating or preventing diseases associated with $\alpha_1$-$GABA_A$ or $\alpha_2$-$GABA_A$ receptor modulation.

Another aspect of the present invention is to provide the use of a compound of formula (I) or a pharmaceutically acceptable salt thereof for preparing a medicament for treating or preventing anxiety epilepsy, sleep disorders, insomnia, for inducing sedation-hypnosis, anesthesia or muscle relaxation or for modulating the necessary time to induce sleep and its duration.

The compounds of general formula (I) wherein $R_3$ is hydrogen and $R_4$ is a carbon group can be obtained following the synthetic strategy showed in Scheme 1.

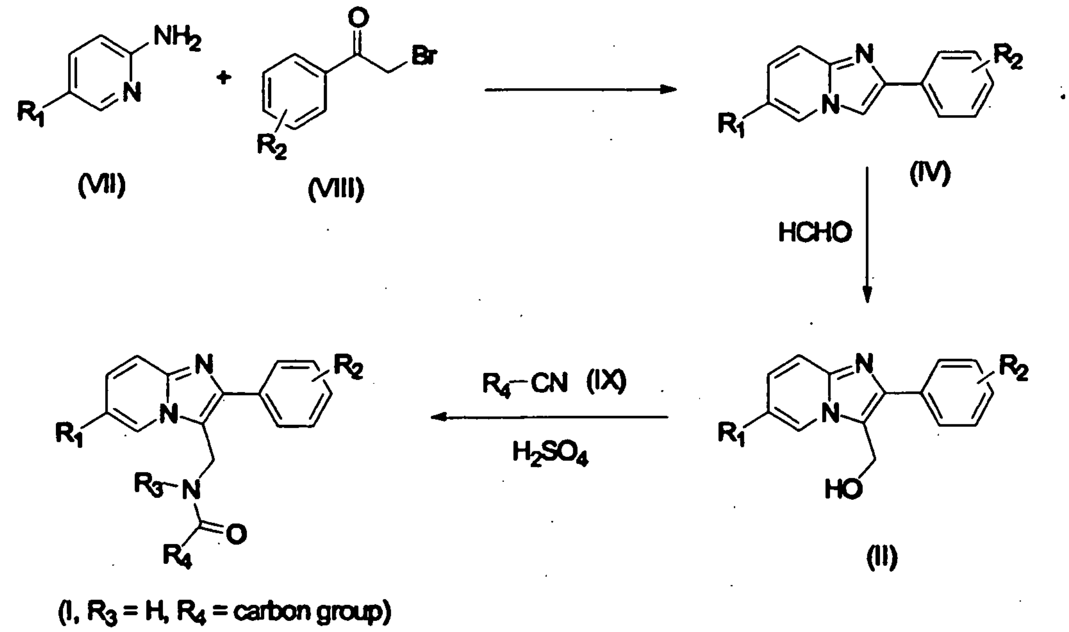


Scheme 1

The imidazopyridine (IV) is obtained by cyclization between the corresponding aminopyridine (VII) and the bromoacetophenone (VIII). This reaction is carried out by heating both components at reflux for 2-8 hours, using a polar solvent such as methanol, ethanol, butanol and the like. The final product (IV) is obtained by evaporation of the crude and crystallization of the residue with the adequate solvent.

The Mannich reaction between this imidazopyridine (IV) and formaldehyde in an acidic moiety, such as diluted acetic acid, yields the alcohol (II). The reaction is carried out by heating the mixture at 55°C for a period of 2-6 h. The solvent is removed and the residue thus obtained is suspended in dichloromethane, and stirred for 12 hours. The alcohol (II) is washed and dried.

Finally, the condensation of the alcohol (II) and the appropriate nitrile (IX) yields compounds of general formula (I), when $R_3$ is hydrogen and $R_4$ is a carbon group, by using sulphuric acid as catalyst and a polar solvent, such as acetic acid, acetonitrile, tetrahydrofurane and the like. The components are stirred and heated at reflux for 2-6 hours. The crude thus obtained is basified with ammonia and extracted with dichloromethane to yield the corresponding amide (I, $R_3$ = H, $R_4$ = carbon group).

Once the amides (I, $R_3$ = H, $R_4$ - carbon group) are obtained, the nitrogen present in this functional group can be alkylated according to a procedure which is well known by an expert skilled in organic chemistry. The reaction is shown in Scheme 2.

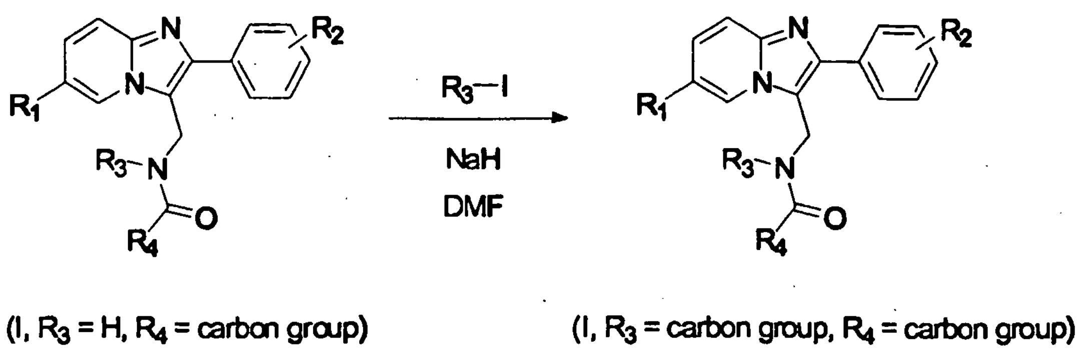


Scheme 2

The reaction is done by using sodium hydride as base and dimethylformamide as solvent under inert atmosphere. The mixture is stirred at room temperature for 1 hour, and the crude thus obtained is removed with dichloromethane. The procedure yields the corresponding N-alkylated amides (I, $R_3$ - carbon group, $R_4$ - carbon group).

We also report!the preparation of urea compounds of general formula (I) when $R_3$ is hydrogen and $R_4$ is $-NHR_5$. The synthetic strategy is shown in Scheme 3.

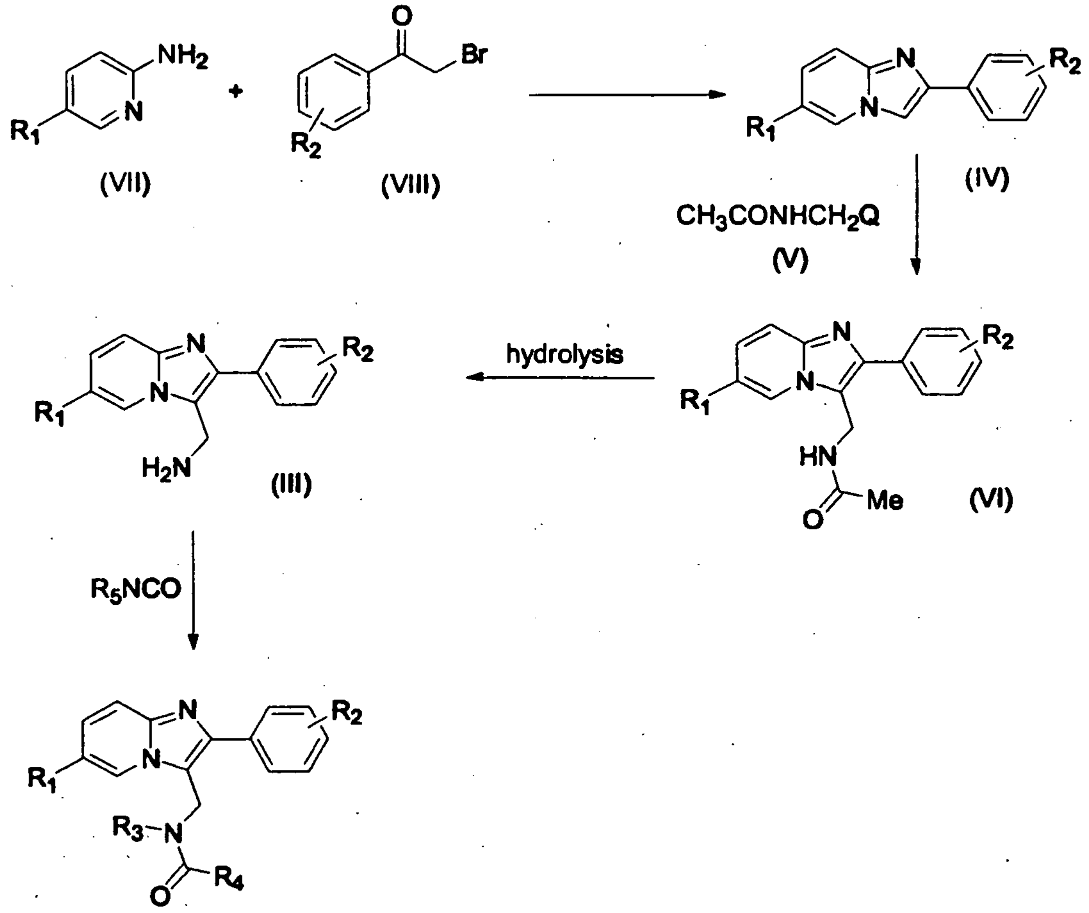


(I, $R_3$ = H, $R_4$ = $-NHR_5$)

Scheme 3

In this case, the imidazopyridine (IV) described above is treated with (V) to yield the corresponding acetamide (VI). Q is selected from the group consisting of -OH, -Oalkyl ($C_1$-$C_3$) -N+ (alkyl ($C_1$-$C_3$)) 3Cl - N+ (alkyl ($C_1$-$C_3$)) 3Br-, -N+(alkyl($C_1$-$C_3$))3I-, preferably OH. This reaction is carried out by using an acidic solvent such as acetic acid and an acid as catalyst. The reaction takes place at room temperature for 1-3 hours and then at reflux for 2-4 hours. An extraction with an organic solvent yields the corresponding acetamide (VI).

The hydrolysis of acetamides (VI) in acidic media leads to amines (III). The reaction takes place at reflux using a protic solvent such as methanol, ethanol, propanol, and the like, for a period of 30-90 min. The solvent is removed and the crude is neutralized and extracted with an organic solvent to obtain amines (III). These amines are the precursors of urea compounds (I, $R_3$ = H, $R_4$ = $-NHR_5$).

Finally, the coupling between amines (III) and isocyanates $R_5NCO$ yields the corresponding urea compounds (I, $R_3$ = H, $R_4$ = $-NHR_5$) as mentioned above. The reaction is carried out by using the appropriate isocyanate, stirring at room temperature for 20-30 hours, and using a basic solvent such as pyridine. The solvent is removed and the products are crystallized with the appropriate solvent.

In parallel, amines (III) react with chloroformiates to yield carbamates of general formula (I) when $R_3$ is hydrogen and $R_4$ is $-OR_5$, as shown in Scheme 4.

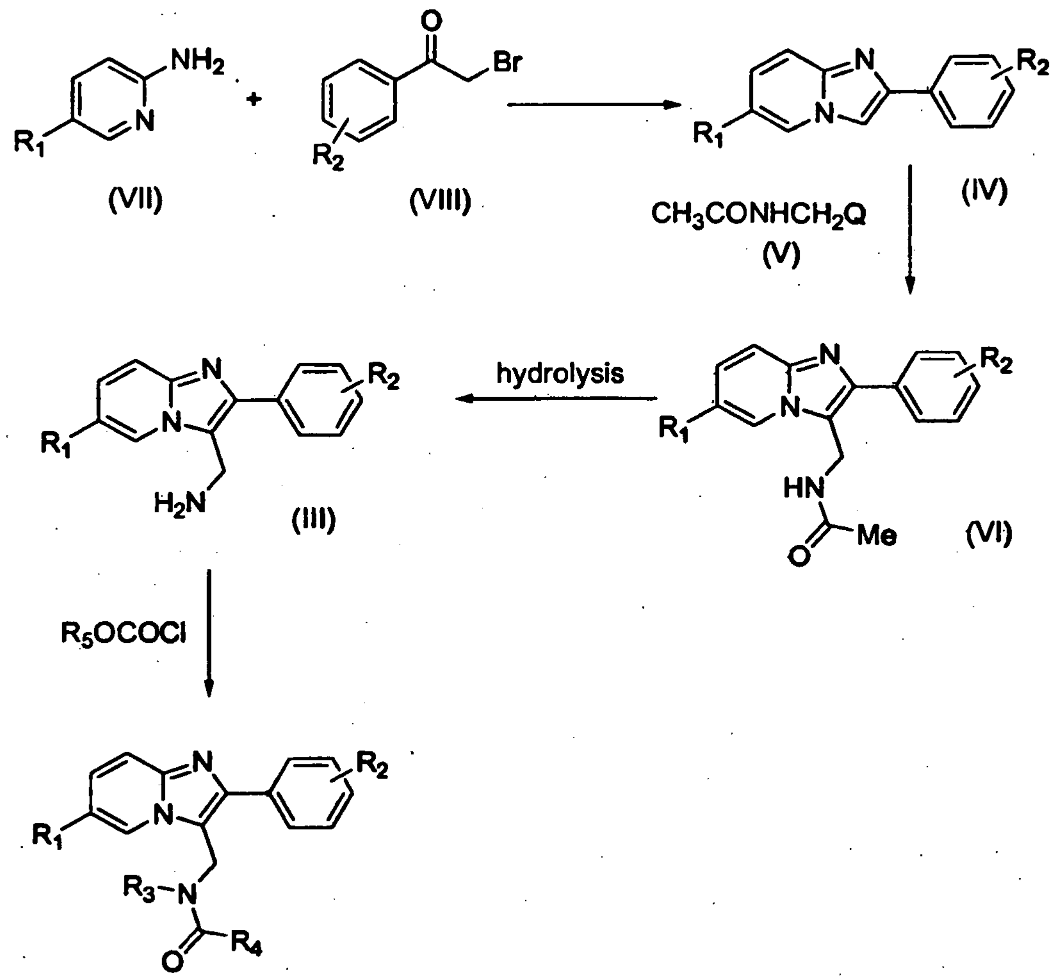


(VII) (VIII) (IV) (VI) (III)

(I, $R_3$ = H, $R_4$ = $-OR_5$)

Scheme 4

The reaction takes place at room temperature for a period of 20-30 hours. The appropriate chloroformiate reacts by using a basic solvent such as pyridine. The solvent is removed and the products are crystallized with water, and filtered off. Thus, carbamates (I, $R_3$ = H, $R_4$ = $-OR_5$) are obtained in good yields.

From the compounds of general formula (I) it is possible to obtain their pharmaceutically acceptable salts by treatment with the corresponding acids.

The applicants have discovered that the compounds of the present invention have a high affinity for $\alpha_1$- and $\alpha_2$- $GABA_A$ receptors as shown in Tables 1 and 2. These in vitro results are consistent with those in vivo results obtained in sedation-hypnosis tests (Table 3).

In accordance with the results obtained, certain compounds of the present invention have surprisingly evidenced high affinity for $\alpha_1$-$GABA_A$ receptors and interesting pharmacological activity in vivo, which have been similar to or higher than those of prior-art compounds. Moreover, some of them displayed lower affinity for $\alpha_2$-$GABA_A$ receptors, indicating increased selectivity for $\alpha_1$-$GABA_A$ versus $\alpha_2$-$GABA_A$ receptors. All these results support their use in diseases or conditions, in which preferential activity on $\alpha_1$-$GABA_A$ is desirable, such as insomnia or anesthesia, in which an induction of sleep and an induction of sedation are needed. Furthermore, lost of righting reflex has been detected in some animals administered with certain compounds of the present invention, supporting their use as anesthetic agents. Indeed, certain compounds of the present invention have demonstrated interesting affinity for $\alpha_2$-$GABA_A$ receptors, which has been similar to or higher than that of prior-art compounds. These results support their use in diseases or conditions in which preferential activity on $\alpha_2$-$GABA_A$ receptors is desirable, such as anxiety or in which an induction of muscle relaxation

is needed.

The pharmacological activity of the compounds of the present invention has been determined as shown below.

Ligand-binding assays. Determination of the affinity of test compounds for $\alpha_1$- and $\alpha_2$-$GABA_A$ receptor. Male Sprague-Dawley rats weighing 200-250 g at the time of experiment were used. After decapitation of the animal, the cerebellum (tissue that mostly contains $\alpha_1$-$GABA_A$ receptor) and spinal cord (tissue that mostly contains $\alpha_2$-$GABA_A$ receptor) were removed. The membranes were prepared according to the method by J. Lameh et al.(Prog. Neuro-Psychopharmacol. Biol. Psychiatry, 24, 979-991, 2000) and H. Noguchi et al. (Eur. J. Pharm., 434, 21-28, 2002). Once the tissues weighed, they were suspended in 50 mM Tris·HCl (pH 7.4), 1:40 (w/v), or sucrose 0.32 M in the case of spinal cord, homogenized and then centrifuged at 20,000 g for 10 min at 7°C twice. The resulting pellet was resuspended under the same conditions and centrifuged again. The pellet was finally resuspended on a minimum volume and kept at - 80°C overnight. On the next day, the process was repeated until the final pellet was resuspended at a ratio of 1:10 (w/v) in the case of cerebellum and at a ratio of 1:5 (w/v) in the case of spinal cord.

Affinity was determined by competitive tests using radiolabeled flumazenil as ligand. The tests were performed according to the methods described by S. Arbilla et al. (Eur. J. Pharmacol., 130, 257-263, 1986); and Y. Wu et al. (Eur. J. Pharmacol., 278, 125-132, 1995) using 96-well microtiter plates. The membranes containing the study receptors, flumazenil (radiolabeling at a final concentration of 1 nM) and ascending concentrations of test compounds (in a total volume of 230 μL in 50 mM [ph 7.4] Tris·HCl buffer) were incubated. Simultaneously, the membranes were only incubated with the radiolabeled flumazenil (total binding, 100%) and in the presence of an elevated concentration of unradiolabeled flumazenil (non-specific binding, % estimation of radiolabeled ligand). The reactions started on adding the radiolabeled ligand followed by incubation for 60 minutes at 4°C. At the end of the incubation period, 200 μL of reaction were transferred to a multiscreen plate (Millipore) and filtered using a vacuum manifold and then washed three times with cold test buffer. The multiscreen plates were equipped with a GF/B filter that retained the membranes containing the receptors and the radiolabeled ligand which has been bound to the receptors. After washing, the plates were left till dry. Once dried, scintillation liquid was added and left under stirring overnight. The next day the plates were counted using a Perkin-Elmer Microbeta scintillation counter.

For analysis of the results the percentage of specific binding for every concentration of test compound was calculated as follows:

$$\% \text{ specific binding} = (X-N/T-N) \times 100$$

where,

X: amount of bound ligand for every concentration of compound.

T: total binding, maximum amount bound to the radiolabeled ligand.

N: non-specific binding, amount of radiolabeled ligand bound in a non-specific way irrespective of the receptor used.

Every concentrations of compound were tested in triplicate and their mean values were used to determine the experimental values of % specific binding versus the concentration of compound. Affinity data are expressed as % inhibition at $10_{-5}$M and $10_{-7}$M-concentrations. The results of these tests are given in Tables 1 and 2.

Table 1. Affinity for $\alpha_1$-$GABA_A$ receptor

| Compound | % Inhibition $10^{-5}$M | % Inhibition $10^{-7}$M |
|---|---|---|
| Example 3 | 73.9 | 21.4 |
| Example 4 | 97.4 | 19.4 |
| Example 5 | 99.8 | 75.5 |
| Example 6 | 96.1 | 22.5 |
| Example 7 | 98.0 | 36.5 |
| Example 11 | 70.7 | 29.0 |
| Example 12 | 73.7 | 18.6 |
| Example 13 | 97.6 | 53.8 |
| Example 14 | 99.4 | 51.4 |
| Example 15 | 74.5 | 0.0 |
| Example 16 | 95.4 | 2.2 |

(continued)

| Compound | % Inhibition $10^{-5}$M | % Inhibition $10^{-7}$M |
|---|---|---|
| Example 17 | 94.5 | 0.0 |
| Example 19 | 95.5 | 0.0 |
| Example 20 | 99.8 | 65.9 |
| Example 21 | 67.9 | 3.5 |
| Example 22 | 84.8 | 62.3 |
| Example 24 | 99.8 | 75.2 |
| Example 25 | 91.9 | 26.6 |
| Example 28 | 99.8 | 73.3 |
| Example 29 | 99.7 | 84.9 |
| Example 30 | 90.5 | 4.6 |
| Example 31 | 82.5 | 1.2 |
| Example 32 | 98.0 | 14.5 |
| Example 33 | 98.4 | 37.3 |
| Example 34 | 98.8 | 39.6 |
| Example 35 | 67.1 | 31.8 |
| Example 42 | 92.7 | 12.4 |
| Example 58 | 98.8 | 6.5 |
| Example 61 | 100.0 | 85.4 |
| Example 63 | 98.5 | 36.1 |
| Example 65 | 99.5 | 83.9 |
| Example 67 | 99.6 | 84.7 |
| Example 69 | 100.4 | 93.4 |
| Example 70 | 100.4 | 79.7 |
| Example 71. | 96.4 | 21.9 |
| Example 72 | 99.8 | 73.4 |
| Example 74 | 99.9 | 95.3 |
| Example 78 | 100.2 | 97.9 |
| Example 80 | 88.9 | 0.0 |
| Example 81 | 99.6 | 69.9 |
| Example 82 | 98.7 | 33.0 |
| Example 83 | 100.0 | 93.6 |
| Example 84 | 96.6 | 10.3 |
| Example 87 | 72.2 | 1.8 |
| Example 88 | 96.2 | 0.0 |
| Example 89 | 100.3 | 88.2 |
| Example 90 | 84.5 | 0.0 |
| Example 94 | 99.5 | 78.5 |
| Example 96 | 99.9 | 80.5 |

(continued)

| Compound | % Inhibition $10^{-5}M$ | % Inhibition $10^{-7}M$ |
|---|---|---|
| Example 99 | 99.2 | 52.5 |
| Zolpidem | 94.4 | 73.6 |

Table 2. Affinity for $\alpha_2$-$GABA_A$ receptor

| Compound | % Inhibition $10^{-5}M$ | % Inhibition $10^{-7}M$ |
|---|---|---|
| Example 3 | 17.4 | 0.0 |
| Example 4 | 82.9 | 0.0 |
| Example 5 | 95.3 | 28.4 |
| Example 6 | 64.4 | 0.0 |
| Example 7 | 81.3 | 0.0 |
| Example 11 | 6.5 | 0.0 |
| Example 12 | 19.0 | 0.0 |
| Example 13 | 67.8 | 0.0 |
| Example 14 | 92.2 | 26.2 |
| Example 15 | 41.6 | 0.0 |
| Example 16 | 52.9 | 0.0 |
| Example 17 | 24.4 | 0.0 |
| Example 19 | 47.6 | 0.0 |
| Example 20 | 95.2 | 0.0 |
| Example 21 | 25.6 | 0.0 |
| Example 22 | 9.4 | 0.0 |
| Example 24 | 14.4 | 0.0 |
| Example 25 | 0.0 | 0.0 |
| Example 28 | 93.9 | 14.3 |
| Example 29 | 90.2 | 25.0 |
| Example 30 | 33.9 | 0.0 |
| Example 31 | 38.8 | 0.0 |
| Example 32 | 77.5 | 0.0 |
| Example 33 | 74.0 | 0.0 |
| Example 34 | 85.0 | 0.0 |
| Example 35 | 3.7 | 0.0 |
| Example 42 | 49.7 | 0.0 |
| Example 58 | 78.6 | 9.2 |
| Example 61 | 98.5 | 48.3 |
| Example 63 | 89.8 | 13.4 |
| Example 65 | 95.4 | 39.1 |
| Example 67 | 96.6 | 47.3 |
| Example 69 | 98.8 | 74.1 |

(continued)

| Compound | % Inhibition $10^{-5}$M | % Inhibition $10^{-7}$M |
|---|---|---|
| Example 70 | 97.7 | 25.6 |
| Example 71 | 67.8 | 7.2 |
| Example 72 | 95 | 33.1 |
| Example 74 | 98.6 | 63.0 |
| Example 78 | 98.8 | 85.9 |
| Example 81 | 94.3 | 1.5 |
| Example 83 | 93.2 | 71.1 |
| Example 89 | 98.2 | 54.5 |
| Example 90 | 31.8 | 0.0 |
| Example 94 | 94.5 | 33.2 |
| Zolpidem | 78.2 | 20.1 |

In vivo determination of predictive sedative-hypnotic action.
The in vivo effects of these compounds were assessed by a predictive sedation-hypnosis test in mice (D. J. Sanger et al., Eur.J.Pharmacol., 313, 35-42, 1996; and G. Griebel et al., Psychopharmacology, 146, 205-213, 1999).
Groups of 5-8 male CD1 mice, weighing 22-26 g at the time of test, were used. The test compounds were administered in single equimolecular intraperitoneal doses, suspended in 0.25% agar with one drop of Tween in a volume of 10 mL/kg. Control animals received the vehicle alone. Using a Smart System (Panlab,S.L., Spain) the traveled distance in cm is recorded for each mouse at 5-min intervals during a period of 30 minutes after dosing. The inhibition percentage of traveled distance of treated animals versus control animals (the first 5 min were discarded) was calculated. The results of this test are given in Table 3.

Table 3. Determination of in vivo sedative-hypnotic activity in mice.

| Compound | % Inhibition Motor Activity |
|---|---|
| Example 3 | 45.59 |
| Example 4 | 73.28 |
| Example 5 | 90.40 |
| Example 6 | 78.09 |
| Example 7 | 72.45 |
| Example 11 | 55.43 |
| Example 12 | 56.84 |
| Example 13 | 90.36 |
| Example 14 | 89.82 |
| Example 15 | 80.12 |
| Example 16 | 82.38 |
| Example 17 | 54.40 |
| Example 19 | 47.98 |
| Example 20 | 91.99 |
| Example 21 | 57.14 |
| Example 22 | 89.45 |
| Example 24 | 80.71 |

(continued)

| Compound | % Inhibition Motor Activity |
|---|---|
| Example 25 | 67.34 |
| Example 28 | 55.64 |
| Example 29 | 93.98 |
| Example 30 | 44.36 |
| Example 31 | 69.67 |
| Example 32 | 92.66 |
| Example 33 | 35.02 |
| Example 34 | 92.98 |
| Example 35 | 33.19 |
| Example 42 | 40.35 |
| Example 58 | ’94.25 |
| Example 61 | 90.55 |
| Example 63 | 74.86 |
| Example 65 | 85.22 |
| Example 67 | 78.09 |
| Example 69 | 78.41 |
| Example 70 | 90.77 |
| Example 71 | 94.08 |
| Example 72 | 73.81 |
| Example 74 | 77.37 |
| Example 78 | 88.10 |
| Example 80 | 61.89 |
| Example 81 | 84.31 |
| Example 82 | 61.93 |
| Example 83 | 93.22 |
| Example 84 | 36.32 |
| Example 87 | 48.13 |
| Example 88 | 51.87 |
| Example 89 | 74.65 |
| Example 90 | 82.12 |
| Example 94 | 89.33 |
| Example 96 | 44.04 |
| Example 99 | 13.33 |
| Zolpidem | 90.80 |

The following non-limiting examples illustrate the scope of the present invention.

**Example 1:** 6-Methyl-2-p-tolyl-imidazo[1,2-a]pyridin-4-ium bromide

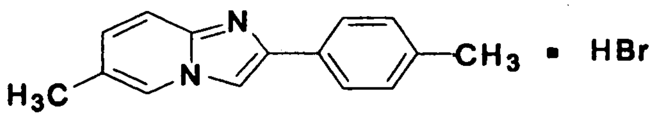

A solution of 11.53 g (106.7 mmol) of 5-methyl-pyridin-2-ylamine in 150 mL of ethanol is added to a solution of 25 g (117.3 mmol) of 2-bromo-1-p-tolyl-ethanone in 150 mL of ethanol. The resulting solution is stirred at reflux for 4 hours. The reaction is allowed to cool, and the solvent is removed in vacuo. The yellow solid obtained is dissolved in 30 mL of hot ethanol, and 40 mL of acetone are added. The solid obtained is filtered off, washed with acetone and dried over calcium chloride to give 20.0 g (65.9 mmol, yield: 62%) of 6-methyl-2-p-tolyl-imidazo[1,2-a]pyridin-4-ium bromide as a white solid.
$^1$H NMR (400 MHz, DMSO-$d_6$) : δ 8.31-7.10 (Ar, 8H, m), 2.36 (Ph-Me, 3H, s), 2.31 (Me, 3H, s). MS (ES) m/z = 223 (MH+)
HPLC = 100%

**Example 2:** (6-Methyl-2-p-tolyl-imidazo[1,2-a]pyridin-3-yl)-methanol

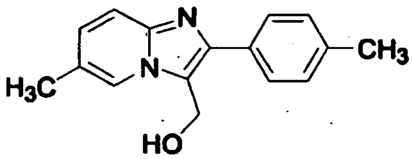

A solution of 6 mL (81 mmol) of formaldehyde in water (37%) is added to a solution of 4 g (18 mmol) of 6-methyl-2-p-tolyl-imidazo[1,2-a]pyridin-4-ium bromide in 30 mL of acetic acid. The reaction is heated at 55°C for 4 h. The resulting solution is allowed to cool, and the solvent is removed in vacuo. To the corresponding residue are added 20 mL of ammonia (25%) and 30 mL of dichloromethane, and the suspension is stirred overnight. The solid obtained is filtered off, washed with dichloromethane and water and dried over calcium chloride, to yield 2.8 g (11 mmol, 62%) of (6-methyl-2-p-tolyl-imidazo[1,2-a]pyridin-3-yl)-methanol as a white solid.
$^1$H NMR (400 MHz, DMSO-$d_6$) : δ 8.23-7.13 (Ar, 7H, m), 5.33 (OH, 1H, t, *J*= 5.2 Hz), 4.85 ($CH_2$, 2H, d, *J*= 5.2 Hz), 2.35 (Ph-Me, 3H, s), 2.33 (Me, 3H, s). MS (ES) m/z = 253 (MH+)
HPLC = 98.3%

**Example 3:** 4-Dimethylamino-N-(6-methyl-2-p-tolyl-imidazo [1,2-a]pyridin-3-ylmethyl)-benzamide

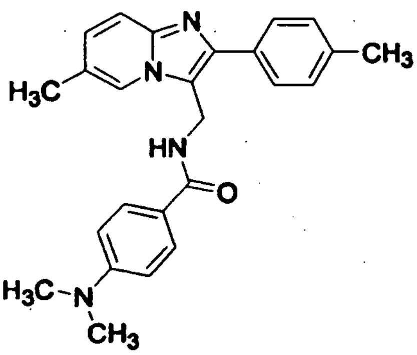

To a solution of 1 eq of (6-methyl-2-p-tolyl-imidazo[1,2-a]pyridin-3-yl)-methanol in acetic acid is added a solution of 4-dimethylaminobenzonitrile (2 eq) in acetic acid. Then, 4 eq of sulphuric acid are added slowly. The mixture is heated at room temperature for 1.5 h, and then at reflux for 2 h. The reaction is allowed to cool and is basified with ammonia

(25%). The suspension is extracted with dichloromethane. The organic phase is dried over magnesium sulphate and filtered off. The solvent is removed in vacuo to give 0.96 eq of 4-Dimethylamino-N-(6-methyl-2-p-tolyl-imidazo[l,2-a]pyridin-3-ylmethyl)-benzamide.
$^1H$ NMR (400 MHz, DMSO-$d_6$) : δ 8.58 (NH, 1H, t, *J*= 5.2 Hz), 8.29-6.65 (Ar, 11H, m), 4.87 ($CH_2$, 2H, d, *J*= 5.2 Hz), 2.94 (N-Me, 6H, s), 2.34 (Ph-Me, 3H, s).
MS (ES) m/z = 399 (MH+)
HPLC = 97.1%

The compounds of examples 4-21 were prepared according to this procedure starting from (6-methyl-2-p-tolyl-imidazo[1,2-a]pyridin-3-yl)-methanol and the corresponding nitrile.

**Example 4:** Furan-2-carboxylic acid (6-methyl-2-p-tolyl-imidazo[1,2-a]pyridin-3-ylmethyl)-amide

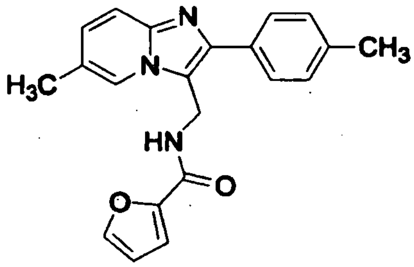

Yield: 47%
$^1H$ NMR (400 MHz, DMSO-$d_6$) : δ 8.17 (NH,. 1H, m), 7.65-6.11 (Ar, 10H, m), 4.51 ($CH_2$, 2H, m), 2.33 (Ph-Me, 3H, s), 2.29 (Me, 3H, s).
MS (ES) m/z = 346 (MH+)
HPLC = 82.7%

**Example 5:** Pyridine-2-carboxylic acid (6-methyl-2-p-tolyl-imidazo[1,2-a]pyridin-3-ylmethyl)-amide

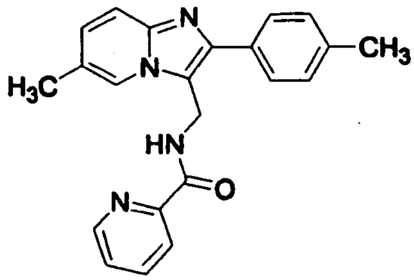

Yield: 17%
$^1H$ NMR (400 MHz, DMSO-$d_6$): δ 9.36 (NH, 1H, t, *J*= 5.6 Hz), 8.61-7.12 (Ar, 14H, m), 4.95 ($CH_2$, 2H, d, J= 5.6 Hz), 2.34 (Ph-Me, 3H, s), 2.29 (Me, 3H, s). MS (ES) m/z = 357 (MH+)
HPLC = 96.7%

**Example 6:** 1,5-Dimethyl-1H-pyrrole-2-carboxylic acid (6-methyl-2-p-tolyl-imidazo[1,2-a]pyridin-3-ylmethyl)-amide

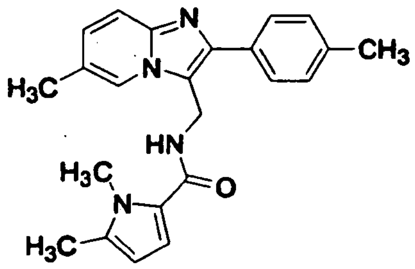

Yield: 14%
$^1$H NMR (400 MHz, DMSO-$d_6$) : δ 8.31 (NH, 1H, m), 7.66-6.99 (Ar, 9H, m), 4.64 ($CH_2$, 2H, m), 3.28. (N-Me, 3H, s), 2.34 (Ph-Me, 3H, s), 2.17 (Me, 3H, s), 1.34 (Mepyrrole, 3H, s).
MS (ES) m/z = 373 (MH+)
HPLC = 97.9%

**Example 7:** N-(6-Methyl-2-p-tolyl-imidazo[1,2-a]pyridin-3-ylmethyl)-isonicotinamide

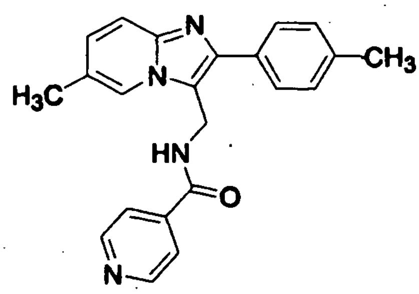

Yield: 19%
$^1$H NMR (400 MHz, DMSO-$d_6$) : δ 9.23 (NH, 1H, t, *J*= 4.8 Hz), 8.70-7.14 (Ar, 11H, m), 4.93 ($CH_2$, 2H, d, *J*= 4.8 Hz), 2.34 (Ph-Me, 3H, s), 2.3 (Me, 3H, s). MS (ES) m/z = 357 (MH+)
HPLC = 92.9%

**Example 8:** N-(6-Methyl-2-p-tolyl-imidazo[1,2-a]pyridin-3-ylmethyl)-4-nitro-benzamide

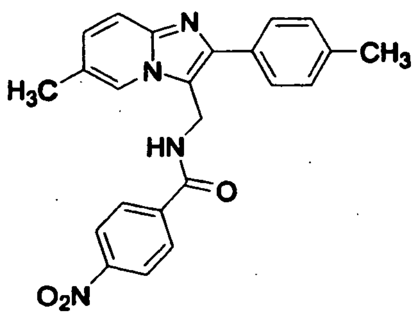

Yield: 29%
$^1$H NMR (400 MHz, DMSO-$d_6$) : δ 9.28 (NH, 1H, t, *J*= 3.6 Hz), 8.29-7.14 (Ar, 11H, m), 4 . 94 (-$CH_2$, 2H, d, *J*= 3.6 Hz), 2.34 (Ph-Me, 3H, s), 2.3 (Me, 3H, s). MS (ES) m/z = 401 (MH+)
HPLC = 98.8%

**Example 9:** Thiophene-2-carboxylic acid (6-methyl-2-p-tolyl-imidazo[1,2-a]pyridin-3-ylmethyl)-amide

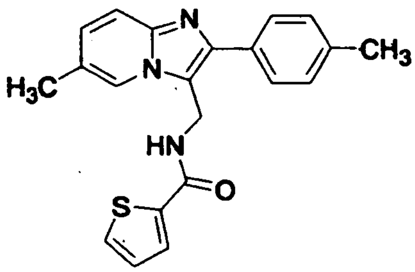

Yield: 8%
$^1$H NMR (400 MHz, DMSO-$d_6$): δ 8.94 (NH, 1H, t, *J*= 5.2 Hz), 8.26-7.10 (Ar, 10H<- m), 4.89 ($CH_2$, 2H, d, *J*= 5.2 Hz), 2.34 (Ph-Me, 3H, s), 2.3 (Me, 3H, s). MS (ES) m/z = 362 (MH+)
HPLC = 92.1%

**Example 10:** N-(6-Methyl-2-p-tolyl-imidazo(1,2-a]pyridin-3-ylmethyl)-4-trifluoromethyl-benzamide

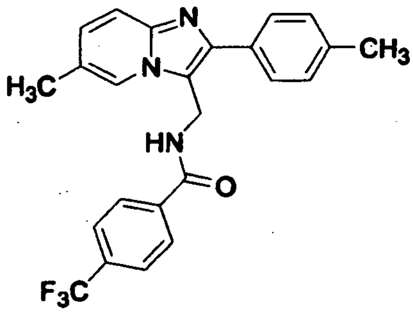

Yield: 89%
$^1$H NMR (400 MHz, DMSO-$d_6$) : δ 9.18 (NH, 1H, t, *J*= 5.2 Hz), 8.27-7.14 (Ar, 11H, m), 4.94 ($CH_2$, 2H, d, *J*= 5.2 Hz), 2.34 (Ph-Me, 3H, s), 2.3 (Me, 3H, s). MS (ES) m/z = 424 (MH+)
HPLC = 98.5%

**Example 11:** 4-Methoxy-N-(6-methyl-2-p-tolyl-imidazo[1,2-a]pyridin-3-ylmethyl)-benzamide

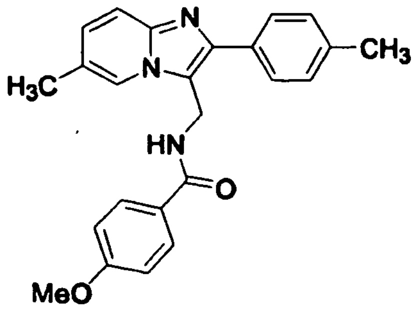

Yield: 14%
$^1H$ NMR (400 MHz, DMSO-$d_6$): δ 8.8 (NH, 1H, t, *J*= 5.2 Hz), 8.28-6.95 (Ar, 11H, m), 4.89 ($CH_2$, 2H, d, *J*= 5.2 Hz), 3.78 (MeO, 3H, s), 2.34 (Ph-Me, 3H, s), 2.29 (Me, 3H, s).
MS (ES) m/z = 386 (MH+)
HPLC = 98.3%

**Example 12:** 4-Acetyl-N-(6-methyl-2-p-tolyl-imidazo[1,2-a] pyridin-3-ylmethyl)-benzamide

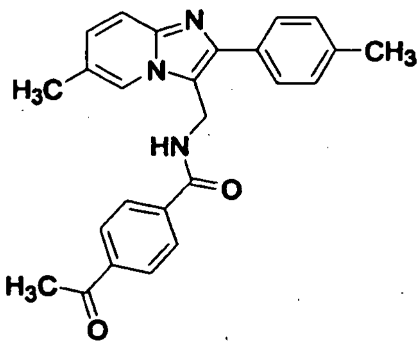

Yield: 17%
$^1H$ NMR (400 MHz, DMSO-$d_6$) : δ 9.14 (NH, 1H, t, *J*= 4.8 Hz), 8.28-7.14 (Ar, 11H, m), 4.93 ($CH_2$, 2H, d, *J*= 4.8 Hz), 2.6 (Me-CO, 3H, s), 2.34 (Ph-Me, 3H, s), 2.3 (Me, 3H, s).
MS (ES) m/z = 398 (MH+)
HPLC = 94.9%

**Example 13:** Cyclopropanecarboxylic acid (6-methyl-2-p-tolyl-imidazo[1,2-a]pyridin-3-ylmethyl)-amide

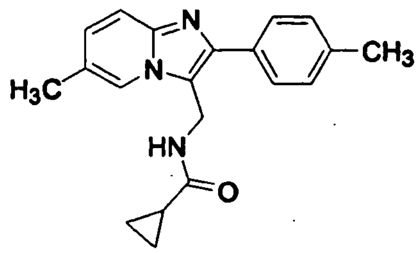

Yield: 60%
$^1H$ NMR (400 MHz, DMSO-$d_6$) : δ 8.6 (NH, 1H, t, *J*= 5.2 Hz), 8.16-7.13 (Ar, 7H, m), 4.72 ($CH_2$, 2H, d, *J*= 5.2 Hz), 2.35 (Ph-Me, 3H, s), 2.3 (Me, 3H, s), 1.59 (CH, 1H, m), 0.76 ($CH_2CH_2$, 4H, m). MS (ES) m/z = 320 (MH+)
HPLC = 99.3%

**Example 14:** 5-Nitro-furan-2-carboxylic acid (6-methyl-2-p-tolyl-imidazo[1,2-a]pyridin-3-ylmethyl)-amide

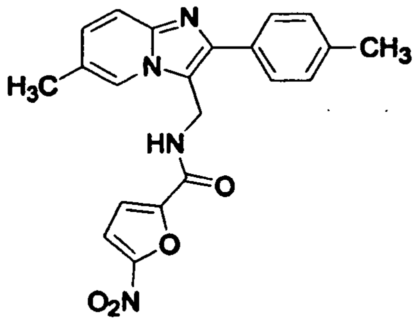

Yield: 27%
$^1H$ NMR (400 MHz, DMSO-$d_6$) : δ 9.38 (NH, 1H, m), 8.25-7.15 (Ar, 9H, m), 4.91 ($CH_2$, 2H, d, *J*= 4 Hz), 2.34 (Ph-Me, 3H, s), 2.3 (Me, 3H, s). MS (ES) m/z = 391 (MH+)
HPLC = 97.9%

**Example 15:** 3-Methyl-furan-2-carboxylic acid (6-methyl-2-p-tolyl-imidazo[1,2-a]pyridin-3-ylmethyl)-amide

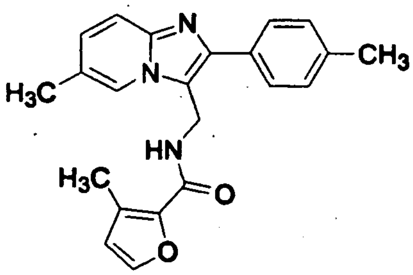

Yield: 3%
$^1H$ NMR (400 MHz, DMSO-$d_6$) δ 8.74 (NH, 1H, t, *J*= 5.2 Hz), 8.25-6.21 (Ar, 9H, m), 5.85 ($CH_2$, 2H, d, *J*= 5.2 Hz), 2.34 (Ph-Me, 3H, s), 2.29 (Me, 3H, s), 1.03 (Mefurane, 3H, s).
MS (ES) m/z = 360 (MH+)
HPLC = 93.7%

**Example 16:** 3-Methyl-thiophene-2-carboxylic acid (6-methyl-2-p-tolyl-imidazo[1,2-a]pyridin-3-ylmethyl)-amide

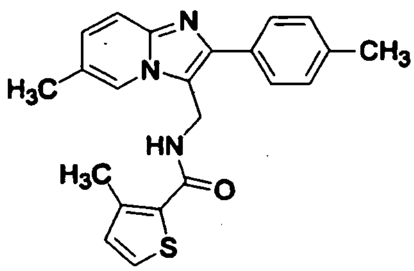

Yield: 29%
$^1H$ NMR (400 MHz, DMSO-$d_6$) : δ8.6 (NH, 1H, m), 8.3-6.9 (Ar, 9H, m), 4.87 ($CH_2$, 2H, d, *J*= 5.2 Hz), 2.37 (Me, 3H, s), 2.34 (Ph-Me, 3H, s), 2.3 (Me, 3H, s).
HPLC = 87.1%

**Example 17:** 2-Chloro-N-(6-methyl-2-p-tolyl-imidazo[1,2-a]pyridin-3-ylmethyl)-isonicotinamide

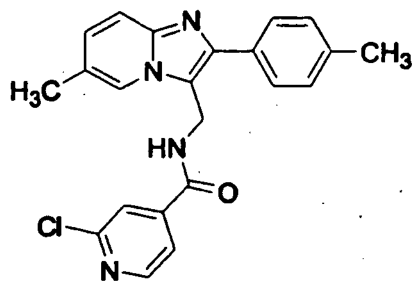

Yield: 15%
$^1$H NMR (400 MHz, DMSO-$d_6$) : δ 9.29 (NH, 1H, m), 8.56-7.16 (Ar, 10H, m), 4.92 ($CH_2$, 2H, d, *J*= 4.4 Hz), 2.34 (Ph-Me, 3H, s), 2.31 (Me, 3H, s).
HPLC = 99.2%

**Example 18:** 2,3,5,6-Tetrafluoro-N-(6-methyl-2-p-tolyl-imidazo[1,2-a]pyridin-3-ylmethyl)-isonicotinamide

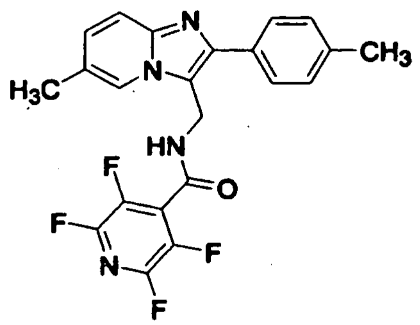

Yield: 13%
$^1$H NMR (400 MHz, DMSO-$d_6$) : δ 9.65 (NH, 1H, m), 8.19 (Ar, 7H, m), 4.99 ($CH_2$, 2H, m), 2.36 (Ph-Me, 3H, s), 2.32 (Me, 3H, s).
HPLC = 96.9%

**Example 19:** Quinoline-2-carboxylic acid (6-methyl-2-p-tolyl-imidazo[1,2-a]pyridin-3-ylmethyl)-amide

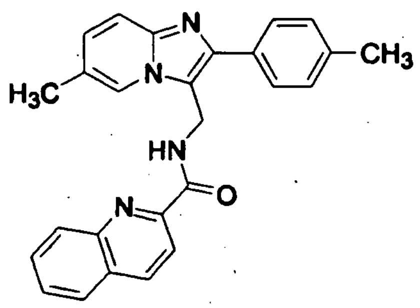

Yield: 17%
$^1H$ NMR (400 MHz, DMSO-$d_6$) : δ 9.47 (NH, 1H, t, *J*= 4.8 Hz), 8.57-7.13 (Ar, 13H, m), 5.05 ($CH_2$, 2H, d, *J*= 4.8 Hz), 2.34 (Ph-Me, 3H, s), 2.29 (Me, 3H, s). MS (ES) m/z = 407 (MH+)
HPLC = 90.5%

**Example 20:** 3,5-Difluoro-pyridine-2-carboxylic acid (6-methyl-2-p-tolyl-imidazo[1,2-a]pyridin-3-ylmethyl)-amide

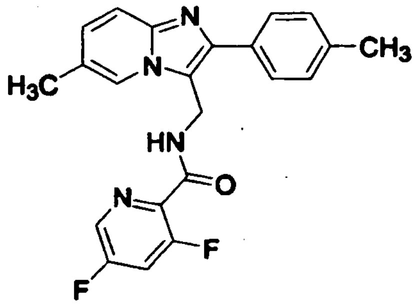

Yield: 86%
$^1H$ NMR (400 MHz, DMSO-$d_6$) δ 9.28 (NH, 1H, t, *J*= 5.2 Hz), 8.54-7.14 (Ar, 9H, m), 4.92 ($CH_2$, 2H, d, *J*= 5.2 Hz), 2.35 (Ph-Me, 3H, s), 2.3 (Me, 3H, s).
MS (ES) m/z = 393 (MH+)
HPLC = 96.6%

**Example 21:** 6-Methoxy-benzothiazole-2-carboxylic acid (6-methyl-2-p-tolyl-imidazo[1,2-a]pyridin-3-ylmethyl)-amide

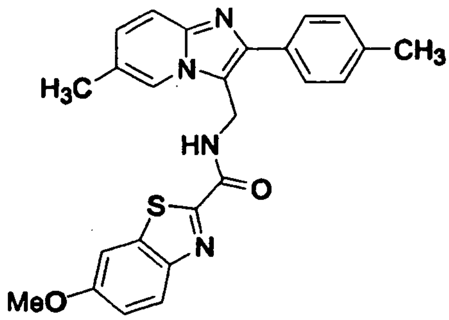

Yield: 10%
$^1H$ NMR (400 MHz, DMSO-$d_6$) : δ 8.18 (NH, 1H, m), 8.02-7.12 (Ar, 10H, m), 4.67 ($CH_2$, 2H, m), 4.06 (MeO, 3H, s), 2.27 (Ph-Me, 3H, s), 2.17 (Me, 3H, s). MS (ES) m/z = 443 (MH+)
HPLC = 100%

**Example 22:** 4-Dimethylamino-N-methyl-N-(6-methyl-2-p-tolyl-imidazo[1,2-a]pyridin-3-ylmethyl)-benzamide

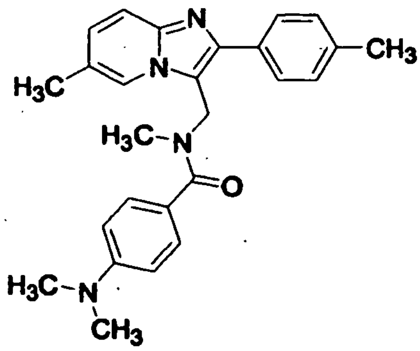

To a solution of 4-dimethylamino-N-(6-methyl-2-p-tolyl-imidazo[1,2-a]pyridin-3-ylmethyl)-benzamide (1 eq) in dry DMF are added 1.2 eq of NaH (60%) under argon. The suspension is stirred for 10 min at room temperature. Then 1.1 eq of MeI are added and the corresponding mixture is stirred for 1 h at room temperature. After this period, 0.5 N NaOH is added. The mixture is extracted with dichloromethane. The organic layer is dried over magnesium sulphate and the solvent is removed in vacuo to obtain 0.58 eq of 4-dimethylamino-N-methyl-N-(6-methyl-2-p-tolyl-imidazo[1,2-a]pyridin-3-ylmethyl)-benzamide.
$^1$H NMR (400 MHz, DMSO-$d_6$): δ 8.23-6.67 (Ar, 11H, m), 5.19 ($CH_2$, 2H, s), 2.92 ($NMe_2$, 6H, s), 2.58 (N-Me, 3H, s), 2.34 (Ph-Me, 3H, s), 2.28 (Me, 3H, s).
MS (ES) m/z = 413 (MH+)
HPLC = 90.8%

The compounds of examples 23-36 were prepared following this procedure starting from the corresponding N-dealkylated amides.

**Example 23:** 4-Isobutyryl-N-methyl-N-(6-methyl-2-p-tolyl-imidazo[1,2-a]pyridin-3-ylmethyl)-benzamide

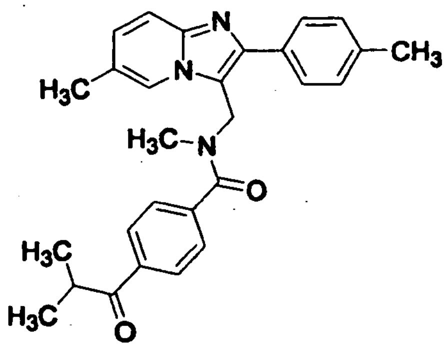

Yield: 20%
$^1$H NMR (400 MHz, DMSO-$d_6$) : δ 8.31-7.18 (Ar, 11H, m), 5.27 ($CH_2$, 2H, s), 3.64 (CH, 1H, hept, *J*= 6.8 Hz), 2.46 (N-Me, 3H, s), 2.35 (Ph-Me, 3H, s), 2.32 (Me, 3H, s), 1.09 (Me, 6H, d, *J*= 6.8 Hz).
MS (ES) m/z = 440 (MH+)
HPLC = 83.7%

**Example 24:** Cyclopropanecarboxylic acid methyl-(6-methyl-2-p-tolyl-imidazo[1,2-a]pyridin-3-ylmethyl)-amide

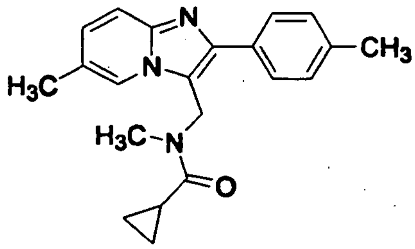

Yield: 100%
$^1H$ NMR (400 MHz, DMSO-$d_6$): δ 8.08-7.14 (Ar, 7H, m), 5.09 ($CH_2$, 2H, s), 2.72 (N-Me, 3H, s), 2.35 (Ph-Me, 3H, s), 2.25 (Me, 3H, s), 1.89 (CH, 1H, m), 0.8 ($CH_2$, 4H, m).
MS (ES) m/z = 334 (MH+)
HPLC = 98.6%

**Example 25:** 4-Methoxy-N-methyl-N-(6-methyl-2-p-tolyl-imidazo[1,2-a]pyridin-3-ylmethyl)-benzamide

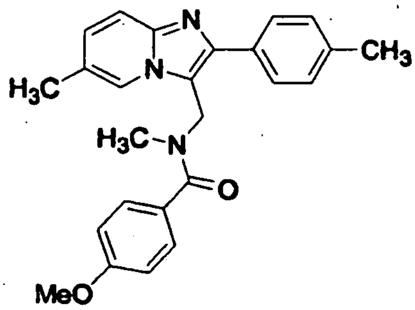

Yield: 93%
$^1H$ NMR (400 MHz, DMSO-$d_6$): δ 9.45-7.68 (Ar, 11H, m), 5.2 ($CH_2$, 2H, s), 3.77 (MeO, 3H, s), 2.55 (N-Me, 3H, s), 2.35 (Ph-Me, 3H, s), 2.3 (Me, 3H, m).
MS (ES) m/z = 400 (MH+)
HPLC = 95%

**Example 26:** N-Methyl-N-(6-methyl-2-p-tolyl-imidazo[1,2-a] pyridin-3-ylmethyl)-4-trifluoromethyl-benzamide

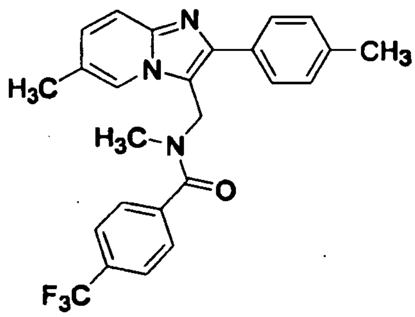

Yield: 100%
$^1H$ NMR (400 MHz, DMSO-$d_6$): δ 8.30-7.18 (Ar, 11H, m), 5.27 ($CH_2$, 2H, s), 2.5 (N-Me, 3H, s), 2.35 (Ph-Me, 3H, s), 2.32 (Me, 3H, s). MS (ES) m/z = 438 (MH+)

HPLC = 95.9%

**Example 27:** N-Methyl-N-(6-methyl-2-p-tolyl-imidazo[1,2-a] pyridin-3-ylmethyl)-isonicotinamide

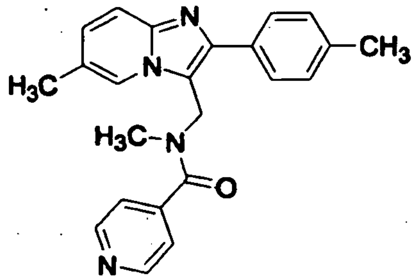

Yield: 22%
$^{1}H$ NMR (400 MHz, DMSO-$d_6$): δ 8.63-7.19 (Ar, 11H, m), 5.25 ($CH_2$, 2H, s), 2.46 (N-Me, 3H, s), 2.35 (Ph-Me, 3H, s), 2.32 (Me, 3H, s).
MS (ES) m/z = 371 (MH+)
HPLC = 94.6%

**Example 28:** Pyridine-2-carboxylic acid methyl- (6-methyl-2-p-tolyl-imidazo[1,2-a]pyridin-3-ylmethyl)-amide

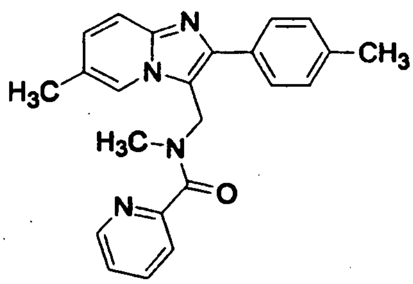

Yield: 77%
$^{1}H$ NMR (400 MHz, DMSO-$d_6$) : δ 8.53-7.18 (Ar, 11H, m), 5.27 ($CH_2$, 2H, s), 2.54 (N-Me, 3H, s), 2.33 (Ph-Me, 3H, s), 2.3 (Me, 3H, s).
MS (ES) m/z = 371 (MH+)
HPLC = 80.6%

**Example 29:** Thiophene-2-carboxylic acid methyl-(6-methyl-2-p-tolyl-imidazo[1,2-a]pyridin-3-ylmethyl)-amide

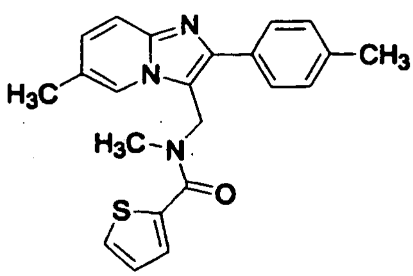

Yield: 100%
$^1H$ NMR (400 MHz, DMSO-$d_6$) : δ 8.22-7.09 (Ar, 10H, m), 5.26 ($CH_2$, 2H, s), 2.77 (N-Me, 3H, s), 2.34 (Ph-Me, 3H, s), 2.28 (Me, 3H, s).
MS (ES) m/z = 376 (MH+)
HPLC = 87%

**Example 30:** 4,N-Dimethyl-N-(6-methyl-2-p-tolyl-imidazo [1,2-a]pyridin-3-ylmethyl)-benzamide

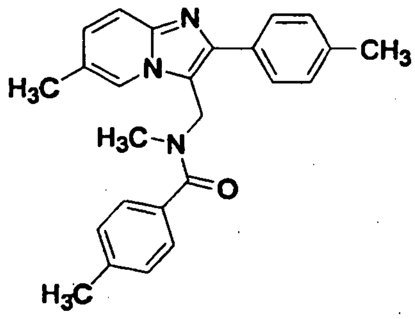

Yield: 83%
$^1H$ NMR (400 MHz, DMSO-$d_6$) : δ 7.67-7.16 (Ar, 11H, m), 5.22 ($CH_2$, 2H, s), 2.52 (N-Me, 3H, s), 2.34 (Ph-Me, 3H, s), 2.31 (Ph-Me, 3H, s), 2.3 (Me, 3H, s).
MS (ES) m/z = 384 (MH+)
HPLC = 99%

**Example 31:** N-Methyl-N-(6-methyl-2-p-tolyl-imidazo[1,2-a] pyridin-3-ylmethyl)-4-nitro-benzamide

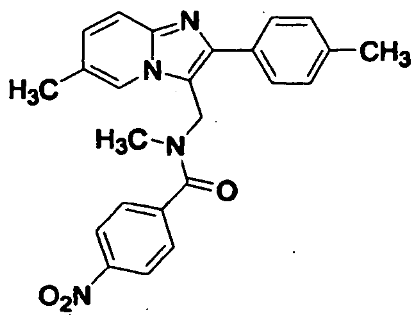

Yield: 48%
$^1H$ NMR (400 MHz, DMSO-$d_6$): δ 8.30-7.19 (Ar, 11H, m), 5.27 ($CH_2$, 2H, s), 2.49 (N-Me, 3H, s), 2.34 (Ph-Me, 3H, s), 2.32 (Me, 3H, s).
MS (ES) m/z = 415 (MH+)
HPLC = 100%

**Example 32:** 5-Nitro-furan-2-carboxylic acid methyl-(6-methyl-2-p-tolyl-imidazo[1,2-a]pyridin-3-ylmethyl)-amide

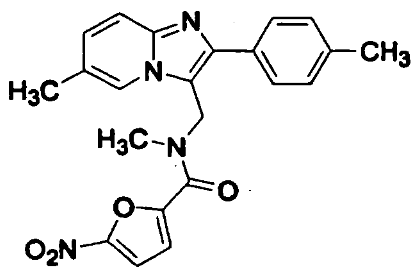

Yield: 42%
$^{1}H$ NMR (400 MHz, DMSO-$d_6$) : δ 8.26-7.19 (Ar, 9H, m) , 5.26 ($CH_2$, 2H, s), 2.82 (N-Me, 3H, s), 2.34 (Ph-Me, 3H, s), 2.3 (Me, 3H, s).
MS (ES) m/z = 405 (MH+)
HPLC = 98.8%

**Example 33:** 3,5-Difluoro-pyridine-2-carboxylic acid methyl-(6-methyl-2-p-tolyl-imidazo[1,2-a]pyridin-3-ylmethyl)-amide

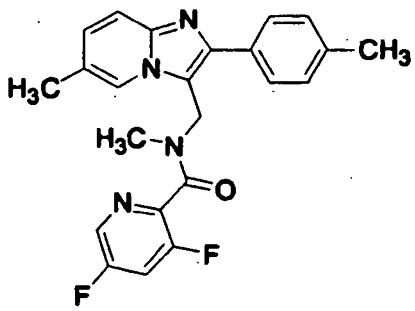

Yield: 27%
$^{1}H$ NMR (400 MHz, DMSO-$d_6$): δ 8.55-7.20 (Ar, 9H, m), 5.3 ($CH_2$, 2H, s), 2.46 (N-Me, 3H, s), 2.34 (Ph-Me, 3H, s), 2.3 (Me, 3H, s).
MS (ES) m/z = 407 (MH+)
HPLC = 96.6%

**Example 34:** 2-Chloro-N-methyl-N-(6-methyl-2-p-tolyl-imidazo [1,2-a]pyridin-3-ylmethyl)-isonicotinamide

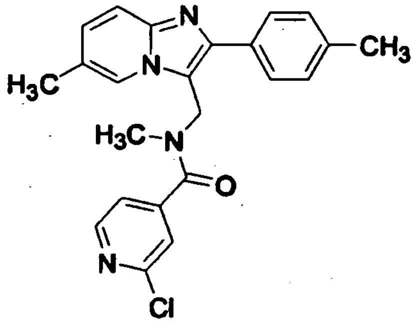

Yield: 61%
$^{1}H$ NMR (400 MHz, DMSO-$d_6$) : δ 8.51-7.22 (Ar, 10H, m), 5.26 ($CH_2$, 2H, s), 2.48 (N-Me, 3H, s), 2.38 (Ph-Me, 3H, s),

2.36 (Me, 3H, s).
MS (ES) m/z = 405 (MH+)
HPLC = 83.6%

**Example 35:** Quinoline-2-carboxylic acid methyl-(6-methyl-2-p-tolyl-imidazo[1,2-a]pyridin-3-ylmethyl)-amide

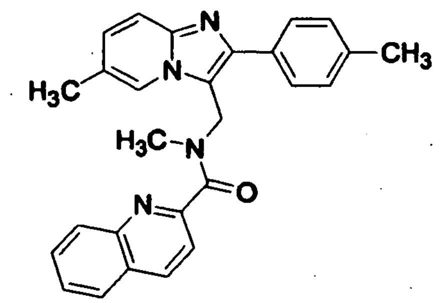

Yield: 60%
$^1$H NMR (400 MHz, DMSO-$d_6$) : δ 8.77-7.16 (Ar, 13H, m), 5. 35 ($CH_2$, 2H, s), 2.65 (N-Me, 3H, s), 2.36 (Ph-Me, 3H, s), 2.33 (Me, 3H, s).
MS (ES) m/z = 421 (MH+)
HPLC = 96.6%

**Example 36:** 6-Methoxy-3-methyl-2-[methyl-(6-methyl-2-p-tolyl-imidazo[1,2-a]pyridin-3-ylmethyl)-carbamoyl]-benzothiazol-3-ium iodide

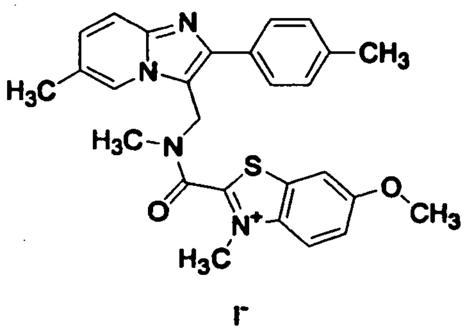

Yield: 85%
$^1$H NMR (400 MHz, DMSO-$d_6$): δ 8.53-7.29 (Ar, 10H, m), 4.73 ($CH_2$, 2H, s), 3.94 (MeO, 3H, s), 3.44 (Methiazole, 3H, s), 2.97 (N-Me, 3H, s), 2.37 (Ph-Me, 3H, s), 2.32 (Me, 3H, s).
MS (ES) m/z = 471 (MH+)
HPLC = 97.6%

**Example 37:** N-Ethyl-N-(6-methyl-2-p-tolyl-imidazo[1,2-a] pyridin-3-ylmethyl)-4-trifluoromethyl-benzamide

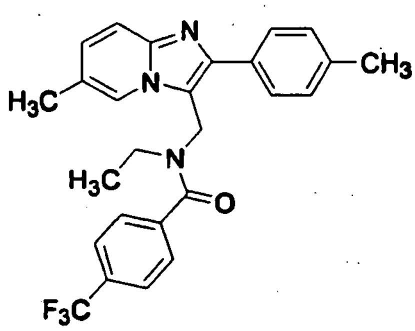

To a solution of N-(6-methyl-2-p-tolyl-imidazo[1,2-a]pyridin-3-ylmethyl)-4-trifluoromethyl-benzamide (1 eq) in dry DMF are added 1.2 eq of NaH (60%) under argon. The suspension is stirred for 10 min at room temperature. Then 1.1 eq of EtI are added and the corresponding mixture is stirred for 1 h at room temperature. After this period, 0.5 N NaOH is added. The mixture is extracted with dichloromethane. The organic layer is dried over magnesium sulphate and the solvent is removed in vacuo to obtain 0.47 eq of N-ethyl-N-(6-methyl-2-p-tolyl-imidazo[1,2-a]pyridin-3-ylmethyl)-4-trifluoromethyl-benzamide.
Yield: 47%
$^1$H NMR (400 MHz, DMSO-$d_6$): δ 8.26-7.19 (Ar, 11H, m), 5.32 ($CH_2$, 2H, s), 2.7 ($CH_2$-Me, 2H, m), 2.36 (Ph-Me, 3H, s), 2.33 (Me, 3H, s) , 0.55 (Me-$CH_2$, 3H, m).
MS (ES) m/z = 452 (MH+)
HPLC = 99.8%

The compound of example 38 was prepared according to this procedure starting from the corresponding N-dealkylated amide.

**Example 38:** Cyclopropanecarboxylic acid ethyl-(6-methyl-2-p-tolyl-imidazo[1,2-a]pyridin-3-ylmethyl)-amide

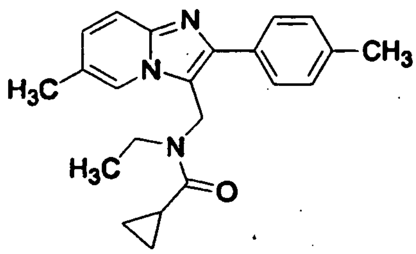

Yield: 41%
$^1$H NMR (400 MHz, DMSO-$d_6$) : δ 8.07-7.14 (Ar, 7H, m) , 5.12 ($CH_2$, 2H, s), 3.08 ($CH_2$-Me, 2H, q, *J*= 7.2 Hz), 2.36 (Ph-Me, 3H, s), 2.25 (Me, 3H, s), 1.85 (CH, 3H, m), 0.81 (Me-$CH_2$, 3H, t, *J*= 7.2 Hz) , 0.73 ($CH_2$, 4H, m). MS (ES) m/z = 348 (MH+)
HPLC = 92.3%

**Example 39:** N-(6-Methyl-2-p-tolyl-imidazo[1,2-a]pyridin-3-ylmethyl)-N-propyl-4-trifluoromethyl-benzamide

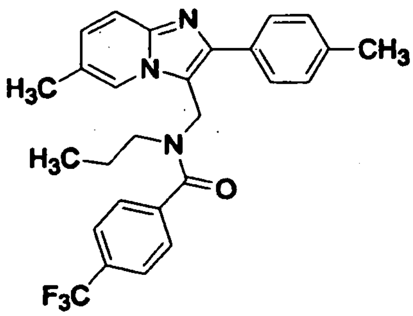

To a solution of N-(6-methyl-2-p-tolyl-imidazo[1,2-a]pyridin-3-ylmethyl)-4-trifluoromethyl-benzamide (1 eq) in dry DMF are added 1.2 eq of NaH (60%) under argon. The suspension is stirred for 10 min at room temperature. Then 1.1 eq of PrI are added and the corresponding mixture is stirred for 1h at room temperature. After this period, 0.5 N NaOH is added. The mixture is extracted with dichloromethane. The organic layer is dried over magnesium sulphate and the solvent is removed in vacuo to obtain 0.17 eq of N-(6-methyl-2-p-tolyl-imidazo[1,2-a]pyridin-3-ylmethyl)-N-propyl-4-trifluoromethyl-benzamide.
Yield: 17%
MS (ES) m/z = 466 (MH+)
HPLC = 83.9%

The compound of example 40 was prepared according to this procedure starting from the corresponding N-dealkylated amide.

**Example 40:** Cyclopropanecarboxylic acid (6-methyl-2-p-tolyl-imidazo[1,2-a]pyridin-3-ylmethyl)-propyl-amide

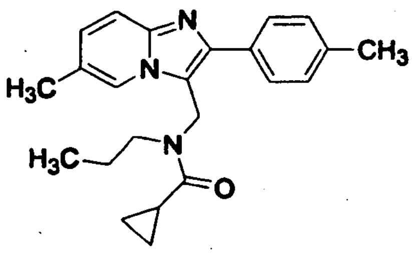

Yield: 35%
MS (ES) m/z = 362 (MH+)
HPLC = 89.8%

**Example 41:** N-(6-methyl-2-p-tolyl-imidazo[1,2-a]pyridin-3-ylmethyl)-N-prop-2-ynyl-4-trifluoromethyl-benzamide

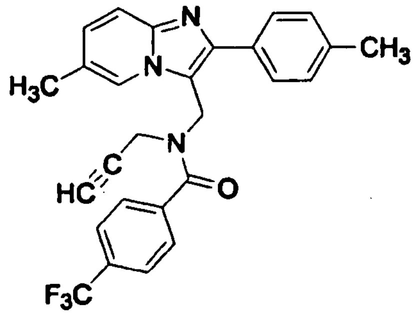

To a solution of N-(6-methyl-2-p-tolyl-imidazo[1,2-a]pyridin-3-ylmethyl)-4-trifluoromethyl-benzamide (1 eq) in dry DMF are added 1.2 eq of NaH (60%) under argon. The suspension is stirred for 10 min at room temperature. Then 1.1 eq of 3-bromo-propyne are added and the corresponding mixture is stirred for 1h at room temperature. After this period, NaOH 0.5 N is added. The mixture is extracted with dichloromethane. The organic layer is dried under magnesium sulphate and the solvent is removed in vacuo to obtain 0.16 eq of N-(6-methyl-2-p-tolyl-imidazo[1,2-a]pyridin-3-ylmethyl)-N-prop-2-ynyl-4-trifluoromethyl-benzamide.
MS (ES) m/z = 462 (MH+)
HPLC = 84%

The compound of example 42 was prepared according to this procedure starting from the corresponding N-dealkylated amide.

**Example 42:** Cyclopropanecarboxylic acid (6-methyl-2-p-tolyl-imidazo[1,2-a]pyridin-3-ylmethyl)-prop-2-ynyl-amide

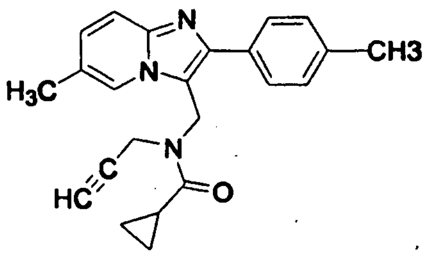

Yield: 29%
MS (ES) m/z = 358 (MH+)
HPLC = 84.7%

**Example 43:** C-(6-Methyl-2-p-tolyl-imidazo(1,2-a]pyridin-3-yl)-methylamine

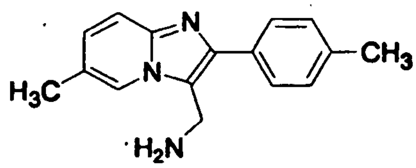

A solution of 3 g (13 mmol) of bromide of 6-methyl-2-p-tolyl-imidazo[1,2-a]pyridin-4-ium in 50 mL of acetic acid is added to a solution of 1 g (13 mmol) N-methanolacetamide in 50 mL of acetic acid. To the resulting solution are added

slowly 5 g (54 mmol) of concentrated sulphuric acid. The crude is stirred at room temperature for 1.5 hours and after at reflux for 2 hours. The reaction is allowed to cool, and 50 mL of water are added. The crude is basified with ammonia 25% and extracted with dichloromethane. The organic layer is dried, filtered off and the solvent is removed, in vacuo, to obtain 3.86 g (13.2 mmol, yield: 97%) of the corresponding amide. This amide is dissolved in 150 mL of ethanol and 50 mL of concentrated hydrochloric acid are added. The mixture is heated at reflux for 30 min.

The crude is neutralized and the solvent is removed. The residue is extracted with DCM-water, and the organic layer is dried, filtered off and evaporated, to obtain 3.2 g (12.8 mmol, 97%) of C-(6-Methyl-2-p-tolyl-imidazo[1,2-a]pyridin-3-yl)-methylamine as a white solid.
MS (ES) m/z = 252 (MH+)
HPLC = 90%

**Example 44:** 1-(4-Dimethylamino-phenyl)-3-(6-methyl-2-p-tolyl-imidazo[1,2-a]pyridin-3-ylmethyl)-urea

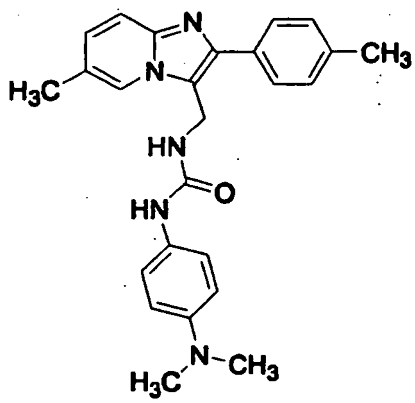

To a solution of 1 eq of C-(6-methyl-2-p-tolyl-imidazo[1,2-a]pyridin-3-yl)-methylamine in pyridine is added a solution of 1-(4-Dimethylamino-phenyl)-isocyanate (1 eq) in pyridine. The mixture is stirred at room temperature for 24 hours. The solvent is removed and water is added to the residue. The solid thus obtained is filtered off, washed with water and dried over calcium chloride to give 1-(4-Dimethylaminophenyl)-3-(6-methyl-2-p-tolyl-imidazo[1,2-a]pyridin-3-ylmethyl)-urea.
Yield: 27%
$^1$H NMR (400 MHz, DMSO-$d_6$): δ 8.78 (NH, 1H, s), 8.45 (NH, 1H, m), 7.80-6.80 (Ar, 11H, m), 4.76 ($CH_2$, 2H, d, *J*= 5.6 Hz), 2.85 ($Me_2N$, 6H, s), 2.43 (Ph-Me, 3H, s), 2.4 (Me, 3H, s).
MS (ES) m/z = 414 (MH+)
HPLC = 94%

The compounds of examples 45-49 were prepared according to this procedure starting from the corresponding isocyanates.

**Example 45:** 1-Ethyl-3-(6-methyl-2-p-tolyl-imidazo[1,2-a] pyridin-3-ylmethyl)-urea

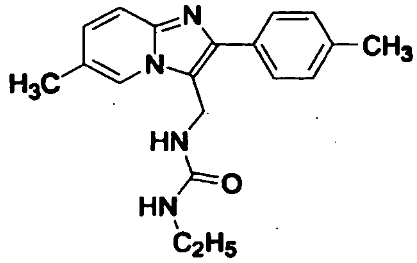

Yield: 27%
$^1H$ NMR (400 MHz, DMSO-$d_6$): δ 8.84-7.41 (Ar, 7H, m), 6.78 (NH, 1H, m), 6.08 (NH, 1H, m), 4.67 ($CH_2$, 2H, d, *J*= 5.2 Hz), 3.01 ($CH_2$, 2H, quint, *J*= 6.8 Hz), 2.44 (Ph-Me, 3H, s), 2.4 (Me, 3H, s), 0.97 (Me-$CH_2$, 3H, t, *J*= 6.8 Hz) .
MS (ES) m/z = 323 (MH+)
HPLC = 100%

**Example 46:** 1-Isopropyl-3-(6-methyl-2-p-tolyl-imidazo[1,2-a]pyridin-3-ylmethyl)-urea

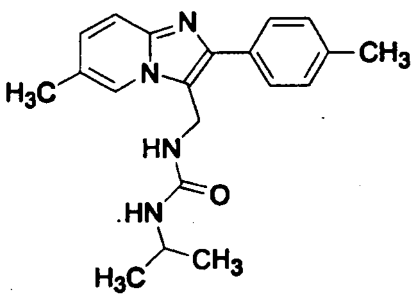

Yield: 28%
$^1H$ NMR (400 MHz, DMSO-$d_6$) : δ 8.83-7.42 (Ar, 7H, m), 6.61 (NH, 1H, m), 5.93 (NH, 1H, d, *J*= 8 Hz), 4.68 ($CH_2$, 2H, d, *J*= 5.2 Hz), 3.67 (CH-$Me_2$, 1H, m), 2.43 (Ph-Me, 3H, s), 2.41 (Me, 3H, s), 1.00 ($Me_2$-CH, 6H, d, *J*= 6 Hz).
MS (ES) m/z = 337 (MH+)
HPLC = 100%

**Example 47:** 1-Cyclopentyl-3-(6-methyl-2-p-tolyl-imidazo [1,2-a]pyridin-3-ylmethyl)-urea

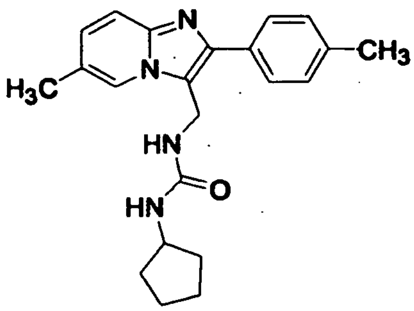

Yield: 36%
$^1H$ NMR (400 MHz, DMSO-$d_6$) : δ 8.79-7.40 (Ar, 7H, m), 6.52 (NH, 1H, m), 6.05 (NH, 1H, d, *J*= 7.6 Hz), 4.68 ($CH_2$, 2H, d, *J*= 5.2 Hz), 3.85 (CH, 1H, m), 2.42 (Ph-Me, 3H, s), 2.4 (Me, 3H, s), 1.76-1.22 (($CH_2$)4, 8H, m).
MS (ES) m/z = 363 (MH+)
HPLC = 99%

**Example 48:** 1-Cyclohexyl-3-(6-methyl-2-p-tolyl-imidazo [1,2-a]pyridin-3-ylmethyl)-urea

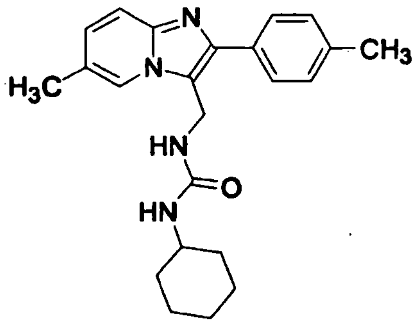

Yield: 46%
$^1H$ NMR (400 MHz, DMSO-$d_6$): δ 8.75-7.40 (Ar, 7H, m), 6.54 (NH, 1H, m), 5.94 (NH, 1H, d, *J*= 6.8 Hz), 4.68 ($CH_2$, 2H, d, *J*= 5.2 Hz) , 3.9 (CH, 1H, m), 2.42 (Ph-Me, 3H, s), 2.4 (Me, 3H, s), 1.72-1.04 ($(CH_2)_5$, 10H, m).
MS (ES) m/z = 377 (MH+)
HPLC = 98%

**Example 49:** 1-(6-Methyl-2-p-tolyl-imidazo[1,2-a]pyridin-3-ylmethyl)-3-phenyl-urea

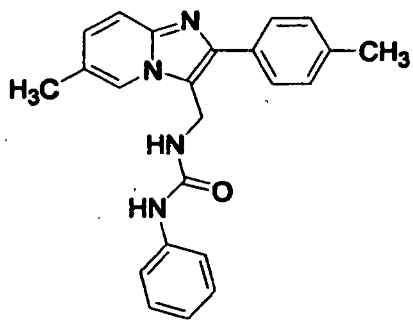

Yield: 30%
$^1H$ NMR (400 MHz, DMSO-$d_6$): δ 8.43 (NH, 1H, s), 8.34-6.88 (Ar, 12H, m), 6.83 (NH, 1H, t, *J*= 5.6 Hz), 4.74 ($CH_2$, 2H, d, *J*= 5.6 Hz), 2.35 (Ph-Me, 3H, s), 2.31 (Me, 3H, s).
MS (ES) m/z = 371 (MH+).
HPLC = 92%

**Example 50:** (6-Methyl-2-p-tolyl-imidazo[1,2-a]pyridin-3-ylmethyl)-carbamic acid p-tolyl ester

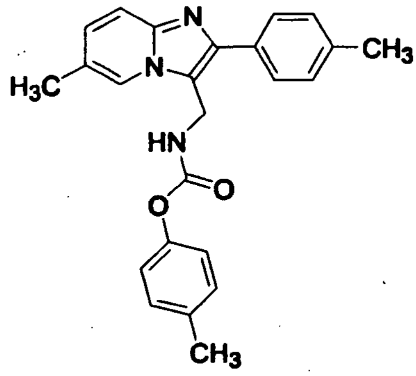

To a solution of 1 eq of C-(6-Methyl-2-p-tolyl-imidazo[1,2-a]pyridin-3-yl)-methylamine in pyridine is added a solution of p-tolyl-chloroformiate (1 eq) in pyridine. The mixture is stirred at room temperature for 24 hours. The solvent is removed and water is added to the residue. The solid thus obtained is filtered off, washed with water and dried over calcium chloride to give (6-methyl-2-p-tolyl-imidazo[1,2-a]pyridin-3-ylmethyl)-carbamic acid p-tolyl ester.
Yield: 16%
$^1$H NMR (400 MHz, DMSO-$d_6$): δ 8.41-6.93 (Ar, 11H, m), 6.62 (NH, 1H, t, *J*= 5.6 Hz), 4.71 ($CH_2$, 2H, d, *J*= 5.6 Hz), 2.35 (impy-Ph-Me, 3H, s), 2.3 (Ph-Me, 3H, s), 2.28 (Me, 3H, s).
MS (ES) m/z = 386 (MH+)
HPLC = 80%

The compounds of examples 51-57 were prepared according to this procedure starting from the corresponding chloroformiates.

**Example 51:** (6-Methyl-2-p-tolyl-imidazo[1,2-a]pyridin-3-ylmethyl)-carbamic acid prop-2-ynyl ester

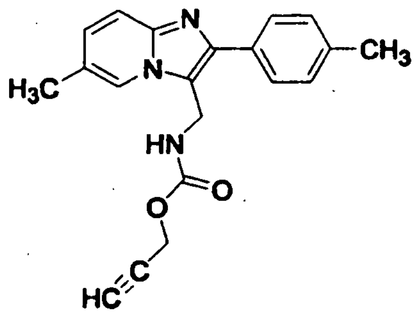

Yield: 5%
$^1$H NMR (400 MHz, DMSO-$d_6$) : δ 8.21-7.15 (Ar, 7H, m), 8.05 (NH, 1H, t, *J*= 5.2 Hz), 4.66 ($CH_2$-C, 2H, d, *J*= 2.4 Hz), 4.64 ($CH_2$, 2H, d, *J*= 5.2 Hz), 3.5 (CH, 1H, t, *J*= 2.4 Hz), 2.34 (Ph-Me, 3H, s), 2.31 (Me, 3H, s).
MS (ES) m/z = 334 (MH+)
HPLC = 98%.

**Example 52:** (6-Methyl-2-p-tolyl-imidazo[1,2-a]pyridin-3-ylmethyl)-carbamic acid methyl ester

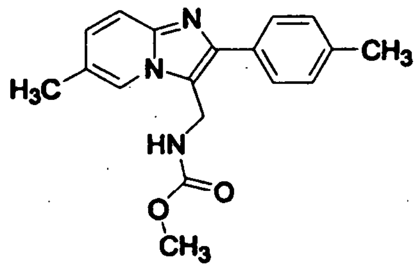

Yield: 30%

$^1H$ NMR (400 MHz, DMSO-$d_6$): δ 8.23-7.14 (Ar, 7H, m), 7.83 (NH, 1H, m), 4.61 ($CH_2$, 2H, d, *J*= 5.2 Hz), 3.56 (MeO, 3H, s), 2.34 (Ph-Me, 3H, s), 2.31 (Me, 3H, s).

MS (ES) m/z = 310 (MH+)

HPLC = 100%

**Example 53:** (6-Methyl-2-p-tolyl-imidazo[1,2-a]pyridin-3-ylmethyl)-carbamic acid benzyl ester

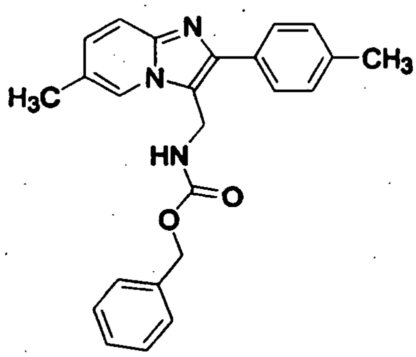

Yield: 15%

$^1H$ NMR (400 MHz, DMSO-$d_6$): δ 8.21-7.14 (Ar, 12H, m), 7.98 (NH, 1H, m), 5.08 ($CH_2$-Ph, 2H, s), 4.65 ($CH_2$, 2H, d, *J*= 5.6 Hz), 2.34 (Ph-Me, 3H, s), 2.28 (Me, 3H, s). MS (ES) m/z = 386 (MH+)

HPLC = 86%

**Example 54:** (6-Methyl-2-p-tolyl-imidazo[1,2-a]pyridin-3-ylmethyl)-carbamic acid 4-methoxy-phenyl ester

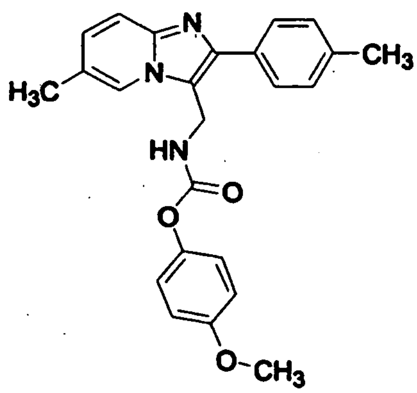

Yield: 41%
$^1H$ NMR (400 MHz, DMSO-$d_6$): δ 8.70-6.89 (Ar, 11H, m), 8.50 (NH, 1H, m), 4.74 ($CH_2$, 2H, d, *J*= 5.2 Hz), 3.73 (MeO, 3H, s), 2.45 (Ph-Me, 3H, s), 2.39 (Me, 3H, s).
MS (ES) m/z = 402 (MH+)
HPLC = 88%

**Example 55:** (6-Methyl-2-p-tolyl-imidazo[1,2-a]pyridin-3-ylmethyl)-carbamic acid ethyl ester

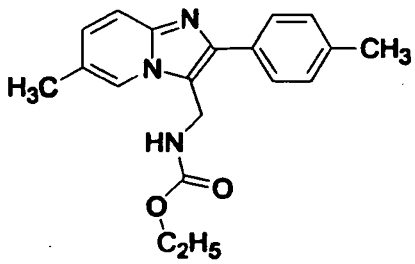

Yield: 23%
$^1H$ NMR (400 MHz, DMSO-$d_6$): δ 8.46-7.32 (Ar, 7H, m), 4.63 ($CH_2$, 2H, d, *J*= 4.4 Hz), 4.02 ($CH_2$-Me, 2H, m), 2.49 (Ph-Me, 3H, s), 2.37 (Me, 3H, s), 1.15 (Me, 3H, t, *J*= 6.8 Hz).
MS (ES) m/z = 324 (MH+)
HPLC = 81%

**Example 56:** (6-Methyl-2-p-tolyl-imidazo[1,2-a]pyridin-3-ylmethyl)-carbamic acid phenyl ester

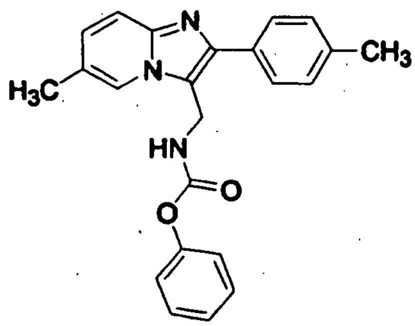

Yield: 22%
$^1H$ NMR (400 MHz, DMSO-$d_6$): δ 8.70-6.72 (Ar, 12H, m), 8.56 (NH, 1H, m), 4.76 ($CH_2$, 2H, d, *J*= 5.6 Hz), 2.45 (Ph-Me, 3H, s), 2.39 (Me, 3H, s).
MS (ES) m/z = 372 (MH+)
HPLC = 90%

**Example 57:** (6-Methyl-2-p-tolyl-imidazo[1,2-a]pyridin-3-ylmethyl)-carbamic acid isopropyl ester

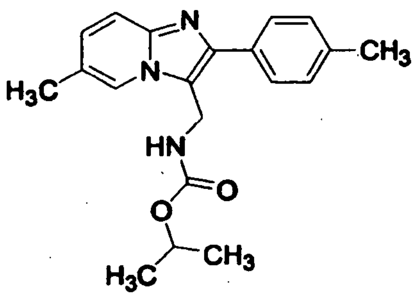

Yield: 16%
$^1H$ NMR (400 MHz, DMSO-$d_6$): δ 8.25-7.14 (Ar, 7H, m), 7.75 (NH, 1H, d, *J*= 5.6 Hz), 4.81 (CH, 1H, m), 4.62 ($CH_2$, 2H, d, *J*= 5.6 Hz), 2.34 (Ph-Me, 3H, s), 2.31 (Me, 3H, s), 1.17 ($Me_2CH$, 6H, d, *J*= 6.4 Hz).
MS (ES) m/z = 338 (MH+)
HPLC = 98%

The compounds of examples 58-90 were prepared according to the procedure described for example 44, starting from C-(6-Methyl-2-p-tolyl-imidazo[1,2-a]pyridin-3-yl)-methylamine and the corresponding acid chlorides.

**Example 58:** Cyclobutanecarboxylic acid (6-methyl-2-p-tolyl-imidazo[1,2-a]pyridin-3-ylmethyl)-amide

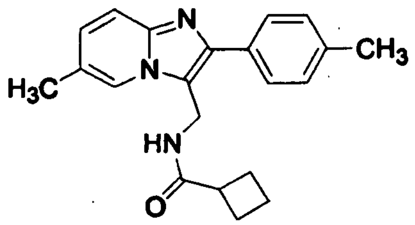

MS (ES) m/z = 334 (MH+)
HPLC = 99%

**Example 59:** Cyclopentanecarboxylic acid (6-methyl-2-p-tolyl-imidazo[1,2-a]pyridin-3-ylmethyl)-amide

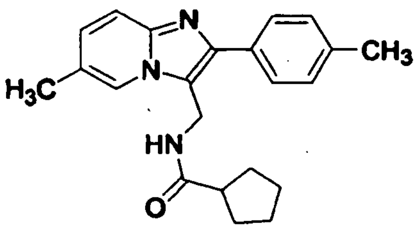

MS (ES) m/z = 348 (MH+)
HPLC = 99%

**Example 60:** Benzo[b]thiophene-3-carboxylic acid (6-methyl-2-p-tolyl-imidazo[1,2-a]pyridin-3-ylmethyl)-amide

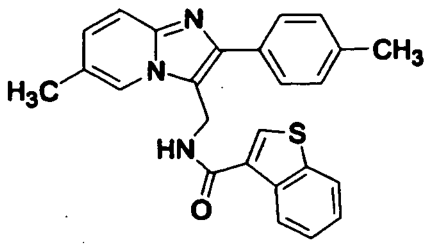

MS (ES) m/z = 413 (MH+)
HPLC = 97%

**Example 61:** 5-Methyl-pyrazine-2-carboxylic acid (6-methyl-2-p-tolyl-imidazo[1,2-a]pyridin-3-ylmethyl)-amide

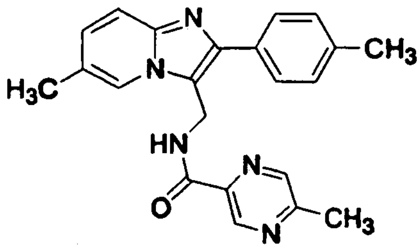

MS (ES) m/z = 372 (MH+)
HPLC = 90%

**Example 62:** 1-Methyl-1H-pyrrole-2-carboxylic acid (6-methyl-2-p-tolyl-imidazo[1,2-a]pyridin-3-ylmethyl)-amide

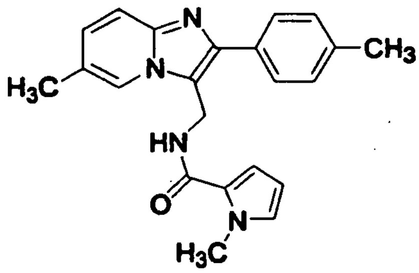

MS (ES) m/z = 359 (MH+)
HPLC = 90%

**Example 63:** N-(6-Methyl-2-p-tolyl-imidazo[1,2-a]pyridin -3-ylmethyl)-nicotinamide

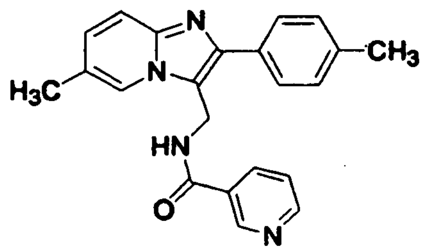

MS (ES) m/z = 357 (MH+)
HPLC = 92%

**Example 64:** 5-Chloro-4-methoxy-thiophene-3-carboxylic acid (6-methyl-2-p-tolyl-imidazo[1,2-a]pyridin-3-ylmethyl)-amide

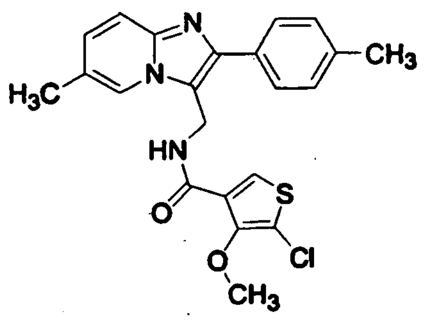

MS (ES) m/z = 427 (MH+)
HPLC = 98%

**Example 65:** 6-Oxo-1,4,5,6-tetrahydro-pyridazine-3-carboxylic acid (6-methyl-2-p-tolyl-imidazo[1,2-a]pyridin-3-ylmethyl)-amide

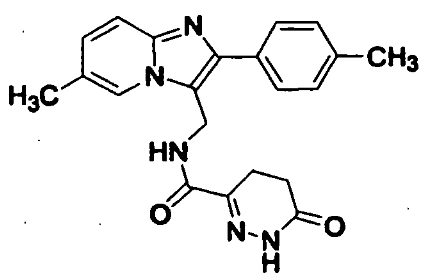

MS (ES) m/z = 376 (MH+)
HPLC = 99%

**Example 66:** Benzo[c]isoxazole-3-carboxylic acid (6-methyl-2-p-tolyl-imidazo[1,2-a]pyridin-3-ylmethyl)-amide

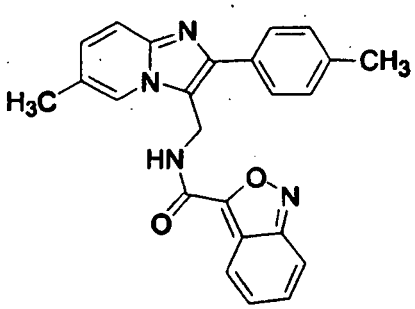

MS (ES) m/z = 397 (MH+)
HPLC = 97%

**Example 67:**1,5-Dimethyl-1H-pyrazole-3-carboxylic acid (6-methyl-2-p-tolyl-imidazo[1,2-a]pyridin-3-ylmethyl)-amide

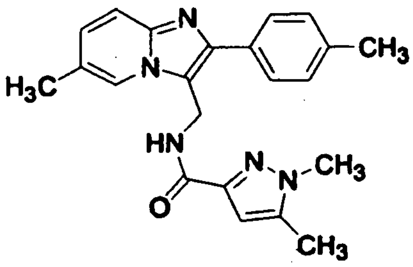

MS (ES) m/z = 374 (MH+)
HPLC = 99%

**Example 68:** 1-Methyl-1H-indole-3-carboxylic acid (6-methyl-2-p-tolyl-imidazo[1,2-a]pyridin-3-ylmethyl)-amide

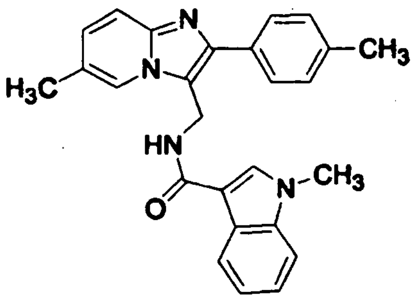

MS (ES) m/z = 410 (MH+)
HPLC = 98%

**Example 69:** 2-Methyl-thiazole-4-carboxylic acid (6-methyl-2-p-tolyl-imidazo[1,2-a]pyridin-3-ylmethyl)-amide

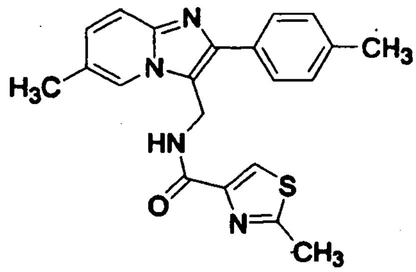

MS (ES) m/z = 377 (MH+)
HPLC = 99%

**Example 70:** [1,2,3]Thiadiazole-4-carboxylic acid (6-methyl-2-p-tolyl-imidazo[1,2-a]pyridin-3-ylmethyl)-amide

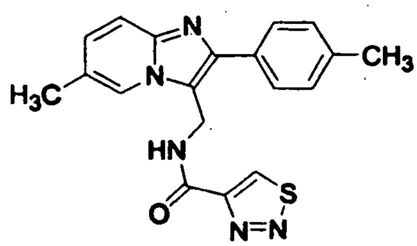

MS (ES) m/z = 364 (MH+)
HPLC = 99%

**Example 71:** N-(6-Methyl-2-p-tolyl-imidazo[1,2-a]pyridin-3-ylmethyl)-2-thiophen-2-yl-acetamide

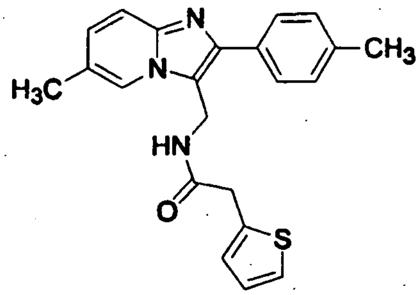

MS (ES) m/z = 376 (MH+)
HPLC = 98%

**Example 72:** 5-Methyl-isoxazole-3-carboxylic acid (6-methyl-2-p-tolyl-imidazo[1,2-a]pyridin-3-ylmethyl)-amide

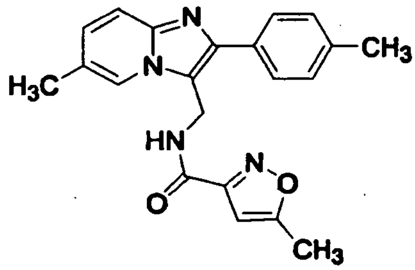

MS (ES) m/z = 361 (MH+)
HPLC = 97%

**Example 73:** N-(6-Methyl-2-p-tolyl-imidazo[1,2-a]pyridin-3-ylmethyl)-6-(2,2,2-trifluoro-ethoxy)-nicotinamide

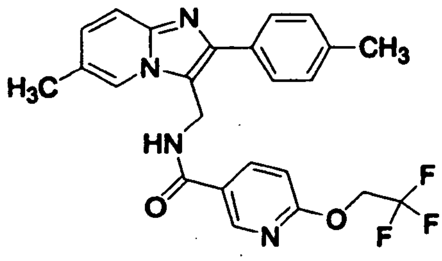

MS (ES) m/z = 455 (MH+)
HPLC = 91%

**Example 74:** 1-Methyl-1H-imidazole-4-carboxylic acid (6-methyl-2-p-tolyl-imidazo[1,2-a]pyridin-3-ylmethyl)-amide

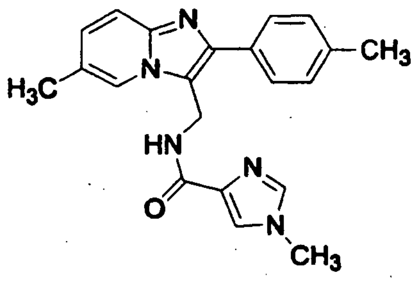

MS (ES) m/z = 360 (MH+)
HPLC = 99%

**Example 75:** 6-Methoxy-2-oxo-2H-chromene-3-carboxylic acid (6-methyl-2-p-tolyl-imidazo[1,2-a]pyridin-3-ylmethyl)-amide

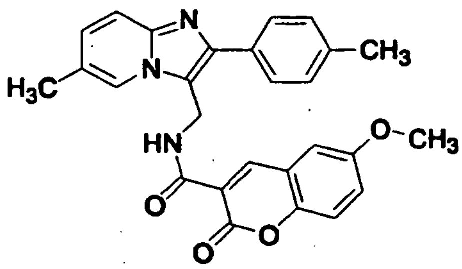

MS (ES) m/z = 454 (MH+)
HPLC = 89%

**Example 76:** 4-Methoxy-thiophene-3-carboxylic acid (6-methyl-2-p-tolyl-imidazo[1,2-a]pyridin-3-ylmethyl)-amide

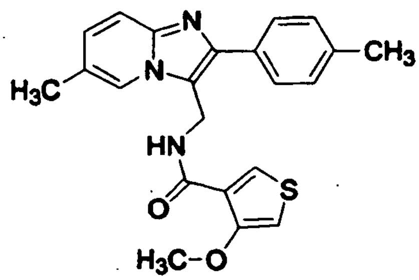

MS (ES) m/z = 392 (MH+)
HPLC = 93%

**Example 77:** 5-Methoxy-thiophene-2-carboxylic acid (6-methyl-2-p-tolyl-imidazo[1,2-a]pyridin-3-ylmethyl)-amide

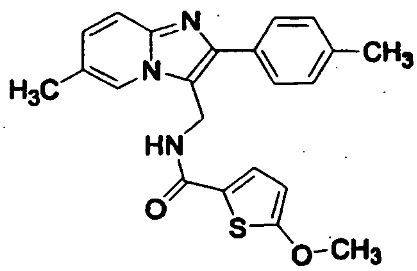

MS (ES) m/z = 392 (MH+)
HPLC = 91%

**Example 78:** 1-Methyl-1H-imidazole-2-carboxylic acid (6-methyl-2-p-tolyl-imidazo[1,2-a]pyridin-3-ylmethyl)-amide

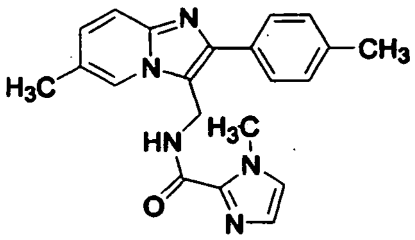

MS (ES) m/z = 360 (MH+)
HPLC = 90%

**Example 79:** 4-Methyl-[1,2,3]thiadiazole-5-carboxylic acid (6-methyl-2-p-tolyl-imidazo[1,2-a]pyridin-3-ylmethyl)-amide

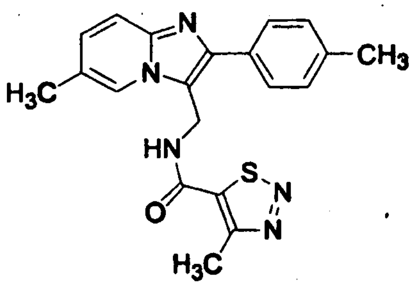

MS (ES) m/z = 378 (MH+)
HPLC = 91%

**Example 80:** 3-Furan-2-yl-N-(6-methyl-2-p-tolyl-imidazo[1,2-a]pyridin-3-ylmethyl)-acrylamide

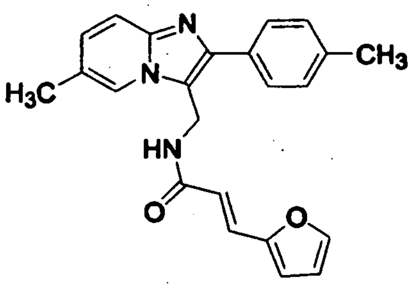

MS (ES) m/z = 372 (MH+)
HPLC = 96%

**Example 81:** Thiazole-4-carboxylic acid (6-methyl-2-p-tolyl-imidazo[1,2-a]pyridin-3-ylmethyl)-amide

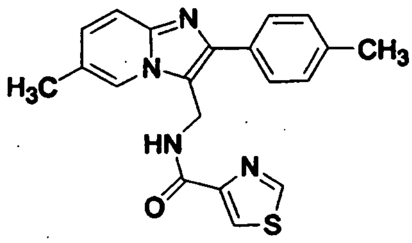

MS (ES) m/z = 363 (MH+)
HPLC = 98%

**Example 82:** Thiophene-3-carboxylic acid (6-methyl-2-p-tolyl-imidazo[1,2-a]pyridin-3-ylmethyl)-amide

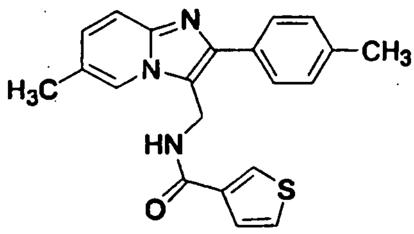

MS (ES) m/z = 362 (MH+)
HPLC = 95%

**Example 83:** 2,5-Dimethyl-oxazole-4-carboxylic acid (6-methyl-2-p-tolyl-imidazo[1,2-a]pyridin-3-ylmethyl)-amide

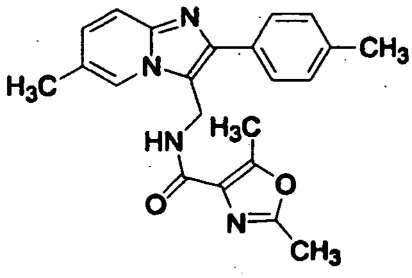

MS (ES) m/z = 375 (MH+)
HPLC = 98%

**Example 84:** 1-Cyclopropyl-2,5-dimethyl-1H-pyrrole-3-carboxylic acid (6-methyl-2-p-tolyl-imidazo[1,2-a]pyridin-3-ylmethyl)-amide

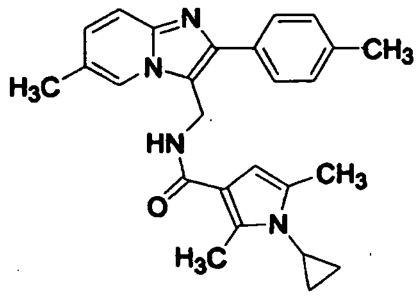

MS (ES) m/z = 414 (MH+)
HPLC = 95%

**Example 85:** 4,5-Dichloro-isothiazole-3-carboxylic acid (6-methyl-2-p-tolyl-imidazo[1,2-a]pyridin-3-ylmethyl)-amide

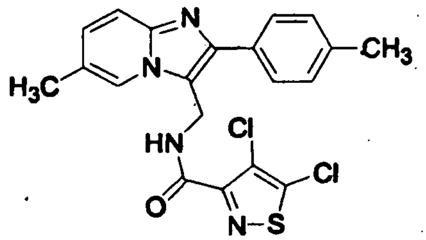

MS (ES) m/z = 432 (MH+)
HPLC = 92%

**Example 86:** 1,2,5-Trimethyl-1H-pyrrole-3-carboxylic acid (6-methyl-2-p-tolyl-imidazo[1,2-a]pyridin-3-ylmethyl)-amide

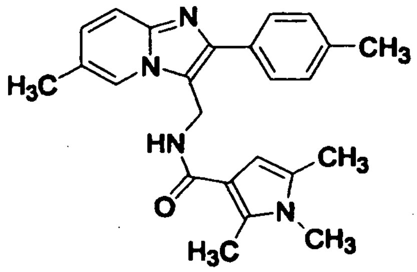

MS (ES) m/z = 387 (MH+)
HPLC = 96%

**Example 87:** 2,4-Dichloro-5-fluoro-N-(6-methyl-2-p-tolyl-imidazo[1,2-a]pyridin-3-ylmethyl)-benzamide

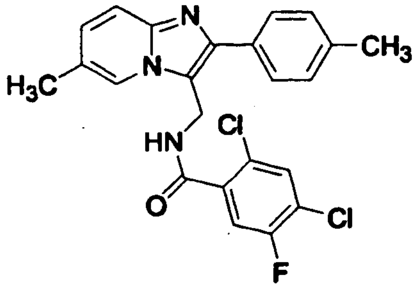

MS (ES) m/z = 443 (MH+)
HPLC = 91%

**Example 88:** 5-Nitro-thiophene-3-carboxylic acid (6-methyl-2-p-tolyl-imidazo[1,2-a]pyridin-3-ylmethyl)-amide

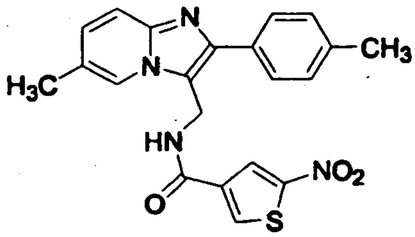

MS (ES) m/z = 407 (MH+)
HPLC = 97%

**Example 89:** Pyrazine-2-carboxylic acid (6-methyl-2-p-tolyl-imidazo[1,2-a]pyridin-3-ylmethyl)-amide

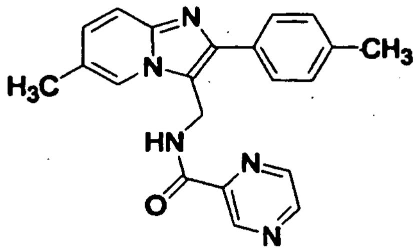

MS (ES) m/z = 358 (MH+)
HPLC = 97%

**Example 90:** 3,5-Dimethyl-isoxazole-4-carboxylic acid (6-methyl-2-p-tolyl-imidazo[1,2-a]pyridin-3-ylmethyl)-amide

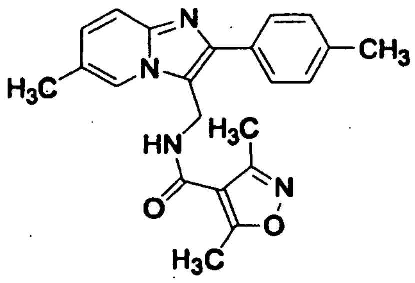

MS (ES) m/z = 375 (MH+)
HPLC = 93%

The compounds of examples 91-100 were prepared according to the procedure described for example 22.

**Example 91:** 2-Methyl-thiazole-4-carboxylic acid methyl-(6-methyl-2-p-tolyl-imidazo[1,2-a]pyridin-3-ylmethyl)-amide

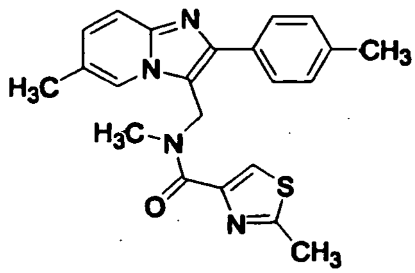

MS (ES) m/z = 392 (MH+)
HPLC = 99%

**Example 92:** 1,5-Dimethyl-1H-pyrazole-3-carboxylic acid methyl-(6-methyl-2-p-tolyl-imidazo[1,2-a]pyridin-3-ylmethyl)-amide

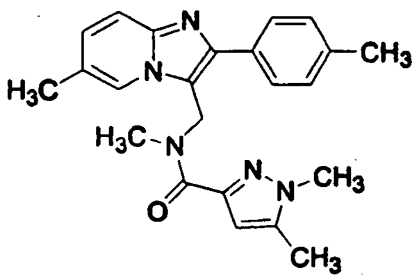

MS (ES) m/z = 388 (MH+)
HPLC = 90%

**Example 93:** 1-Methyl-6-oxo-1,4,5,6-tetrahydro-pyridazine-3-carboxylic acid methyl-(6-methyl-2-p-tolyl-imidazo[1,2-a]pyridin-3-ylmethyl)-amide

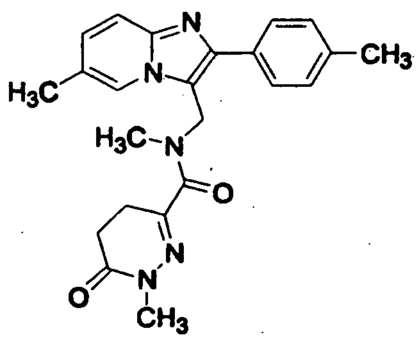

MS (ES) m/z = 404 (MH+)
HPLC = 90%

**Example 94:** Thiazole-4-carboxylic acid methyl-(6-methyl-2-p-tolyl-imidazo[1,2-a]pyridin-3-ylmethyl)-amide

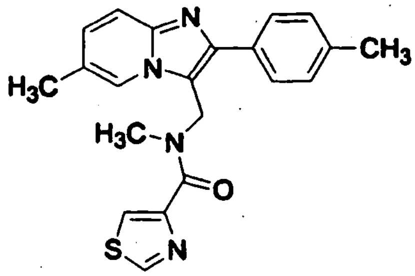

MS (ES) m/z = 377 (MH+)
HPLC = 99%

**Example 95:** 2,5-Dimethyl-oxazole-4-carboxylic acid methyl-(6-methyl-2-p-tolyl-imidazo[1,2-a]pyridin-3-ylmethyl)-amide

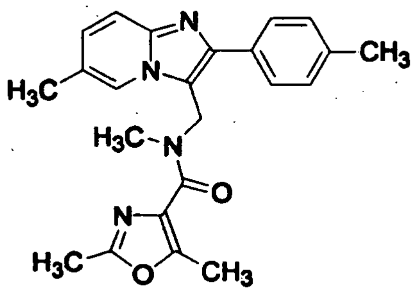

MS (ES) m/z = 389 (MH+).
HPLC = 91%

**Example 96:** Pyrazine-2-carboxylic acid methyl- (6-methyl-2-p-tolyl-imidazo[1,2-a]pyridin-3-ylmethyl)-amide

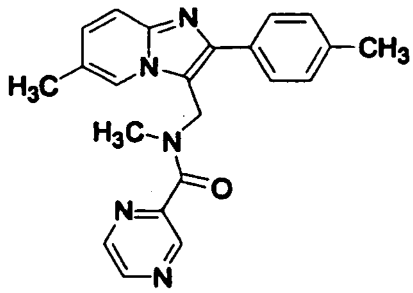

MS (ES) m/z = 372 (MH+)
HPLC = 98%

**Example 97:** 1-Methyl-1H-imidazole-4-carboxylic acid methyl-(6-methyl-2-p-tolyl-imidazo[1,2-a]pyridin-3-ylmethyl)-amide

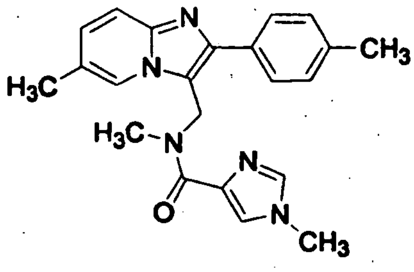

MS (ES) m/z = 374 (MH+)
HPLC = 95%

**Example 98:** 1-Methyl-1H-imidazole-2-carboxylic acid methyl-(6-methyl-2-p-tolyl-imidazo[1,2-a]pyridin-3-ylmethyl)-amide

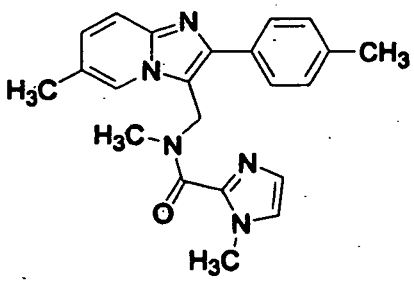

MS (ES) m/z = 374 (MH+)
HPLC = 99%

**Example 99:** 5-Methyl-isoxazole-4-carboxylic acid methyl-(6-methyl-2-p-tolyl-imidazo[1,2-a]pyridin-3-ylmethyl)-amide

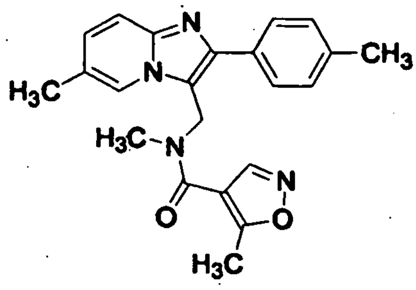

MS (ES) m/z = 375 (MH+)
HPLC 99%.

**Example 100:** 5-Nitro-thiophene-3-carboxylic acid methyl-(6-methyl-2-p-tolyl-imidazo[1,2-a]pyridin-3-ylmethyl)-amide

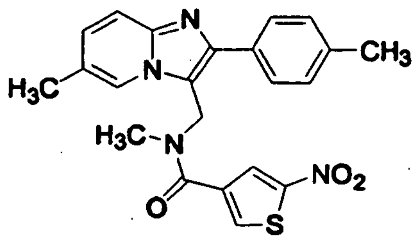

MS (ES) m/z = 421 (MH+)
HPLC = 90%

**Example 101:** 5 mg tablets

| Active ingredient | 5.0 mg |
|---|---|
| Colloidal silicon dioxide | 0.6 mg |
| Croscarmellose sodium | 12.0 mg |
| Talc | 4.0 mg |
| Magnesium stearate | 1.5 mg |
| Polysorbate 80 | 1.0 mg |
| Lactose | 75.0 mg |
| Hydroxypropyl methylcellulose | 3.0 mg |
| Polyethylene glycol 4000 | 0.5 mg |
| Titanium dioxide E171 | 1.5 mg |
| Microcrystalline cellulose q.s. to | 125.0 mg |

**Example 102:** 10 mg capsules

| Active ingredient | 10.0 mg |
|---|---|
| Colloidal silicon dioxide | 0.6 mg |

(continued)

| Active ingredient | 10.0 mg |
|---|---|
| Crospovidone | 12.0 mg |
| Talc | 4.0 mg |
| Magnesium stearate | 1.5 mg |
| Lauryl sulfate sodium | 1.5 mg |
| Lactose | 77.0 mg |
| Gelatin | 28.5 mg |
| Titanium dioxide E171 | 1.5 mg |
| Indigotin E132 | 0.02 mg |
| Microcrystalline cellulose q.s. to | 155.0 mg |

**Example 103:** oral drops

| Active ingredient | 0.5 g |
|---|---|
| Propylene glycol | 10.0 g |
| Glycerin | 5.0 g |
| Saccharin sodium | 0.1 g |
| Polysorbate 80 | 1.0 g |
| Lemon flavor | 0.2 g |
| Ethanol | 25.0 mL |
| Purified water q.s. to | 100.0 mL |

## Claims

1. An imidazo[1,2-a] pyridine compound of formule (I):

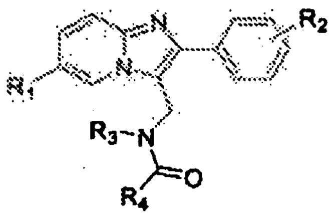

(I)

as well as pharmaceutically acceptable salts thereof;
wherein
$R_1$ and $R_2$ are independently selected from the group consisting of hydrogen, linear or branched alkyl($C_1$-$C_6$), alkenyl ($C_2$-$C_6$), alkynyl($C_2$-$C_6$), haloalkyl ($C_1$-$C_6$),-O-alkyl($C_1$-$C_6$), fluoro, chloro and bromo;
$R_3$ is selected from the group consisting of hydrogen, linear or branched alkyl ($C_1$-$C_6$) , cycloalkyl ($C_3$-$C_6$) , cycloalkyl ($C_3$-$C_6$) alkyl ($C_1$-$C_6$), alkenyl ($C_1$-$C_6$), alkynyl ($C_2$-$C_6$) alkyl ($C_1$-$C_6$) , alkynyl ($C_2$-$C_6$) , alkynyl ($C_2$-$C_6$) alkyl ($C_1$-$C_6$) ;
$R_4$ is selected from the group consisting of haloalkyl ($C_2$-$C_6$) cycloalkyl ($C_3$-$C_5$), cycloalkyl ($C_3$-$C_6$)alkyl($C_1$-$C_6$), alkynyl ($C_2$-$C_6$) alkyl ($C_1$-$C_6$), alkyl($C_1$-$C_6$)-O-alkyl ($C_1$-$C_6$), alkyl ($C_1$-$C_6$)-NH-alkyl ($C_1$-$C_6$) , alkyl ($C_1$-$C_6$)-N(dialkyl ($C_1$-$C_6$)), $-OR_5$, $-NHR_5$, $-NR_5R_6$,

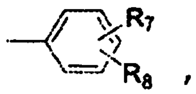

phenylalkyl ($C_2$-$C_6$), Phenylalkenyl ($C_2$-$C_6$) , naphthyl, monosubstituted naphthyl, disubstituted naphthyl, naphthylalkyl($C_1$-$C_6$), naphthylalkenyl ($C_2$-$C_6$) furyl, substituted furyl, benzofuryl, substituted benzofuryl, pyrrolyl, substituted pyrrolyl, isoxazolyl, substituted isoxazolyl, benzoisoxazolyl, substituted benzoisoxazolyl, imidazolyl, substituted imidazolyl, benzimidazolyl, substituted benzimidazolyl, indolyl, substituted indolyl, pyrazolyl, substituted pyrazolyl, thienyl, substituted thienyl, benzothienyl, substituted benzothienyl, thiazolyl, substituted thiazolyl, benzothiazolyl, substituted benzothiazolyl, quinolinyl, substituted quinolinyl, isoquinolinyl, substituted isoquinolinyl, pyridyl, substituted pyridyl, pyrazinyl, substituted pyrazinyl, 6-oxo-1,4,5,6-tetrahydropyridazinyl, substituted 6-oxo-1,4,5,6-tetrahydropyridazinyl, thiadiazolyl, substituted thiadiazolyl, isothiazolyl, substituted isothiazolyl, thienylmethyl, 2-oxochromenyl, substituted 2-oxochromenyl, 2-(furan-2-yl)vinyl; oxazolyl, substituted oxazolyl, and benzisoxazolyl;

$R_5$ and $R_6$ are independently selected from the group consisting of hydrogen, linear or branched alkyl($C_1$-$C_6$) , phenylalkyl ($C_1$-$C_6$) , haloalkyl ($C_1$-$C_6$) , cycloalkyl ($C_3$-$C_6$) , cycloalkyl($C_3$-$C_6$)alkyl($C_1$-$C_6$) , alkenyl($C_2$-$C_6$) and alkynyl($C_2$-$C_6$), alkenyl($C_2$-$C_6$)alkyl($C_1$-$C_6$) , alkynyl($C_2$-$C_6$)alkyl($C_1$-$C_6$), phenyl, substituted phenyl, heteroaryl, substituted heteroaryl; and

$R_7$ and $R_8$ are independently selected from the group consisting of linear or branched alkyl($C_2$-$C_6$), cycloalkyl($C_3$-$C_6$), alkenyl($C_2$-$C_6$), alkynyl($C_2$-$C_6$), -OH, - O-alkyl($C_1$-$C_6$), -SH, -S-alkyl($C_1$-$C_6$), halo-alkyl ($C_1$-$C_6$) , ω,ω,ω-trifluoroalkyl ($C_1$-$C_6$), -NHalkyl($C_1$-$C_6$) , - Ndialkyl($C_1$-$C_6$) , $-NO_2$, -CN, $-SO_2$alkyl($C_1$-$C_6$) , - Coalkyl($C_1$-$C_6$), -COOalkyl($C_1$-$C_6$ , -CO-NHalkyl($C_1$-$C_6$), CONdialkyl ($C_1$-$C_6$), phenyl, substituted phenyl, heteroaryl, and substituted heteroaryl and wherein the substituents of the radicals that are substituted are selected from the group consisting of linear or branched alkyl ($C_2$-$C_6$), cycloalkyl ($C_3$-$C_6$), alkenyl($C_2$-$C_6$), alkynyl ($C_2$-$C_6$), -OH, -O-alkyl ($C_1$-$C_6$), -SH, S-alkyl ($C_1$-$C_6$), halo-alkyl($C_1$-$C_6$), ω,ω,ω-trifluoroalkyl ($C_1$-$C_6$), -NHalkyl($C_2$-$C_6$), -Ndialkyl ($C_1$-$C_6$),$--NO_2$, -CN, $-SO_2$alkyl ($C_1$-$C_6$), -COalkyl($C_1$-$C_6$), -COOalkyl($C_1$-$C_6$) , -CO-NHalkyl($C_1$-$C_6$), -CONdialkyl($C_1$-$C_6$), phenyl, substituted phenyl, heteroaryl, substituted heteroaryl, fluoro, chloro and bromo.

**2.** An imidazo [1,2-a] pyridine compound of formula (I):

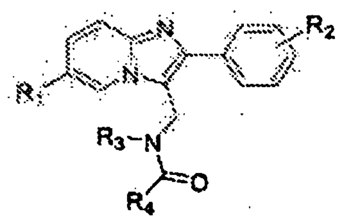

(I)

as well as pharmaceutically acceptable salts thereof;
wherein

$R_1$ and $R_2$ are independently selected from the group consisting of hydrogen, linear or branched alkyl ($C_1$-$C_6$), alkenyl ($C_2$-$C_6$), alkynyl ($C_2$-$C_6$) , haloalkyl ($C_1$-$C_6$), - O-alkyl($C_1$-$C_6$), fluoro, chloro and bromo;

$R_3$ is selected from the group consisting of hydrogen, linear or branched alkyl ($C_1$-$C_6$), cycloalkyl ($C_3$-$C_6$) , cycloalkyl ($C_3$-$C_6$) alkyl ($C_1$-$C_6$), alkynyl ($C_2$-$C_6$), alkenyl($C_2$-$C_6$)alkyl($C_1$-$C_6$), alkynyl ($C_2$-$C_6$) , alkynyl ($C_2$-$C_6$) alkyl ($C_1$-$C_6$);

$R_4$ is selected from the group consisting of haloalkyl($C_2$-$C_6$), cycloalkyl ($C_3$-$C_5$) , cycloalkyl ($C_3$-$C_6$) alkyl ($C_1$-$C_6$), alkynyl ($C_2$-$C_6$) alkyl ($C_1$-$C_6$) , alkyl ($C_1$-$C_6$) - O-alkyl ($C_1$-$C_6$) , alkyl ($C_1$-$C_6$) -NH-alkyl ($C_1$-$C_6$) , alkyl ($C_1$-$C_6$)-N(dialkyl ($C_1$-$C_6$)) , $-OR_5$, $-NHR_5$, $-NR_5R_6$,

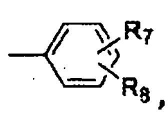

phenylalkyl ($C_2$-$C_6$) , phenylalkenyl ($C_2$-$C_6$), naphthyl, monosubstituted naphthyl, disubstituted naphthyl, naphthylalkyl($C_1$-$C_6$), naphthylalkenyl ($C_2$-$C_6$), furyl, substituted furyl, benzofuryl, substituted benzofuryl, pyrrolyl, substituted pyrrolyl, isoxazolyl, substituted isoxazolyl, benzoisoxazolyl, substituted benzoisoxazolyl, imidazolyl, substituted imidazolyl, benzimidazolyl, substituted benzimidazolyl, indolyl, substituted indolyl, pyrazolyl, substituted pyrazolyl, thienyl, substituted thienyl, benzothienyl, substituted benzothienyl, thiazolyl, substituted thiazolyl, benzothiazolyl, substituted benzothiazolyl, quinolinyl, substituted quinolinyl, isoquinolinyl, substituted isoquinolinyl, pyridyl, and substituted pyridyl;

$R_5$ and $R_6$ are independently selected from the group consisting of hydrogen, linear or branched alkyl($C_1$-$C_6$), phenylalkyl ($C_1$-$C_6$) , haloalkyl ($C_1$-$C_6$) , cycloalkyl ($C_3$-$C_6$) , cycloalkyl ($C_3$-$C_6$) alkyl ($C_1$-$C_6$), alkenyl ($C_2$-$C_6$) and alkynyl ($C_2$-$C_6$) , alkenyl($C_2$-$C_6$) alkyl ($C_1$-$C_6$) , alkynyl ($C_2$-$C_6$)alkyl($C_1$-$C_6$) , phenyl, substituted phenyl, heteroaryl, substituted heteroaryl; and

$R_7$ and $R_8$ are independently selected from the group consisting of linear or branched alkyl($C_2$-$C_6$), cycloalkyl($C_3$-$C_6$), alkenyl($C_2$-$C_6$), alkynyl($C_2$-$C_6$), -OH, - O-alkyl ($C_1$-$C_6$) , -SH, -S-alkyl ($C_1$-$C_6$) , halo-alkyl ($C_1$-$C_6$) , ω,ω,ω-trifluoroalkyl ($C_1$-$C_6$), -NHalkyl ($C_1$-$C_6$), - Ndialkyl ($C_1$-$C_6$) , -$NO_2$, -CN, -$SO_2$alkyl ($C_1$-$C_6$), - COalkyl ($C_1$-$C_6$) , -COOalkyl ($C_1$-$C_6$) , -CO-NHalkyl($C_1$-$C_6$), - CONdialkyl($C_1$-$C_6$),- phenyl, substituted phenyl, heteroaryl and substituted heteroaryl.

**3.** A compound according to claim 1, wherein $R_1$ is a methyl group and $R_2$ is a methyl group in para-position; and $R_3$ is selected from the group consisting of hydrogen, methyl, ethyl, n-propyl, i-propyl, cyclopropyl and 2-propynyl.

**4.** A compound according to claim 3, wherein $R_4$ is selected from the group consisting of cyclopropyl, cyclobutyl, 2-propynyl, N,N-dimethyl-4-aminophenyl, 2-furyl, 5-$NO_2$-2-furyl, 2-pyrrolyl, 2-thienyl, 2-pyridyl, 4,6-difluoro-2-pyridyl, 2-chloro-4-pyridyl, 4-pyridyl, 5-methyl-2-pyrazinyl, 6-oxo-1,4,5,6-tetrahydro-pyridazin-3-yl, [1,2,3]thiadiazol-4-yl, 2-thienylmethyl, 1-methyl-1H-imidazol-2-yl, 4-thiazolyl, 2,5-dimethyl-4-oxazolyl and 3,5-dimethyl-4-isoxazolyl.

**5.** A compound according to claim 4, wherein $R_4$ is selected from the group consisting of cyclopropyl, 2-propynyl, *N*, *N*-dimethyl-4-aminophenyl, 2-furyl, 5-$NO_2$-2-furyl, 2-pyrrolyl, 2-thienyl, 2-pyridyl, 4,6-difluoro-2-pyridyl, 2-chloro-4-pyridyl and 4-pyridyl.

**6.** A compound according to claim 3, wherein $R_4$ is - $NR_5R_6$.

**7.** A compound according to claim 6, wherein $R_5$ is hydrogen or methyl; and $R_6$ is selected from the group consisting of methyl, ethyl, n-propyl, i-propyl, cyclopropyl, cyclopentyl, cyclohexyl, 2-propinyl and phenyl.

**8.** A compound according to claim 3, wherein $R_4$ is -$OR_5$.

**9.** A compound according to claim8, wherein $R_5$ is selected from the group consisting of methyl, ethyl, n-propyl, i-propyl, cyclopropyl, cyclopentyl, cyclohexyl, 2-propinyl, 4-methyl-phenyl, 4-methoxy-phenyl and phenyl.

**10.** A compound according to claim 4, wherein said compound is selected from the group consisting of:

Furan-2-carboxylic acid (6-methyl-2-p-tolyl-imidazo[1,2-a]pyridin-3-ylmethyl)-amide;
Pyridine-2-carboxylic acid (6-methyl-2-p-tolyl-imidazo[1,2-a]pyridin-3-ylmethyl)-amide;
Thiophene-2-carboxylic acid (6-methyl-2-p-tolyl-imidazo[1,2-a]pyridin-3-ylmethyl)-amide;
Cyclopropanecarboxylic acid (6-methyl-2-p-tolyl-imidazo[1,2-a]pyridin-3-ylmethyl)-amide;
5-Nitro-furan-2-carboxylic acid (6-methyl-2-p-tolyl-imidazo[1,2-a]pyridin-3-ylmethyl)-amide;
3,5-Difluoro-pyridine-2-carboxylic acid (6-methyl-2-p-tolyl-imidazo[1,2-a]pyridin-3-ylmethyl)-amide;
6-Methoxy-benzothiazole-2-carboxylic acid (6-methyl-2-p-tolyl-imidazo[1,2-a]pyridin-3-ylmethyl)-amide;
4-Dimethylamino-N-methyl-N-(6-methyl-2-p-tolyl-imidazo[1,2-a]pyridin-3-ylmethyl)-benzamide;
Cyclopropanecarboxylic acid methyl-(6-methyl-2-p-tolyl-imidazo[1,2-a]pyridin-3-ylmethyl)-amide;
Pyridine-2-carboxylic acid methyl-(6-methyl-2-p-tolyl-imidazo[1,2-a]pyridin-3-ylmethyl)-amide;
Thiophene-2-carboxylic acid methyl-(6-methyl-2-p-tolyl-imidazo[1,2-a]pyridin-3-ylmethyl)-amide;
5-Nitro-furan-2-carboxylic acid methyl-(6-methyl-2-p-tolyl-imidazo[1,2-a]pyridin-3-ylmethyl)-amide;
2-Chloro-N-methyl-N-(6-methyl-2-p-tolyl-imidazo[1,2-a]pyridin-3-ylmethyl)-isonicotinamide;
Cyclobutanecarboxylic acid (6-methyl-2-p-tolyl-imidazo[1,2-a]pyridin-3-ylmethyl)-amide;
5-Methyl-pyrazine-2-carboxylic acid (6-methyl-2-p-tolyl-imidazo[1,2-a]pyridin-3-ylmethyl)-amide;
6-Oxo-1,4,5,6-tetrahydro-pyridazine-3-carboxylic acid (6-methyl-2-p-tolyl-imidazo[1,2-a]pyridin-3-ylmethyl)-amide;
[1,2,3]Thiadiazole-4-carboxylic acid (6-methyl-2-p-tolyl-imidazo[1,2-a]pyridin-3-ylmethyl)-amide;
N-(6-Methyl-2-p-tolyl-imidazo[1,2-a]pyridin-3-ylmethyl)-2-thiophen-2-yl-acetamide;
1-Methyl-1H-imidazole-2-carboxylic acid (6-methyl-2-p-tolyl-imidazo[1,2-a]pyridin-3-ylmethyl)-amide;
Thiazole-4-carboxylic acid (16-methyl-2-p-tolyl-imidazo[1,2-a]pyridin-3-ylmethyl)-amide;

2,5-Dimethyl-oxazole-4-carboxylic acid (6-methyl-2-p-tolyl-imidazo[1,2-a]pyridin-3-ylmethyl)-amide;
3,5-Dimethyl-isoxazole-4-carboxylic acid (6-methyl-2-p-tolyl-imidazo[1,2-a]pyridin-3-ylmethyl)-amide; and
Thiazole-4-carboxylic acid methyl-(6-methyl-2-p-tolyl-imidazo[1,2-a]pyridin-3-ylmethyl)-amide.

**11.** A compound according to claims 6 and 7, wherein said compound is selected from the group consisting of:

1-(4-Dimethylamino-phenyl)-3-(6-methyl-2-p-tolyl-imidazo[1,2-a]pyridin-3-ylmethyl)-urea:
1-Ethyl-3-(6-methyl-2-p-tolyl-imidazo[1,2-a]pyridin-3-ylmethyl)-urea:
1-Isopropyl-3-(6-methyl-2-p-tolyl-imidazo[1,2-a]pyridin-3-ylmethyl)-urea:
1-Cyclopentyl-3-(6-methyl-2-p-tolyl-imidazo[1,2-a]pyridin-3-ylmethyl)-urea:
1-Cyclohexyl-3-(6-methyl-2-p-tolyl-imidazo[1,2-a]pyridin-3-ylmethyl)-urea; and
1-(6-Methyl-2-p-tolyl-imidazo[1,2-a]pyridin-3-ylmethyl)-3-phenyl-urea.

**12.** A compound according to claims 8 and 9, wherein said compound is selected from the group consisting of:

(6-Methyl-2-p-tolyl-imidazo[1,2-a]pyridin-3-ylmethyl)-carbamic acid p-tolyl ester;
(6-Methyl-2-p-tolyl-imidazol[1,2-a]pyridin-3-ylmethyl)-carbamic acid prop-2-ynyl ester;
(6-Methyl-2-p-tolyl-imidazo[1,2-a]pyridin-3-ylmethyl)-carbamic acid methyl ester;
(6-Methyl-2-p-tolyl-imidazo[1,2-a]pyridin-3-ylmethyl)-carbamic acid benzyl ester;
(6-Methyl-2-p-tolyl-imidazo[1,2-a]pyridin-3-ylmethyl)-carbamic acid 4-methoxy-phenyl ester;
(6-Methyl-2-p-tolyl-imidazo[1,2-a]pyridin-3-ylmethyl)-carbamic acid ethyl ester;
(6-Methyl-2-p-tolyl-imidazo[1,2-a]pyridin-3-ylmethyl)-carbamic acid phenyl ester; and
(6-Methyl-2-p-tolyl-imidazo[1,2-a]pyridin-3-ylmethyl)-carbamic acid isopropyl ester.

**13.** A process for preparing a compound of formula (I) or a pharmaceutically acceptable salt thereof, according to claim 1, comprising reacting intermediate (II):

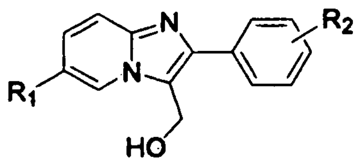

(II)

with the nitrile of the formula $R_4$-CN wherein $R_1$, $R_2$ and $R_4$ are as defined in (I).

**14.** A process for preparing a compound of formula (I) or a pharmaceutically acceptable salt thereof, according to claim 1, comprising reacting intermediate (III) :

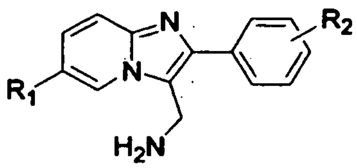

(III)

with an acyl chloride of the formula $R_4$-COCl, an isocyanate of the formula $R_4$-CNO or a chloroformiate of the formula $R_4$-OCOCl, wherein $R_1$, $R_2$ and $R_4$ are as defined in (I).

**15.** The process according to claim 14, further comprising reacting previously in the adequate acid conditions, an intermediate of formula (IV)

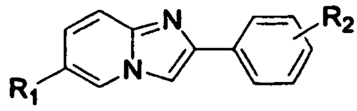

(IV)

with an intermediate of formula (V):

$CH_3CONHCH_2Q$ (V)

wherein Q is selected from the group consisting of -OH, -Oalkyl ($C_1$-$C_3$) , -N+(alkyl($C_1$-$C_3$)3Cl-, -N+(alkyl(C1-$C_3$)) 3Br-, -N+(alkyl($C_1$-$C_3$) ) 3I-, and then hydrolyzing the obtained intermediate (VI):

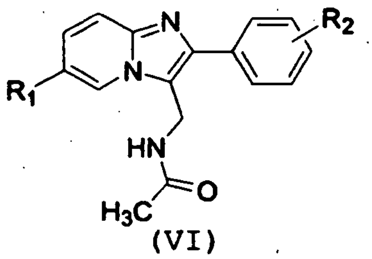

(VI)

to obtain said intermediate (III).

**16.** The process according to claim 15, comprising utilizing the intermediate of formula (V) wherein Q is -OH.

**17.** A composition comprising a compound of claim 1 in association with a therapeutically inert carrier.

**18.** The use of a compound of claim 1 for preparing a medicament for treating or preventing diseases associated with $GABA_A$ receptor modulation.

**19.** The use of claim 18 wherein the diseases are associated with $\alpha_1$-$GABA_A$ or $\alpha_2$-$GABA_A$ receptor modulation.

**20.** The use of a compound of claim 1 for preparing a medicament for treating or preventing anxiety, epilepsy, sleep disorders or insomnia; for inducing sedation-hypnosis, anesthesia or muscle relaxation; or for modulating the necessary time to induce sleep and its duration.

**Patentansprüche**

**1.** Imidazo[1,2-a]pyridinverbindung der Formel (1):

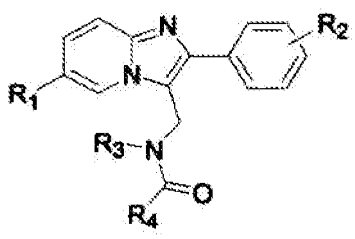

(I)

sowie pharmazeutisch verträgliche Salze davon;
worin

$R_1$ und $R_2$ unabhängig voneinander ausgewählt sind aus der Gruppe bestehend aus Wasserstoff, linearem oder verzweigtem Alkyl($C_1$-$C_6$), Alkenyl($C_2$-$C_6$), Alkinyl($C_2$-$C_6$), Halogenalkyl($C_1$-$C_6$), -O-Alkyl($C_1$-$C_6$), Fluor, Chlor und Brom;

$R_3$ ausgewählt ist aus der Gruppe bestehend aus Wasserstoff, linearem oder verzweigtem Alkyl($C_1$-$C_6$), Cycloalkyl ($C_3$-$C_6$), Cycloalkyl($C_3$-$C_6$)alkyl($C_1$-$C_6$), Alkenyl($C_2$-$C_6$), Alkenyl($C_2$-$C_6$)alkyl($C_1$-$C_6$), Alkinyl($C_2$-$C_6$), Alkinyl($C_2$-$C_6$) alkyl($C_2$-$C_6$);

$R_4$ ausgewählt ist aus der Gruppe bestehend aus Halogenalkyl($C_2$-$C_6$), Cycloalkyl($C_3$-$C_5$), Cycloalkyl($C_3$-$C_6$)alkyl ($C_1$-$C_6$), Alkinyl($C_2$-$C_6$)alkyl($C_1$-$C_6$), Alkyl($C_1$-$C_6$)-O-alkyl($C_1$-$C_6$), Alkyl($C_1$-$C_6$)-NH-alkyl($C_1$-$C_6$), Alkyl($C_1$-$C_6$)-N(di-alkyl($C_1$-$C_6$)), -$OR_5$, -$NHR_5$, -$NR_5R_6$,

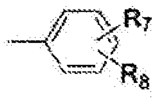 ,

Phenylalkyl($C_2$-$C_6$), Phenylalkenyl($C_2$-$C_6$), Naphthyl, monosubstituiertem Naphthyl, disubstituiertem Naphthyl, Naphthylalkyl($C_1$-$C_6$), Naphthylalkenyl($C_2$-$C_6$), Furyl, substituiertem Furyl, Benzofuryl, substituiertem Benzofuryl, Pyrrolyl, substituiertem Pyrrolyl, Isoxazolyl, substituiertem Isoxazolyl, Benzoisoxazolyl, substituiertem Benzoisoxazolyl, Imidazolyl, substituiertem Imidazolyl, Benzimidazolyl, substituiertem Benzimidazolyl, Indolyl, substituiertem Indolyl, Pyrazolyl, substituiertem Pyrazolyl, Thienyl, substituiertem Thienyl, Benzothienyl, substituiertem Benzothienyl, Thiazolyl, substituiertem Thiazolyl, Benzothiazolyl, substituiertem Benzothiazolyl, Chinolinyl, substituiertem Chinolinyl, Isochinolinyl, substituiertem Isochinolinyl, Pyridyl, substituiertem Pyridyl, Pyrazinyl, substituiertem Pyrazinyl, 6-Oxo-1,4,5,6-tetrahydropyridazinyl, substituiertem 6-Oxo-1,4,5,6-tetrahydropyridazinyl, Thiadiazolyl, substituiertem Thiadiazolyl, Isothiazolyl, substituiertem Isothiazolyl, Thienylmethyl, 2-Oxochromenyl, substituiertem 2-Oxochromenyl, 2-(Furan-2-yl)vinyl, Oxazolyl, substituiertem Oxazolyl und Benzisoxazolyl;

$R_5$ und $R_6$ unabhängig voneinander ausgewählt sind aus der Gruppe bestehend aus Wasserstoff, linearem oder verzweigtem Alkyl($C_1$-$C_6$), Phenylalkyl($C_1$-$C_6$), Halogenalkyl($C_1$-$C_6$), Cycloalkyl($C_3$-$C_6$), Cycloalkyl($C_3$-$C_6$)alkyl ($C_1$-$C_6$), Alkenyl($C_2$-$C_6$) und Alkinyl($C_2$-$C_6$), Alkenyl($C_2$-$C_6$)alkyl($C_1$-$C_6$), Alkinyl($C_2$-$C_6$)alkyl($C_1$-$C_6$), Phenyl, substituiertem Phenyl, Heteroaryl, substituiertem Heteroaryl;

und

$R_7$ und $R_8$ unabhängig voneinander ausgewählt sind aus der Gruppe bestehend aus linearem oder verzweigtem Alkyl($C_2$-$C_6$), Cycloalkyl($C_3$-$C_6$), Alkenyl($C_2$-$C_6$), Alkinyl($C_2$-$C_6$), -OH, -O-Alkyl($C_1$-$C_6$), -SH, -S-Alkyl($C_1$-$C_6$), Halogen-alkyl($C_1$-$C_6$), ω,ω,ω-Trifluoralkyl($C_1$-$C_6$), -NHAlkyl($C_1$-$C_6$), -NDialkyl($C_1$-$C_6$), -$NO_2$, -CN, -$SO_2$Alkyl($C_1$-$C_6$), -CO-Alkyl($C_1$-$C_6$), -COOAlkyl($C_1$-$C_6$), -CO-NHAlkyl($C_1$-$C_6$), -CONDialkyl($C_1$-$C_6$), Phenyl, substituiertem Phenyl, Heteroaryl und substituiertem Heteroaryl

und worin die Substituenten der Reste, welche substituiert sind, ausgewählt sind aus der Gruppe bestehend aus linearem oder verzweigtem Alkyl($C_2$-$C_6$), Cycloalkyl($C_3$-$C_6$), Alkenyl($C_2$-$C_6$), Alkinyl($C_2$-$C_6$), -OH, -O-Alkyl($C_1$-$C_6$), -SH, S-Alkyl($C_1$-$C_6$), Halogenalkyl($C_1$-$C_6$), ω,ω,ω-Trifluoralkyl($C_1$-$C_6$), -NHAlkyl($C_1$-$C_6$), -NDialkyl($C_1$-$C_6$), -$NO_2$, -CN, -$SO_2$Alkyl($C_1$-$C_6$), -COAlkyl($C_1$-$C_6$), -COOAlkyl($C_1$-$C_6$), -CO-NHAlkyl($C_1$-$C_6$), -CONDialkyl($C_1$-$C_6$), Phenyl, substituiertem Phenyl, Heteroaryl, substituiertem Heteroaryl, Fluor, Chlor und Brom.

**2.** Imidazo[1,2-a]pyridinverbindung der Formel (I):

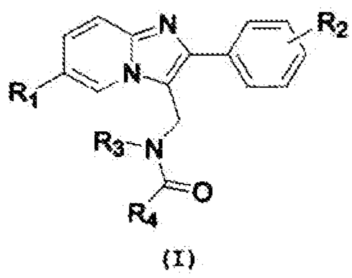

(I)

sowie pharmazeutisch verträgliche Salze davon;

worin

$R_1$ und $R_2$ unabhängig voneinander ausgewählt sind aus der Gruppe bestehend aus Wasserstoff, linearem oder

verzweigtem Alkyl($C_1$-$C_6$), Alkenyl($C_2$-$C_6$), Alkinyl($C_2$-$C_6$), Halogenalkyl($C_1$-$C_6$), -O-Alkyl($C_1$-$C_6$), Fluor, Chlor und Brom;

$R_3$ ausgewählt ist aus der Gruppe bestehend aus Wasserstoff, linearem oder verzweigtem Alkyl($C_1$-$C_6$), Cycloalkyl ($C_3$-$C_6$), Cycloalkyl($C_3$-$C_6$)alkyl($C_1$-$C_6$), Alkenyl($C_2$-$C_6$), Alkenyl($C_2$-$C_6$)alkyl($C_1$-$C_6$), Alkinyl($C_2$-$C_6$), Alkinyl($C_2$-$C_6$) alkyl($C_1$-$C_6$);

$R_4$ ausgewählt ist aus der Gruppe bestehend aus Halogenalkyl($C_2$-$C_6$), Cycloalkyl($C_3$-$C_5$), Cycloalkyl($C_3$-$C_6$)alkyl ($C_1$-$C_6$), Alkinyl($C_2$-$C_6$)alkyl($C_1$-$C_6$), Alkyl($C_1$-$C_6$)-O-alkyl($C_1$-$C_6$), Alkyl($C_1$-$C_6$)-NH-alkyl($C_1$-$C_6$), Alkyl($C_1$-C6)-N(dialkyl($C_1$-$C_6$)), -$OR_5$, -$NHR_5$, -$NR_5R_6$,

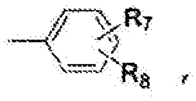

Phenylalkyl($C_2$-$C_6$), Phenylalkenyl($C_2$-$C_6$), Naphthyl, monosubstituiertem Naphthyl, disubstituiertem Naphthyl, Naphthylalkyl($C_1$-$C_6$), Naphthylalkenyl($C_2$-$C_6$), Furyl, substituiertem Furyl, Benzofuryl, substituiertem Benzofuryl, Pyrrolyl, substituiertem Pyrrolyl, Isoxazolyl, substituiertem Isoxazolyl, Benzoisoxazolyl, substituiertem Benzoisoxazolyl, Imidazolyl, substituiertem Imidazolyl, Benzimidazolyl, substituiertem Benzimidazolyl, Indolyl, substituiertem Indolyl, Pyrazolyl, substituiertem Pyrazolyl, Thienyl, substituiertem Thienyl, Benzothienyl, substituiertem Benzothienyl, Thiazolyl, substituiertem Thiazolyl, Benzothiazolyl, substituiertem Benzothiazolyl, Chinolinyl, substituiertem Chinolinyl, Isochinolinyl, substituiertem Isochinolinyl, Pyridyl und substituiertem Pyridyl; $R_5$ und $R_6$ unabhängig voneinander ausgewählt sind aus der Gruppe bestehend aus Wasserstoff, linearem oder verzweigtem Alkyl($C_1$-$C_6$), Phenylalkyl($C_1$-$C_6$), Halogenalkyl($C_1$-$C_6$), Cycloalkyl($C_3$-$C_6$), Cycloalkyl($C_3$-$C_6$)alkyl($C_1$-$C_6$), Alkenyl($C_2$-$C_6$) und Alkinyl ($C_2$-$C_6$), Alkenyl($C_2$-$C_6$)alkyl($C_1$-$C_6$), Alkinyl($C_2$-$C_6$)alkyl($C_1$-$C_6$), Phenyl, substituiertem Phenyl, Heteroaryl, substituiertem Heteroaryl;

und

$R_7$ und $R_8$ unabhängig voneinander ausgewählt sind aus der Gruppe bestehend aus linearem oder verzweigtem Alkyl($C_2$-$C_6$), Cycloalkyl($C_3$-$C_6$), Alkenyl($C_2$-$C_6$), Alkinyl($C_2$-$C_6$), -OH, -O-Alkyl($C_1$-$C_6$), -SH, -S-Alkyl($C_1$-$C_6$), Halogen-alkyl($C_1$-$C_6$), ω,ω,ω-Trifluoralkyl($C_1$-$C_6$), -NHAlkyl($C_1$-$C_6$), -NDialkyl($C_1$-$C_6$), -$NO_2$, -CN, -$SO_2$Alkyl($C_1$-$C_6$), -CO-Alkyl($C_1$-$C_6$), -COOAlkyl($C_1$-$C_6$), -CO-NHAlkyl($C_1$-$C_6$), -CONDialkyl($C_1$-$C_6$), Phenyl, substituiertem Phenyl, Heteroaryl und substituiertem Heteroaryl.

3. Verbindung nach Anspruch 1, worin $R_1$ eine Methylgruppe ist und $R_2$ eine Methylgruppe in para-Stellung ist; und $R_3$ ausgewählt ist aus der Gruppe bestehend aus Wasserstoff, Methyl, Ethyl, n-Propyl, i-Propyl, Cyclopropyl und 2-Propinyl.

4. Verbindung nach Anspruch 3, worin $R_4$ ausgewählt ist aus der Gruppe bestehend aus Cyclopropyl, Cyclobutyl, 2-Propinyl, N,N-Dimethyl-4-aminophenyl, 2-Furyl, 5-$NO_2$-2-furyl, 2-Pyrrolyl, 2-Thienyl, 2-Pyridyl, 4,6-Difluor-2-pyridyl, 2-Chlor-4-pyridyl, 4-Pyridyl, 5-Methyl-2-pyrazinyl, 6-Oxo-1,4,5,6-tetrahydro-pyridazin-3-yl, [1,2,3]Thiadiazol-4-yl, 2-Thienylmethyl, 1-Methyl-1 H-imidazol-2-yl, 4-Thiazolyl, 2,5-Dimethyl-4-oxazolyl und 3,5-Dimethyl-4-isoxazolyl.

5. Verbindung nach Anspruch 4, worin $R_4$ ausgewählt ist aus der Gruppe bestehend aus Cyclopropyl, 2-Propinyl, N, N-Dimethyl-4-aminophenyl, 2-Furyl, 5-$NO_2$-2-furyl, 2-Pyrrolyl, 2-Thienyl, 2-Pyridyl, 4,6-Difluor-2-pyridyl, 2-Chlor-4-pyridyl und 4-Pyridyl.

6. Verbindung nach Anspruch 3, worin $R_4$ -$NR_5R_6$ is.

7. Verbindung nach Anspruch 6, worin $R_5$ Wasserstoff oder Methyl ist; und $R_6$ ausgewählt ist aus der Gruppe bestehend aus Methyl, Ethyl, n-Propyl, i-Propyl, Cyclopropyl, Cyclopentyl, Cyclohexyl, 2-Propinyl und Phenyl.

8. Verbindung nach Anspruch 3, worin $R_4$ -$OR_5$ ist.

9. Verbindung nach Anspruch 8, worin $R_5$ ausgewählt ist aus der Gruppe bestehend aus Methyl, Ethyl, n-Propyl, i-Propyl, Cyclopropyl, Cyclopentyl, Cyclohexyl, 2-Propinyl, 4-Methyl-phenyl, 4-Methoxy-phenyl und Phenyl.

10. Verbindung nach Anspruch 4, wobei die Verbindung ausgewählt ist aus der Gruppe bestehend aus:

Furan-2-carbonsäure-(6-methyl-2-p-tolyl-imidazo[1,2-a]pyridin-3-ylmethyl)-amid;

Pyridin-2-carbonsäure-(6-methyl-2-p-tolyl-imidazo[1,2-a]pyridin-3-ylmethyl)-amid;
Thiophen-2-carbonsäure-(6-methyl-2-p-tolyl-imidazo[1,2-a]pyridin-3-ylmethyl)-amid;
Cyclopropancarbonsäure-(6-methyl-2-p-tolyl-imidazo[1,2-a]pyridin-3-ylmethyl)-amid;
5-Nitro-furan-2-carbonsäure-(6-methyl-2-p-tolyl-imidazo[1,2-a]pyridin-3-ylmethyl)-amid;
3,5-Difluor-pyridin-2-carbonsäure-(6-methyl-2-p-tolyl-imidazo[1,2-a]pyridin-3-ylmethyl)-amid;
6-Methoxy-benzothiazol-2-carbonsäure-(6-methyl-2-p-tolyl-imidazo[1,2-a]pyridin-3-ylmethyl)-amid;
4-Dimethylamino-N-methyl-N-(6-methyl-2-p-tolyl-imidazo[1,2-a]pyridin-3-ylmethyl)-benzamid;
Cyclopropancarbonsäure-methyl-(6-methyl-2-p-tolyl-imidazo[1,2-a]pyridin-3-ylmethyl)-amid;
Pyridin-2-carbonsäure-methyl-(6-methyl-2-p-tolyl-imidazo[1,2-a]pyridin-3-ylmethyl)-amid;
Thiophen-2-carbonsäure-methyl-(6-methyl-2-p-tolyl-imidazo[1,2-a]pyridin-3-ylmethyl)-amid;
5-Nitro-furan-2-carbonsäure-methyl-(6-methyl-2-p-tolyl-imidazo[1,2-a]pyridin-3-ylmethyl)-amid;
2-Chlor-N-methyl-N-(6-methyl-2-p-tolyl-imidazo[1,2-a]pyridin-3-ylmethyl)-isonicotinamid;
Cyclobutancarbonsäure-(6-methyl-2-p-tolyl-imidazo[1,2-a]pyridin-3-ylmethyl)-amid;
5-Methyl-pyrazin-2-carbonsäure-(6-methyl-2-p-tolyl-imidazo[1,2-a]pyridin-3-ylmethyl)-amid;
6-Oxo-1,4,5,6-tetrahydro-pyridazin-3-carbonsäure-(6-methyl-2-p-tolyl-imidazo[1,2-a]pyridin-3-ylmethyl)-amid;
[1,2,3]Thiadiazol-4-carbonsäure-(6-methyl-2-p-tolyl-imidazo[1,2-a]pyridin-3-ylmethyl)-amid;
N-(6-Methyl-2-p-tolyl-imidazo[1,2-a]pyridin-3-ylmethyl)-2-thiophen-2-yl-acetamid;
1-Methyl-1 H-imidazol-2-carbonsäure-(6-methyl-2-p-tolyl-imidazo[1,2-a]pyridin-3-ylmethyl)-amid;
Thiazol-4-carbonsäure-(6-methyl-2-p-tolyl-imidazo[1,2-a]pyridin-3-ylmethyl)-amid;
2,5-Dimethyl-oxazol-4-carbonsäure-(6-methyl-2-p-tolyl-imidazo[1,2-a]pyridin-3-ylmethyl)-amid;
3,5-Dimethyl-isoxazol-4-carbonsäure-(6-methyl-2-p-tolyl-imidazo[1,2-a]pyridin-3-ylmethyl)-amid; und
Thiazol-4-carbonsäure-methyl-(6-methyl-2-p-tolyl-imidazo[1,2-a]pyridin-3-ylmethyl)-amid.

**11.** Verbindung nach den Ansprüchen 6 und 7, wobei die Verbindung ausgewählt ist aus der Gruppe bestehend aus:

1-(4-Dimethylamino-phenyl)-3-(6-methyl-2-p-tolyl-imidazo[1,2-a]pyridin-3-ylmethyl)-harnstoff;
1-Ethyl-3-(6-methyl-2-p-tolyl-imidazo[1,2-a]pyridin-3-ylmethyl)-harnstoff;
1-Isopropyl-3-(6-methyl-2-p-tolyl-imidazo[1,2-a]pyridin-3-ylmethyl)-harnstoff;
1-Cyclopentyl-3-(6-methyl-2-p-tolyl-imidazo[1,2-a]pyridin-3-ylmethyl)-harnstoff;
1-Cyclohexyl-3-(6-methyl-2-p-tolyl-imidazo[1,2-a]pyridin-3-ylmethyl)-harnstoff; und
1-(6-Methyl-2-p-tolyl-imidazo[1,2-a]pyridin-3-ylmethyl)-3-phenyl-harnstoff.

**12.** Verbindung nach den Ansprüchen 8 und 9, wobei die Verbindung ausgewählt ist aus der Gruppe bestehend aus:

(6-Methyl-2-p-tolyl-imidazo[1,2-a]pyridin-3-ylmethyl)-carbaminsäure-p-tolylester;
(6-Methyl-2-p-tolyl-imidazo[1,2-a]pyridin-3-ylmethyl)-carbaminsäure-prop-2-inylester;
(6-Methyl-2-p-tolyl-imidazo[1,2-a]pyridin-3-ylmethyl)-carbaminsäure-methylester;
(6-Methyl-2-p-tolyl-imidazo[1,2-a]pyridin-3-ylmethyl)-carbaminsäure-benzylester;
(6-Methyl-2-p-tolyl-imidazo[1,2-a]pyridin-3-ylmethyl)-carbaminsäure-4-methoxy-phenylester;
(6-Methyl-2-p-tolyl-imidazo[1,2-a]pyridin-3-ylmethyl)-carbaminsäure-ethylester;
(6-Methyl-2-p-tolyl-imidazo[1,2-a]pyridin-3-ylmethyl)-carbaminsäure-phenylester; und
(6-Methyl-2-p-tolyl-imidazo[1,2-a]pyridin-3-ylmethyl)-carbaminsäure-isopropylester.

**13.** Verfahren zum Herstellen einer Verbindung der Formel (I) oder eines pharmazeutisch verträglichen Salzes davon, gemäß Anspruch 1, umfassend das Umsetzen von Zwischenprodukt (II):

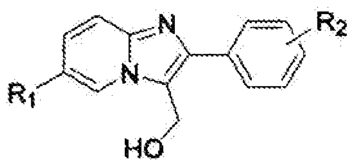

(II)

mit dem Nitril der Formel $R_4$-CN, worin $R_1$, $R_2$ und $R_4$ wie in (I) definiert sind.

**14.** Verfahren zum Herstellen einer Verbindung der Formel (I) oder eines pharmazeutisch verträglichen Salzes davon,

gemäß Anspruch 1, umfassend das Umsetzen von Zwischenprodukt (III):

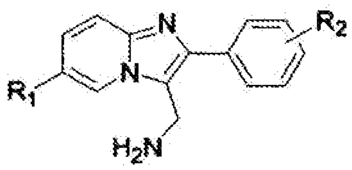

(III)

mit einem Acylchlorid der Formel $R_4$-COCl, einem Isocyanat der Formel $R_4$-CNO oder einem Chlorformiat der Formel $R_4$-OCOCl, worin $R_1$, $R_2$ und $R_4$ wie in (I) definiert sind.

**15.** Verfahren nach Anspruch 14, außerdem umfassend das vorherige Umsetzen eines Zwischenprodukts der Formel (IV) unter den angemessenen sauren Bedingungen

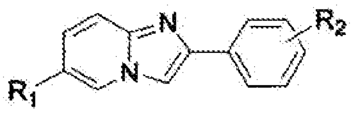

(IV)

mit einem Zwischenprodukt der Formel (V):

$CH_3CONHCH_2Q$ (V)

worin Q ausgewählt ist aus der Gruppe bestehend aus -OH, -OAlkyl($C_1$-$C_3$), -$N^+$(Alkyl($C_1$-$C_3$))$_3Cl^-$, -$N^+$(Alkyl($C_1$-$C_3$))$_3Br^-$, -$N^+$(Alkyl($C_1$-$C_3$))$_3I^-$, und anschließend das Hydrolysieren des erhaltenen Zwischenprodukts (VI):

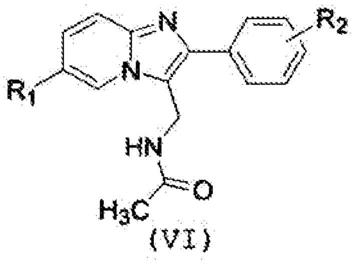

(VI)

um das Zwischenprodukt (III) zu erhalten.

**16.** Verfahren nach Anspruch 15, umfassend das Einsetzen des Zwischenprodukts der Formel (V), worin Q -OH ist.

**17.** Zusammensetzung umfassend eine Verbindung nach Anspruch 1 in Verbindung mit einem therapeutisch inerten Träger.

**18.** Verwendung einer Verbindung nach Anspruch 1 zum Herstellen eines Medikamentes zum Behandeln oder Verhindern von Krankheiten, die mit der Modulation des $GABA_A$-Rezeptors zusammenhängen.

**19.** Verwendung nach Anspruch 18, wobei die Krankheiten mit der Modulation des $\alpha_1$-$GABA_A$ oder $\alpha_2$-$GABA_A$-Rezeptors zusammenhängen.

**20.** Verwendung einer Verbindung nach Anspruch 1 zum Herstellen eines Medikaments zum Behandeln oder Verhüten von Angst, Epilepsie, Schlafstörungen oder Schlaflosigkeit; zum Einleiten einer Sedierung-Hypnose, Anästhesie

oder Muskelrelaxation; oder zum Modulieren der erforderlichen Zeit zum Herbeiführen des Schlafes und seiner Dauer.

## Revendications

**1.** Composé d'imidazo[1,2-a]pyridine de formule (I) :

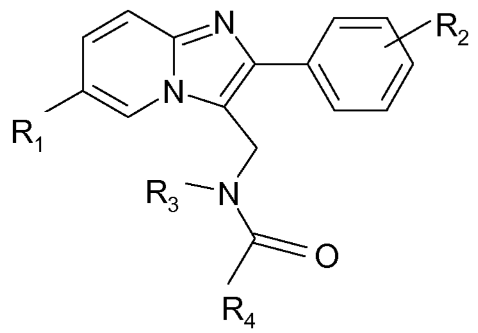

(I)

ainsi que ses sels pharmaceutiquement acceptables ;
où
$R_1$ et $R_2$ sont choisis indépendamment dans le groupe consistant en l'hydrogène, alkyl($C_1$-$C_6$) et linéaire ou ramifié, alcényl($C_2$-$C_6$), alcynyl($C_2$-$C_6$), haloalkyl($C_1$-$C_6$), -O-alkyl($C_1$-$C_6$), fluoro, chloro et bromo ;
$R_3$ est choisi dans le groupe consistant en l'hydrogène, alkyl($C_1$-$C_6$) linéaire ou ramifié, cycloalkyl($C_3$-$C_6$), cycloalkyl ($C_3$-$C_6$)alkyl($C_1$-$C_6$), alcényl($C_2$-$C_6$), alcényl($C_2$-$C_6$)alkyl($C_1$-$C_6$), alcynyl($C_2$-$C_6$), alcynyl($C_2$-$C_6$)alkyl($C_1$-$C_6$) ;
$R_4$ est choisi dans le groupe consistant en haloalkyl($C_2$-$C_6$), cycloalkyl ($C_3$-$C_5$), cycloalkyl($C_3$-$C_6$)alkyl($C_1$-$C_6$), alcynyl ($C_2$-$C_6$)alkyl($C_1$-$C_6$), alkyl($C_1$-$C_6$)-O-alkyl($C_1$-$C_6$), alkyl($C_1$-$C_6$)-NH-alkyl($C_1$-$C_6$), alkyl($C_1$-$C_6$)-N-(dialkyl($C_1$-$C_6$)), -$OR_5$, -$NHR_5$, -$NR_5R_6$,

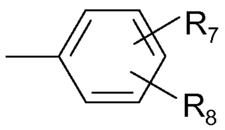

,

phénylalkyl($C_2$-$C_6$), phénylalcényl($C_2$-$C_6$), naphtyle monosubstitué, naphtyle, naphtyle disubstitué, naphtylalkyl ($C_1$-$C_6$), naphtylalcényl($C_2$-$C_6$), furyle, furyle substitué, benzofuryle, benzofuryle substitué, pyrrolyle, pyrrolyle substitué, isoxazolyle, isoxazolyle substitué, benzoisoxazolyle, benzoisoxazolyle substitué, imidazolyle, imidazolyle substitué, benzimidazolyle, benzimidazolyle substitué, indolyle, indolyle substitué, pyrazolyle, pyrazolyle substitué, thiényle, thiényle substitué, benzothiényle, benzothiényle substitué, thiazolyle, thiazolyle substitué, benzothiazolyle, benzothiazolyle substitué, quinolinyle, quinolinyle substitué, isoquinolinyle, isoquinolinyle substitué, pyridyle, pyridyle substitué, pyrazinyle, pyrazinyle substitué, 6-oxo-1,4,5,6-tétrahydropyridazinyle, 6-oxo-1,4,5,6-tétrahydropyridazinyle substitué, thiadiazolyle, thiadiazolyle substitué, isothiazolyle, isothiazolyle substitué, thiénylméthyle, 2-oxochroményle, 2-oxochroményle substitué, 2-(furan-2-yl)vinyle, oxazolyle, oxazolyle substitué et benzisoxazolyle ;
$R_5$ et $R_6$ sont choisis indépendamment dans le groupe consistant en l'hydrogène, alkyl($C_1$-$C_6$) linéaire ou ramifié, phénylalkyl($C_1$-$C_6$), haloalkyl($C_1$-$C_6$), cycloalkyl($C_3$-$C_6$), cycloalkyl($C_3$-$C_6$)alkyl($C_1$-$C_6$), alcényl($C_2$-$C_6$) et alcynyl ($C_2$-$C_6$), alcényl($C_2$-$C_6$), alcynyl($C_2$-$C_6$)alkyl($C_1$-$C_6$), phényle, phényle substitué, hétéroaryle, hétéroaryle substitué ;
et
$R_7$ et $R_8$ sont choisis indépendamment dans le groupe consistant en alkyl($C_2$-$C_6$), cycloalkyl($C_3$-$C_6$), alcényl($C_2$-$C_6$), alcynyl($C_2$-$C_6$), -OH, -O-alkyl($C_1$-$C_6$), -SH, -S-alkyl($C_1$-$C_6$), halo-alkyl($C_1$-$C_6$), ω,ω,ω-trifluoroalkyl($C_1$-$C_6$), -NHalkyl ($C_1$-$C_6$), -Ndialkyl($C_1$-$C_6$), -$NO_2$, -CN, -$SO_2$alkyl($C_1$-$C_6$), -COalkyl($C_1$-$C_6$), -COOalkyl($C_1$-$C_6$), -CO-NHalkyl($C_1$-$C_6$), -CONdialkyl($C_1$-$C_6$), phényle, phényle substitué, hétéroaryle et hétéroaryle substitué
et où les substituants des radicaux qui sont substitués sont choisis dans le groupe consistant en alkyl($C_2$-$C_6$), cycloalkyl($C_3$-$C_6$), alcényl($C_2$-$C_6$), alcynyl($C_2$-$C_6$), -OH, -O-alkyl($C_1$-$C_6$), -SH, S-alkyl($C_1$-$C_6$), halo-alkyl($C_1$-$C_6$), ω, ω,ω-trifluoroalkyl($C_1$-$C_6$), -NHalkyl($C_1$-$C_6$), -Ndialkyl($C_1$-$C_6$), -$NO_2$, -CN, -$SO_2$alkyl($C_1$-$C_6$), -COalkyl($C_1$-$C_6$), -COalk-

yl($C_1$-$C_6$), -CO-NHalkyl($C_1$-$C_6$), -CONdialkyl($C_1$-$C_6$), phényle, phényle substitué, hétéroaryle, hétéroaryle substitué, fluoro, chloro et bromo.

**2.** Composé d'imidazo[1,2-a]pyridine de formule (I) :

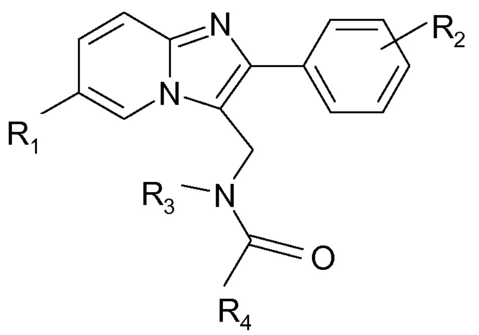

(I)

ainsi que ses sels pharmaceutiquement acceptables ;
où
$R_1$ et $R_2$ sont choisis indépendamment dans le groupe consistant en l'hydrogène, alkyl($C_1$-$C_6$) et linéaire ou ramifié, alcényl($C_2$-$C_6$), alcynyl($C_2$-$C_6$), haloalkyl($C_1$-$C_6$), -O-alkyl($C_1$-$C_6$), fluoro, chloro et bromo ;
$R_3$ est choisi dans le groupe consistant en l'hydrogène, alkyl($C_1$-$C_6$) linéaire ou ramifié, cycloalkyl($C_3$-$C_6$), cycloalkyl ($C_3$-$C_6$)alkyl($C_1$-$C_6$), alcényl($C_2$-$C_6$), alcényl($C_2$-$C_6$)alkyl($C_1$-$C_6$), alcynyl($C_2$-$C_6$), alcynyl($C_2$-$C_6$)alkyl($C_1$-$C_6$) ;
$R_4$ est choisi dans le groupe consistant en haloalkyl($C_2$-$C_6$), cycloalkyl ($C_3$-$C_5$), cycloalkyl($C_3$-$C_6$)alkyl($C_1$-$C_6$), alcynyl ($C_2$-$C_6$)alkyl($C_1$-$C_6$), alkyl($C_1$-$C_6$)-O-alkyl($C_1$-$C_6$), alkyl($C_1$-$C_6$)-NH-alkyl($C_1$-$C_6$), alkyl($C_1$-$C_6$)-N-(dialkyl($C_1$-$C_6$)), -$OR_5$, -$NHR_5$, -$NR_5R_6$,

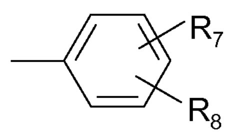

,

phénylalkyl($C_2$-$C_6$), phénylalcényl($C_2$-$C_6$), naphtyle monosubstitué, naphtyle, naphtyle disubstitué, naphtylalkyl ($C_1$-$C_6$), naphtylalcényl($C_2$-$C_6$), furyle, furyle substitué, benzofuryle, benzofuryle substitué, pyrrolyle, pyrrolyle substitué, isoxazolyle, isoxazolyle substitué, benzoisoxazolyle, benzoisoxazolyle substitué, imidazolyle, imidazolyle substitué, benzimidazolyle, benzimidazolyle substitué, indolyle, indolyle substitué, pyrazolyle, pyrazolyle substitué, thiényle, thiényle substitué, benzothiényle, benzothiényle substitué, thiazolyle, thiazolyle substitué, benzothiazolyle, benzothiazolyle substitué, quinolinyle, quinolinyle substitué, isoquinolinyle, isoquinolinyle substitué, pyridyle, pyridyle substitué.
$R_5$ et $R_6$ sont choisis indépendamment dans le groupe consistant en l'hydrogène, alkyl($C_1$-$C_6$) linéaire ou ramifié, phénylalkyl($C_1$-$C_6$), haloalkyl($C_1$-$C_6$), cycloalkyl($C_3$-$C_6$), cycloalkyl($C_3$-$C_6$)alkyl($C_1$-$C_6$), alcényl($C_2$-$C_6$) et alcynyl ($C_2$-$C_6$), alcényl($C_2$-$C_6$), alcynyl($C_2$-$C_6$)alkyl($C_1$-$C_6$), phényle, phényle substitué, hétéroaryle, hétéroaryle substitué.
$R_7$ et $R_8$ sont choisis indépendamment dans le groupe consistant en alkyl($C_2$-$C_6$), cycloalkyl($C_3$-$C_6$), alcényl($C_2$-$C_6$), alcynyl($C_2$-$C_6$), -OH, -O-alkyl($C_1$-$C_6$), -SH, -S-alkyl($C_1$-$C_6$), halo-alkyl($C_1$-$C_6$), ω,ω,ω-trifluoroalkyl($C_1$-$C_6$),- NHalkyl ($C_1$-$C_6$), -Ndialkyl($C_1$-$C_6$), -$NO_2$, -CN, -$SO_2$alkyl($C_1$-$C_6$), -COalkyl($C_1$-$C_6$), -COOalkyl($C_1$-$C_6$), -CO-NHalkyl($C_1$-$C_6$), -CONdialkyl($C_1$-$C_6$), phényle, phényle substitué, hétéroaryle et hétéroaryle substitué.

**3.** Composé selon la revendication 1 où $R_1$ est un groupe méthyle et $R_2$ est un groupe méthyle en position para ; et $R_3$ est choisi dans le groupe consistant en l'hydrogène, méthyle, éthyle, n-propyle, i-propyle, cyclopropyle et 2-propynyle.

**4.** Composé selon la revendication 3 où $R_4$ est choisi dans le groupe consistant en cyclopropyle, cyclobutyle, 2-propynyle, N,N-diméthyl-4-aminophényle, 2-furyle, 5-$NO_2$-2-furyle, 2-pyrrolyle, 2-thiényle, 2-pyridyle, 4,6-difluoro-2-pyridyle, 2-chloro-4-pyridyle, 4-pyridyle, 5-méthyl-2-pyrazinyle, 6-oxo-1,4,5,6-tétrahydropyridazin-3-yle,

[1,2,3]-thiadiazol-4-yle, 2-thiénylméthyle, 1-méthyl-1H-imidazol-2-yle, 4-thiazolyle, 2,5-diméthyl-4-oxazolyle et 3,5-diméthyl-4-isoxazolyle.

**5.** Composé selon la revendication 4, où $R_4$ est choisi dans le groupe consistant en cyclopropyle, 2-propynyle, N,N-diméthyl-4-amino-phényle, 2-furyle, 5-$NO_2$-2-furyle, 2-pyrrolyle, 2-thiényle, 2-pyridyle, 4,6-difluoro-2-pyridyle, 2-chloro-4-pyridyle et 4-pyridyle.

**6.** Composé selon la revendication 3, où $R_4$ est -$NR_5R_6$.

**7.** Composé selon la revendication 6 où $R_5$ est l'hydrogène ou méthyle ; et $R_6$ est choisi dans le groupe consistant en méthyle, éthyle, n-propyle, i-propyle, cyclopropyle, cyclopentyle, cyclohexyle, 2-propinyle et phényle.

**8.** Composé selon la revendication 3 où $R_4$ est -$OR_5$.

**9.** Composé selon la revendication 8 où $R_5$ est choisi dans le groupe consistant en méthyle, éthyle, n-propyle, i-propyle, cyclopropyle, cyclopentyle, cyclohexyle, 2-propinyle, 4-méthyl-phényle, 4-méthoxy-phényle et phényle.

**10.** Composé selon la revendication 4 où ledit composé est choisi dans le groupe consistant en :

(6-méthyl-2-p-tolyl-imidazo[1,2-a]pyridin-3-ylméthyl)-amide d'acide furane-2-carboxylique ;
(6-méthyl-2-p-tolyl-imidazo[1,2-a]pyridin-3-ylméthyl)-amide d'acide pyridine-2-carboxylique ;
(6-méthyl-2-p-tolyl-imidazo[1,2-a]pyridin-3-ylméthyl)-amide d'acide thiophène-2-carboxylique ;
(6-méthyl-2-p-tolyl-imidazo[1,2-a]pyridin-3-ylméthyl)-amide d'acide cyclopropanecarboxylique ;
(6-méthyl-2-p-tolyl-imidazo[1,2-a]pyridin-3-ylméthyl)-amide d'acide 5-nitro-furane-2-carboxylique ;
(6-méthyl-2-p-tolyl-imidazo[1,2-a]pyridin-3-ylméthyl)-amide d'acide 3,5-difluoro-pyridine-2-carboxylique ;
(6-méthyl-2-p-tolyl-imidazo[1,2-a]pyridin-3-ylméthyl)-amide d'acide 6-méthoxy-benzothiazole-2-carboxylique ;
4-diméthylamino-N-méthyl-N-(6-(6-méthyl-2-p-tolylimidazo[1,2-a]pyridin-3-ylméthyl)-benzamide ;
(6-méthyl-2-p-tolyl-imiedazo[1,2-a]pyridin-3-ylméthyl)-amide d'acide cyclopropanecarboxylique ;
(6-méthyl-2-p-tolyl-imidazo[1,2-a]pyridin-3-ylméthyl)-amide d'acide pyridine-2-carboxylique ;
(6-méthyl-2-p-tolyl-imidazo[1,2-a]pyridin-3-ylméthyl)-amide d'acide thiophène-2-carboxylique ;
(6-méthyl-2-ptolyl-imidazo[1,2-a]pyridin-3-ylméthyl)-amide d'acide 5-nitro-furane-2-carboxylique ;
2-Chloro-N-méthyl-N-(6-méthyl-2-p-tolyl-imidazo[1,2-a]pyridin-3-yl-méthyl)-isonicotinamide ;
(6-méthyl-2-p-tolyl-imidazo[1,2-a]pyridin-3-ylméthyl)-amide d'acide cyclobutanecarboxylique ;
(6-méthyl-2-p-tolyl-imidazo[1,2-a]pyridin-3-ylméthyo)-amide d'acide 5-méthyl-pyrazine-2-carboxylique ;
(6-méthyl-2-p-tolyl-imidazo[1,2-a]pyridin-3-ylméthyl)-amide d'acide 6-oxo-1,4,5,6-tétrahydro-pyridazine-3-carboxylique ;
(6-méthyl-2-p-tolyl-imidazo[1,2-a]pyridin-3-ylméthyl)-amide d'acide [1,2,3]thiadiazole-4-carboxylique ;
N-(6-Méthyl-2-p-tolyl-imidazo[1,2-a]pyridin-3-ylméthyl)-2-thiophèn-2-yl-acétamide ;
(6-méthyl-2-p-tolyl-imidazo[1,2-a]pyridin-3-ylméthyl)-amide d'acide 1-méthyl-1H-imidazole-2-carboxylique ;
(6-méthyl-2-p-tolyl-imidazo[1,2-a]pyridin-3-ylméthyl)-amide d'acide thiazole-4-carboxylique ;
(6-méthyl-2-p-tolyl-imidazo[1,2-a]pyridin-3-ylméthyl)-amide d'acide 2,5-diméthyl-oxazole-4-carboxylique ;
(6-méthyl-2-p-tolyl-imidazo[1,2-a]pyridin-3-ylméthyl)-amide d'acide 3,5-diméthyl-isoxazole-4-carboxylique ; et
(6-méthyl-2-p-tolyl-imidazo[1,2-a]pyridin-3-ylméthyl)-amide d'acide thiazole-4-carboxylique.

**11.** Composé selon la revendication 6 et 7 où ledit composé est choisi dans le groupe consistant en :

1-(4-diméthylamino-pyényl)-3-(6-méthyl-2-p-tolyl-imidazo[1,2-a]pyridin-3-ylméthyl)-urée ;
1-Ethyl-3-(6-méthyl-2-p-tolyl-imidazo[1,2-a]pyridin-3-ylméthyl)-urée ;
1-Isopropyl-3-(6-méthyl-2-p-tolyl-imidazo[1,2-a]pyridin-3-ylméthyl)-urée ;
1-Cyclopentyl-3-(6-méthyl-2-p-tolyl-imidazo[1,2-a]pyridin-3-ylméthyl)-urée ;
1-Cyclohexyl-3-(6-méthyl-2-p-tolyl-imidazo[1,2-a]pyridin-3-ylméthyl)-u rée ; et
1-(6-Méthyl-2-p-tolyl-imidazo[1,2-a]pyridin-3-ylméthyl)-3-phényl-urée.

**12.** Composition selon les revendications 8 et 9 où ledit composé est choisi dans le groupe consistant en :

p-tolylester d'acide (6-méthyl-2-p-tolyl-imidazo[1,2-a]pyridin-3-ylméthyl)-carbamique ;
prop-2-ynylester d'acide (6-méthyl-2-p-tolyl-imidazo[1,2-a]pyridin-3-ylméthyl)-carbamique ;
méthylester d'acide (6-méthyl-2-p-tolyl-imidazo[1,2-a]pyridin-3-ylméthyl)-carbamique ;

benzylester d'acide (6-méthyl-2-p-tolyl-imidazo[1,2-a]pyridin-3-ylméthyl)-carbamique ;
4-méthoxy-phénylester d'acide (6-méthyl-2-p-tolyl-imidazo[1,2-a]pyridin-3-ylméthyl)-carbamique ;
éthylester d'acide (6-méthyl-2-p-tolyl-imidazo[1,2-a]pyridin-3-ylméthyl)-carbamique ;
phénylester d'acide (6-méthyl-2-p-tolyl-imidazo[1,2-a]pyridin-3-ylméthyl)-carbamique ; et
isopropylester d'acide (6-méthyl-2-p-tolyl-imidazo[1,2-a]pyridin-3-ylméthyl)-carbamique.

**13.** Procédé pour préparer un composé de formule (I) ou un sel pharmaceutiquement acceptable de celui-ci, selon la revendication 1, comprenant la réaction d'un intermédiaire (II) :

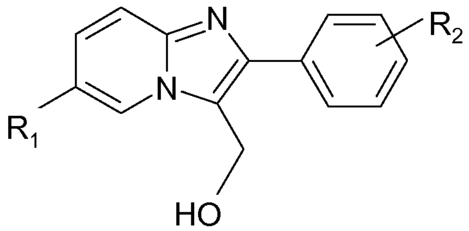

(II)

avec le nitrile de formule $R_4$-CN où $R_1$, $R_2$ et $R_4$ sont définis comme dans (I).

**14.** Procédé pour préparer un composé de formule (I) ou un sel pharmaceutiquement acceptable de celui-ci, selon la revendication 1, comprenant la réaction d'un intermédiaire (III) :

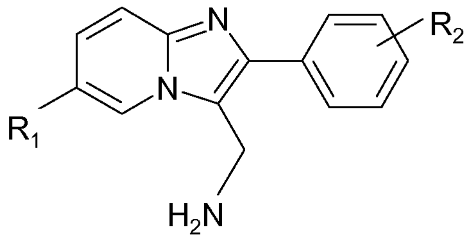

(III)

avec un chlorure d'acide de formule $R_4$-COCl, un isocyanate de formule $R_4$-CNO ou un chloroformiate de formule $R_4$-OCOCl, où $R_1$, $R_2$ et $R_4$ sont définis comme dans (I).

**15.** Procédé selon la revendication 14 comprenant en outre la réaction préalable dans les conditions acide adéquat d'un intermédiaire de formule (IV)

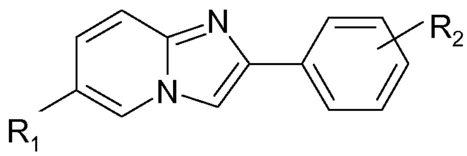

(IV)

avec un intermédiaire de formule (V) :

$CH_3CONHCH_2Q$ (V)

où Q est choisi dans le groupe consistant en -OH, -Oalkyl($C_1$-$C_3$), -N+(alkyl($C_1$-$C_3$))3Cl-, -N+(alkyl(Cl-$C_3$))3Br-, -N+(alkyl($C_1$-$C_3$))3I-, puis hydrolyse de l'intermédiaire (VI) obtenu :

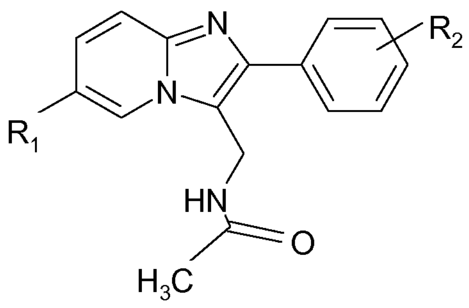

(VI)

pour obtenir ledit intermédiaire (III).

**16.** Procédé selon la revendication 15 comprenant l'utilisation d'un intermédiaire de formule (V) où Q est -OH.

**17.** Composition comprenant un composé selon la revendication 1 en association avec un vecteur thérapeutiquement inerte.

**18.** Utilisation d'un composé selon la revendication 1 pour préparer un médicament pour traiter ou prévenir les maladies associées avec la modulation des récepteurs $GABA_A$.

**19.** Utilisation selon la revendication 18 où les maladies sont associées avec la modulation des récepteurs $\alpha_1$-$GABA_A$ ou $\alpha_2$-$GABA_A$.

**20.** Utilisation d'un composé selon la revendication 1 pour préparer un médicament pour traiter ou prévenir l'anxiété, l'épilepsie, les troubles du sommeil ou l'insomnie ; pour induire une sédation-hypnose, une anesthésie ou une relaxation musculaire ; ou pour moduler le temps nécessaire pour induire le sommeil et sa durée.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4382938 A
- FR 2593818
- US 4650796 A
- EP 172096 A
- US 4626538 A
- US 4654347 A
- US 6399621 B
- EP 129847 A
- EP 430738 A


### Non-patent literature cited in the description


- **H. MÖHLER et al.** *J. Pharmacol. Exp. Ther.,* 2002, vol. 300, 2-8
- **H. MÖHLER et al.** *Curr. Opin. Pharmacol.,* 2001, vol. 1, 22-25
- **U. RUDOLPH et al.** *Nature,* 1999, vol. 401, 796-800
- **D.J. NUTT et al.** *Br. J. Psychiatry,* 2001, vol. 179, 390-396
- **C. F. P. GEORGE.** *The Lancet,* 2001, vol. 358, 1623-1626
- **J. LAMEH et al.** *Prog. Neuro-Psychopharmacol. Biol. Psychiatry,* 2000, vol. 24, 979-991
- **H. NOGUCHI et al.** *Eur. J. Pharm.,* 2002, vol. 434, 21-28
- **S. ARBILLA et al.** *Eur. J. Pharmacol.,* 1986, vol. 130, 257-263
- **Y. WU et al.** *Eur. J. Pharmacol.,* 1995, vol. 278, 125-132
- **D. J. SANGER et al.** *Eur.J.Pharmacol.,* 1996, vol. 313, 35-42
- **G. GRIEBEL et al.** *Psychopharmacology,* 1999, vol. 146, 205-213